(19) Europäisches Patentamt
European Patent Office
Office européen des brevets

(11) EP 4 289 843 A1

(12) **EUROPEAN PATENT APPLICATION**
published in accordance with Art. 153(4) EPC

(43) Date of publication:
**13.12.2023 Bulletin 2023/50**

(21) Application number: **22749253.5**

(22) Date of filing: **08.02.2022**

(51) International Patent Classification (IPC):
***C07D 471/04*** *(2006.01)* ***C07D 453/02*** *(2006.01)*
***C07D 519/00*** *(2006.01)* ***A61K 31/519*** *(2006.01)*
***A61P 35/00*** *(2006.01)* ***A61P 35/02*** *(2006.01)*

(52) Cooperative Patent Classification (CPC):
**A61K 31/519; A61P 35/00; A61P 35/02; C07C 217/58; C07C 317/32; C07C 381/10; C07D 333/64; C07D 453/02; C07D 471/04; C07D 519/00**

(86) International application number:
**PCT/CN2022/075428**

(87) International publication number:
**WO 2022/166974 (11.08.2022 Gazette 2022/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.02.2021 CN 202110172372**
**08.11.2021 CN 202111315868**

(71) Applicant: **Wuhan Humanwell Innovative Drug Research and Development Center Limited Company**
**Wuhan, Hubei 430075 (CN)**

(72) Inventors:
- **ZHANG, Xuejun**
  **Wuhan, Hubei 430075 (CN)**
- **CHANG, Shaohua**
  **Wuhan, Hubei 430075 (CN)**
- **LI, Xueqiang**
  **Wuhan, Hubei 430075 (CN)**
- **YE, Dabing**
  **Wuhan, Hubei 430075 (CN)**
- **WANG, Hongqiang**
  **Wuhan, Hubei 430075 (CN)**
- **SUN, Hongna**
  **Wuhan, Hubei 430075 (CN)**
- **YANG, Jun**
  **Wuhan, Hubei 430075 (CN)**
- **LI, Li'e**
  **Wuhan, Hubei 430075 (CN)**

(74) Representative: **Secerna LLP**
**The Old Fire Station**
**18 Clifford Street**
**York YO1 9RD (GB)**

(54) **PYRIDOPYRIMIDINONE DERIVATIVE, PREPARATION METHOD THEREFOR, AND USE THEREOF**

(57) A pyridopyrimidinone derivative as represented by formula I, and a tautomer, a stereoisomer, a hydrate, a solvate, a pharmaceutically acceptable salt thereof or a prodrug thereof. The pyridopyrimidinone derivative has a good SOS1 inhibitory effect.

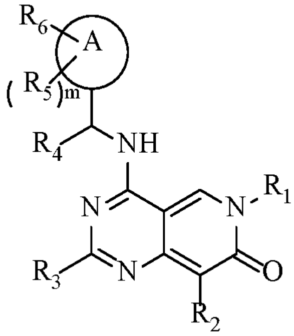

I

EP 4 289 843 A1

## Description

The present application claims priorities to Chinese Patent Application 2021101723722 filed on February 08, 2021 and Chinese Patent Application 2021113158687 filed on November 08, 2021. The contents of the Chinese Patent Applications are incorporated herein by reference in their entireties.

TECHNICAL FIELD

The present disclosure belongs to the field of medicine, specifically, the present disclosure relates to a pyridopyrimidinone derivative, a preparation method therefor and a use thereof.

BACKGROUND

RAS protein is a membrane-bound protein with intrinsic GTPase activity that can be activated by many extracellular stimuli, and cycles between a GDP-bound (off) state and a GTP-bound (on) state. When the RAS protein is in the GTP-bound (on) state, it can activate downstream pathways and promote a series of processes such as cell proliferation, differentiation, migration and immunity.

The RAS protein family comprises three highly homologous isoforms: KRAS (Kirsten rat sarcoma virus oncogene), HRAS (Harvey rat sarcoma virus oncogene) and NRAS (Neuroblastoma ras oncogene), and KRAS comprises two variable splice variants: KRAS4A and KRAS4B. RAS family proteins have weak endogenous GTPase activity and slow nucleotide exchange rate (Hunter et al. Mol. Cancer Res., 2015, 13(9): 1325-1335).

Activation of RAS gene mutation is a significant cause of tumorigenesis, with RAS mutation occurring in 27% of all tumor patients (Hobbs GA, et al. J Cell Sci., 2016, 129(7): 1287-1292). Herein, KRAS has the highest mutation frequency, accounting for 86% (Cox, Adrienne D., et al. Nat Rev Drug Discov., 2014, 13(11): 828-851). KRAS-4B mutation is found in approximately 90% of pancreatic cancers, 30% to 40% of colon cancers, and 15% to 20% of lung cancers. Such mutation is also found in biliary tract malignancies, endometrial cancers, cervical cancers, bladder cancers, liver cancers, myeloid leukemia, and breast cancers (Liu P, et al. Acta Pharm Sin B., 2019, 9(5): 871-879). Point mutation is the most common KRAS gene mutation, with KRAS-G12D (41%), KRAS-G12V (28%) and KRAS-G12C (14%) mutations being common. Mutant KRAS affects its ability to bind to GTPase activating protein (GAP), thereby inhibiting GAP-induced GTP hydrolysis. As the hydrolysis ability of GTPase decreases, GTP gradually accumulates, and KRAS is more likely to bind to GTP, such that KRAS is mostly activated, which induces the occurrence and development of malignancies.

The transition of RAS protein from inactivated state to activated state involves the release of GDP and the binding of GTP. The release of GDP requires the involvement of GMP exchange factor (GEF), such as SOS (Son of Sevenless) protein. SOS protein, first identified in Drosophila in 1992, is the GEF of RAS and Rac proteins, and plays an important role in the RAS and Rac signaling pathways. There are two human SOS homologs, SOS1 and SOS2, which are highly similar in structure and sequence with 70% homology, but differ in biological function. SOS1 protein consists of 1300 amino acid residues with a proline-rich C-terminal domain, which can interact with growth factor receptor-bound protein 2 (Grb2) in the RAS pathway. Grb2 binds to SOS1 to form a complex that can bring SOS1 to the vicinity of the cell membrane RAS protein. The interaction between SOS1 and RAS involves two domains of SOS1: CDC25 domain and REM domain. The CDC25 domain has an active site for nucleotide exchange, and the REM domain contains a site that can bind RAS-GTP and cause allosteric activation of the CDC25 domain (Pierre S, et al. Biochem Pharmacol., 2011, 82(9): 1049-1056). SOS1 can convert GDP to GTP through catalytic exchange. GTP is hydrolyzed by RAS, and then activates downstream signals, causing a series of corresponding biological effects.

Specific SOS1 inhibitors can inhibit the interaction between SOS1 and KRAS-GDP, thereby reducing the formation of activated KRAS-GTP. A reduction in KRAS-GTP levels leads to a reduction in downstream MAPK signaling, which plays a role in both wild-type and multiple KRAS mutant types. BAY-293, a small molecule inhibitor of SOS 1, can effectively reduce the activity of mutant KRAS and wild-type KRAS in tumor cells (Hillig, Roman C., et al. Proc Nat Acad Sci USA., 2019, 116(7): 2551-2560). The SOS1 inhibitors BI-3406 and BI-1701963 developed by Boehringer Ingelheim can bind to the catalytic domain of SOS1, prevent its interaction with KRAS, reduce the formation of KRAS-GTP, and inhibit KRAS-driven proliferation of various cancer cells. The combination of SOS1 inhibitors with MEK inhibitors significantly reduces KRAS signaling and enhances antitumor activity through a complementary mechanism (Hofmann, Marco H, et al. Cancer Discov., 2020: CD-20-0142). According to Boehringer Ingelheim (AACR Annual Meeting, April 27$^{th}$ 2020), BI-3406 inhibits cytochrome P450 3A4 (CYP3A4) in a time-dependent manner with a potential drug-drug interaction (DDI) risk, so the development of cytochrome P450-free inhibition has the advantage that SOS1 inhibitors without CYP3A4 inhibition are more clinically valuable, and BI-1701963 as well as a combination therapy of BI-1701963 and MEK inhibitor Trametinib has entered clinical research.

In addition to cancer, SOS1 gene mutation and abnormal expression are also closely related to the occurrence of several genetic diseases. Noonan syndrome (NS) is an autosomal dominant genetic disease in which about 20% of

NS patients have SOS1 mutations distributed in six domains of SOS1. Patients with SOS1 mutations exhibit phenotypic features of curly hair and abnormal ectoderm. Mutations in the CDC25 domain can directly increase the GEF activity of SOS1 and induce hyperactivation of the RAS/ERK pathway (José M Rojas, et al. Genes Cancer., 2011, 2(3): 298-305). Cardio-facio-cutaneous syndrome is one of renin-angiotensin system (RAS) cardiomyopathy group, and it has been reported that there is a SOS 1 mutation in the disease (Narumi, Yoko, et al. J Hum Genet., 2008, 53(9): 834-841). Hereditary gingival fibromatosis type 1 is an autosomal dominant genetic disease whose etiology is associated with mutations in the proline-rich domain of SOS1 (Jang SI, et al. J Biol Chem., 2007, 282(28): 20245-20255).

CONTENT OF THE PRESENT INVENTION

The purpose of the present disclosure is to provide a pyridopyrimidinone derivative used as an inhibitor of an interaction between a catalytic site of SOS 1 and a RAS family protein involved in the regulation of cell proliferation, which can be used for the treatment of diseases with excessive or abnormal cell proliferation.

The present disclosure solves the above technical problem by the following technical solutions.

A first aspect of the present disclosure provides a pyridopyrimidinone derivative (compound) as represented by formula I, a tautomer thereof, a stereoisomer thereof, a hydrate thereof, a solvate thereof, a pharmaceutically acceptable salt thereof or a prodrug thereof:

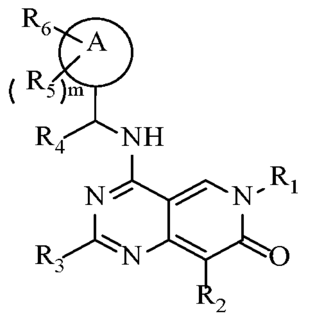

I;

wherein ring A is a 6- to 10-membered aromatic ring or a 9- to 11-membered heteroaromatic ring;

$R_1$ is 3- to 10-membered cycloalkyl or 4- to 10-membered heterocycloalkyl, the $R_1$ is optionally substituted by one or more than one $R_{11}$, the $R_{11}$ is a substituent selected from: halogen, hydroxyl, $C_1$-$C_6$ alkyl, $C_1$-$C_6$ haloalkyl, $C_1$-$C_6$ alkoxy,

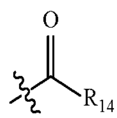,
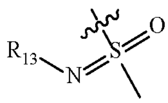

or

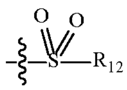;

when there is more than one substituent $R_{11}$, the substituents $R_{11}$ are the same or different;

the $R_{11}$ is optionally substituted by a substituent selected from: $C_1$-$C_6$ alkyl, $C_1$-$C_6$ alkoxy, halogen, hydroxyl; $R_{12}$ is $C_1$-$C_6$ alkyl, $C_1$-$C_6$ alkyl substituted by one or more than one F, or 3- to 6-membered cycloalkyl;

$R_{13}$ is hydrogen, $C_1$-$C_6$ alkyl or cyano;

$R_{14}$ is hydrogen, $C_1$-$C_6$ alkyl, $C_1$-$C_6$ haloalkyl;

$R_2$ is hydrogen or a substituent selected from: halogen, $C_1$-$C_6$ alkyl, 3- to 6-membered cycloalkyl, $C_1$-$C_6$ alkoxy; the $C_1$-$C_6$ alkyl, 3- to 6-membered cycloalkyl, $C_1$-$C_6$ alkoxy are each independently substituted by one or more than one $R_{21}$; the $R_{21}$ is a substituent selected from: hydroxyl, halogen, $C_1$-$C_3$ alkoxy; when there is more than one substituent, the $R_{21}$ are the same or different;

$R_3$ is hydrogen or a substituent selected from: halogen, $C_1$-$C_6$ alkyl, $C_1$-$C_6$ haloalkyl;
$R_4$ is $C_1$-$C_6$ alkyl or $C_1$-$C_6$ haloalkyl;
$R_5$ is hydrogen or a substituent selected from: halogen, $C_1$-$C_6$ alkyl, $C_1$-$C_6$ haloalkyl;
$R_6$ is $-SF_5$,

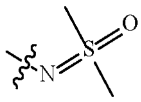 , 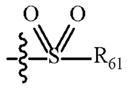 or 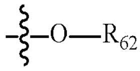 ;

the $R_{61}$ and the $R_{62}$ are each independently $C_1$-$C_6$ alkyl substituted by halogen, or 3- to 6-membered cycloalkyl;
or, the ring A together with $R_6$ and $R_5$ forms a group moiety

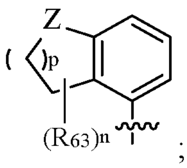 ;

wherein Z is

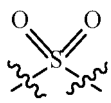 ;

$R_{63}$ is hydrogen or a substituent selected from: halogen, hydroxyl, $C_1$-$C_6$ alkyl, $C_1$-$C_6$ alkyl substituted by halogen;
when there is more than one substituent $R_{63}$, the $R_{63}$ are the same or different;
m is 1 or 2; p is 1, 2, or 3; and n is 1, 2, or 3.

In another preferred embodiment, ring A is a 6- to 10-membered aromatic ring or a 9- to 11-membered heteroaromatic ring;

$R_1$ is 3- to 10-membered cycloalkyl or 4- to 10-membered heterocycloalkyl, the cycloalkyl is optionally substituted by one or more than one $R_{11}$, the $R_{11}$ is a substituent selected from: halogen, $C_1$-$C_6$ alkyl, $C_1$-$C_6$ haloalkyl, $C_1$-$C_6$ alkoxy,

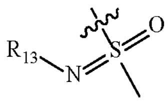 or 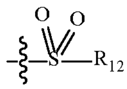 ;

when there is more than one substituent $R_{11}$, the substituents $R_{11}$ are the same or different;
$R_{12}$ is $C_1$-$C_6$ alkyl, $C_1$-$C_6$ alkyl substituted by one or more than one F, or 3- to 6-membered cycloalkyl; $R_{13}$ is hydrogen, $C_1$-$C_6$ alkyl or cyano;
$R_2$ is hydrogen or a substituent selected from: halogen, $C_1$-$C_6$ alkyl, 3- to 6-membered cycloalkyl, $C_1$-$C_6$ alkoxy; the $C_1$-$C_6$ alkyl, 3- to 6-membered cycloalkyl, $C_1$-$C_6$ alkoxy are each independently substituted by one or more than one $R_{21}$; the $R_{21}$ is a substituent selected from: hydroxyl, halogen, $C_1$-$C_3$ alkoxy; when there is more than one substituent, the $R_{21}$ are the same or different;
$R_3$ is hydrogen or a substituent selected from: halogen, $C_1$-$C_6$ alkyl, $C_1$-$C_6$ haloalkyl;
$R_4$ is $C_1$-$C_6$ alkyl or $C_1$-$C_6$ haloalkyl;
$R_5$ is hydrogen or a substituent selected from: halogen, $C_1$-$C_6$ alkyl, $C_1$-$C_6$ haloalkyl;
$R_6$ is $-SF_5$,

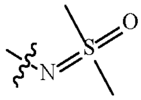 , 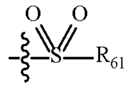 or 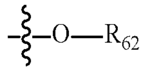 ;

the $R_{61}$ and the $R_{62}$ are each independently $C_1$-$C_6$ alkyl substituted by halogen, or 3- to 6-membered cycloalkyl; or, the ring A together with $R_6$ and $R_5$ forms the group moiety

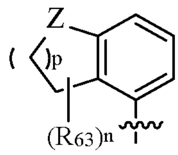 ;

wherein Z is

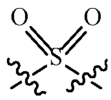 ;

$R_{63}$ is hydrogen or a substituent selected from: halogen, hydroxyl, $C_1$-$C_6$ alkyl, $C_1$-$C_6$ alkyl substituted by halogen; when there is more than one substituent $R_{63}$, the $R_{63}$ are the same or different;
m is 1 or 2; p is 1, 2, or 3; and n is 1, 2, or 3.

In the present disclosure, the definitions of some substituents in the pyridopyrimidinone derivative (compound) as represented by formula I, the tautomer thereof, the stereoisomer thereof, the hydrate thereof, the solvate thereof, the pharmaceutically acceptable salt thereof or the prodrug thereof may be described as follows, and the definitions of unmentioned substituents are as described in any one of the embodiments of the present disclosure (hereinafter referred to as "in another preferred embodiment").

In another preferred embodiment, 9- to 11-membered heteroaromatic ring is a 9- to 11-membered benzoheterocyclic ring, wherein heteroatom is selected from O, N or S; and the number of heteroatoms is one or two.

In another preferred embodiment, the ring A is benzene ring or a 9-membered benzoheterocyclic ring, wherein the heteroatom is selected from O, N or S; preferably, the heteroatom is S; there are one or two heteroatoms in the ring A, preferably, there is one heteroatom in the ring A.

In another preferred embodiment, the pyridopyrimidinone derivative (compound) as represented by formula I has a structure of I-1,

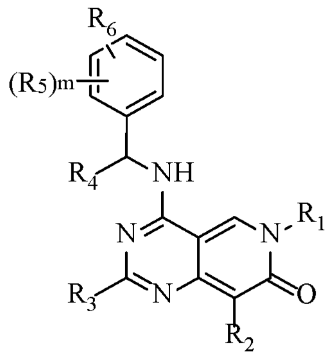

I-1

;

preferably, the pyridopyrimidinone derivative (compound) as represented by formula I has a structure of I-2,

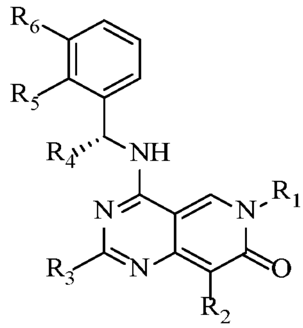

I-2

;

wherein $R_1$, $R_2$, $R_3$, $R_5$, $R_6$ and m are as defined in the first aspect of the present disclosure; $R_4$ is methyl or $-CH_2F$; preferably, $R_4$ is methyl.

In another preferred embodiment, $R_5$ is hydrogen, fluorine or methyl; m is 1 or 2; preferably, m is 1; $R_6$ is $-SF_5$,

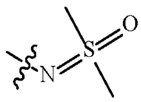

,

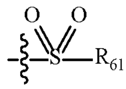

or

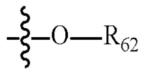

;

$R_{61}$ and $R_{62}$ are each independently $C_1$-$C_6$ alkyl substituted by one or more than one F, or 3- to 6-membered cycloalkyl;

preferably, $R_{61}$ is $-CH_2F$, $-CHF_2$, $-CF_3$, $-CF_2CH_3$ or cyclopropyl;
preferably, $R_{62}$ is $-CH_2F$, $-CHF_2$, $-CF_3$; more preferably, $R_{62}$ is $-CHF_2$.

In another preferred embodiment, ring A together with $R_6$ and $R_5$ forms a group moiety

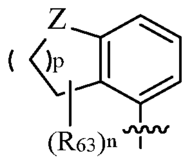

;

wherein Z is

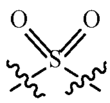

;

p is 1, n is 2 or 3, $R_{63}$ is fluorine or hydroxyl, and when there is more than one $R_{63}$, the $R_{63}$ are the same or different; preferably, the group moiety

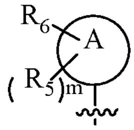

has a structure of

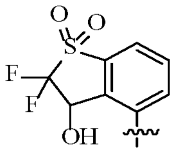
;

more preferably,

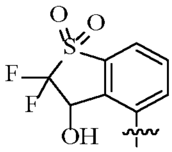 is 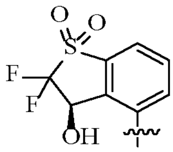 .

In another preferred embodiment, the benzene ring together with $R_6$ and $R_5$ forms a group moiety

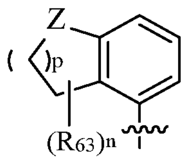
;

wherein Z is

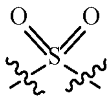
;

p is 1, n is 2 or 3, $R_{63}$ is fluorine or hydroxyl, and when there is more than one $R_{63}$, the $R_{63}$ are the same or different; preferably, a group moiety

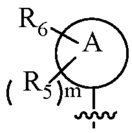

has a structure of

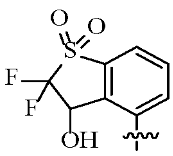
;

more preferably,

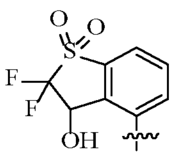 is 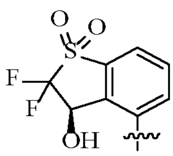 .

In another preferred embodiment, $R_5$ is hydrogen; $R_6$ is $-SF_5$,

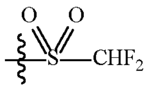

or

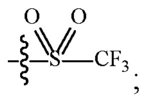

preferably, $R_6$ is $-SF_5$.

In another preferred embodiment, $R_4$ is $C_1$-$C_3$ alkyl or $C_1$-$C_3$ haloalkyl; preferably, $R_4$ is methyl or $-CH_2F$; more preferably, $R_4$ is methyl.

In another preferred embodiment, the halogen is fluorine, chlorine, bromine; preferably, the halogen is fluorine.

In another preferred embodiment, in the $R_1$, the 3- to 10-membered cycloalkyl comprises a monocyclic, bicyclic, tricyclic, spiro or bridged ring; the 4- to 10-membered heterocycloalkyl has one or more than one heteroatom, the heteroatom is N, O or S.

In another preferred embodiment, the 3- to 10-membered cycloalkyl is: cyclopropyl, cyclobutyl, cyclopentyl, cyclohexyl, cycloheptyl, bicyclo[1.1.1]pentyl, bicyclo[2.2.0]hexyl, bicyclo[3.2.0]heptyl, bicyclo[3.2.1]octyl, bicyclo[2.2.2]octyl, bicyclo[4.3.0]nonyl (octahydroindenyl), bicyclo[4.4.0]decyl (decahydronaphthalene), bicyclo[2.2.1]heptyl (norbomyl), bicyclo[4.1.0]heptyl (norcaranyl), bicyclo[3.1.1]heptyl (pinanyl), spiro[2.5]octyl, spiro[3.3]heptyl; more preferably, the 3- to 10-membered cycloalkyl is:

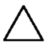 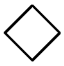 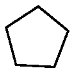 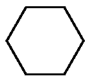 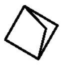 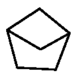 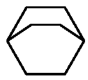 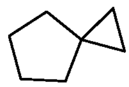 .

In another preferred embodiment, in the $R_1$, the 3- to 10-membered cycloalkyl is cyclopropyl or cyclobutyl.

In another preferred embodiment, the 4- to 10-membered heterocycloalkyl is: tetrahydrofuranyl, pyrrolidinyl, pyrrolinyl, imidazolidinyl, thiazolidinyl, imidazolinyl, pyrazolidinyl, pyrazolinyl, piperidinyl, piperazinyl, oxiranyl, aziridinyl, azetidinyl, 1,4-dioxanyl, azepanyl, diazepanyl, morpholinyl, thiomorpholinyl, homomorpholinyl, homopiperidinyl, homopiperazinyl, homothiomorpholinyl, thiomorpholinyl-S-oxide, thiomorpholinyl-S,S-dioxide, 1,3-dioxolanyl, tetrahydropyranyl, tetrahydrothiopyranyl, [1.4]-oxazepanyl, tetrahydrothienyl, homothiomorpholinyl-S, S-dioxide, oxazolidonyl, dihydropyrazolyl, dihydropyrrolyl, dihydropyrazinyl, dihydropyridinyl, dihydropyrimidinyl, dihydrofuranyl, dihydropyranyl, tetrahydrothienyl-S-oxide, tetrahydrothienyl-S,S-dioxide, homothiomorpholinyl-S-oxide, 2,3-dihydroazetidinyl, 2H-pyrrolyl, 4H-pyranyl, 1,4-dihydropyridinyl, 8-aza-bicyclo[3.2.1]octyl, 8-aza-bicyclo[5.1.0]octyl, 2-oxa-5-azabicyclo[2.2.1]heptyl, 8-oxa-3-aza-bicyclo[3.2.1]octyl, 3,8-diaza-bicyclo[3.2.1]octyl, 2,5-diaza-bicyclo-[2.2.1]heptyl, 1-aza-bicyclo[2.2.2]octyl, 3,8-diaza-bicyclo[3.2.1]octyl, 3,9-diaza-bicyclo[4.2.1]nonyl, 2,6-diaza-bicyclo[3.2.2]nonyl; more preferably, the 4- to 10-membered heterocycloalkyl is:

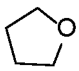 , 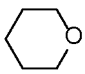 , 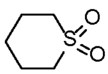 , 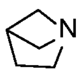 , 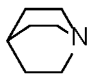 , 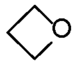 , 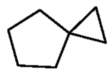 ,

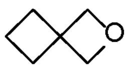 , 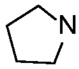 , 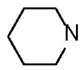 ;

or, the 4- to 10-membered heterocycloalkyl is:

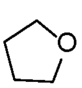 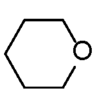 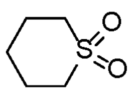 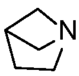 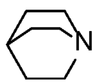 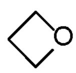 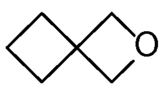 .

In another preferred embodiment, in the $R_1$, the 4- to 10-membered heterocycloalkyl is 4- to 6-membered

heterocycloalkyl;

the 4- to 10-membered heterocycloalkyl has 1, 2 or 3 heteroatoms, and the heteroatom is N, O or S; for example, the number of heteroatoms is 1 or 2, for example, the heteroatom is N or O; for example, oxetanyl, piperidinyl, pyrrolidinyl, tetrahydrofuranyl, tetrahydropyranyl; for another example,

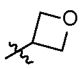, 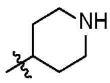, 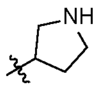, 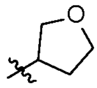, 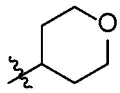.

In another preferred embodiment, $R_1$ is 3- to 6-membered cycloalkyl or 4- to 6-membered heterocycloalkyl, the cycloalkyl is optionally substituted by one or more than one $R_{11}$, the $R_{11}$ is a substituent selected from: halogen, $C_1$-$C_6$ alkyl, $C_1$-$C_6$ haloalkyl; when there is more than one substituent $R_{11}$, the substituents $R_{11}$ are the same or different; more preferably, $R_1$ is

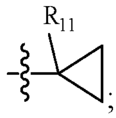;

wherein the $R_{11}$ is a substituent selected from: halogen, $C_1$-$C_6$ alkyl, $C_1$-$C_6$ haloalkyl; preferably, $R_{11}$ is $C_1$-$C_6$ alkyl or $C_1$-$C_6$ alkyl substituted by fluorine; more preferably, $R_{11}$ is methyl, $-CH_2F$ or $-CHF_2$.

In another preferred embodiment, $R_1$ is 3- to 10-membered cycloalkyl or 4- to 10-membered heterocycloalkyl, the heterocycloalkyl is optionally substituted by one or more than one $R_{11}$, the $R_{11}$ is hydroxyl or

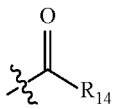;

when there is more than one substituent $R_{11}$, the substituents $R_{11}$ are the same or different;

the $R_{11}$ is optionally substituted by a substituent selected from: $C_1$-$C_6$ alkyl, $C_1$-$C_6$ alkoxy, halogen, hydroxyl; preferably, $R_{11}$ is optionally substituted by a substituent selected from: $C_1$-$C_3$ alkyl, $C_1$-$C_3$ alkoxy, halogen, hydroxyl; $R_{14}$ is hydrogen, $C_1$-$C_6$ alkyl, $C_1$-$C_6$ haloalkyl; preferably, $R_{14}$ is hydrogen, $C_1$-$C_3$ alkyl, $C_1$-$C_3$ haloalkyl.

In another preferred embodiment, $R_1$ is 3- to 6-membered cycloalkyl or 4- to 6-membered heterocycloalkyl, the $R_1$ is optionally substituted by one or more than one $R_{11}$, the $R_{11}$ is a substituent selected from: halogen, hydroxyl, $C_1$-$C_6$ alkyl, $C_1$-$C_6$ alkoxy,

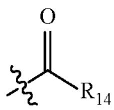;

$R_{14}$ is hydrogen, $C_1$-$C_6$ alkyl, $C_1$-$C_6$ haloalkyl; preferably, $R_{14}$ is hydrogen, $C_1$-$C_3$ alkyl, $C_1$-$C_3$ haloalkyl; the $R_{11}$ is optionally substituted by a substituent selected from: $C_1$-$C_3$ alkoxy, halogen; preferably, the halogen is fluorine.

In another preferred embodiment, $R_1$ is selected from cyclopropyl, cyclobutyl, tetrahydrofuranyl, tetrahydropyranyl, epoxypropanyl, pyrrolidinyl or piperidinyl; the $R_1$ is optionally substituted by one or more than one $R_{11}$, and the $R_{11}$ is a substituent selected from: fluorine, hydroxyl, $C_1$-$C_6$ alkyl, $C_1$-$C_6$ alkyl substituted by $C_1$-$C_3$ alkoxy, $C_1$-$C_6$ alkoxy,

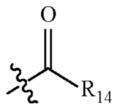,

$R_{14}$ is $C_1$-$C_6$ alkyl;
preferably, $R_1$ is selected from:

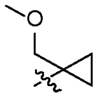, 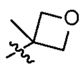, 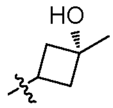, 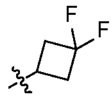, 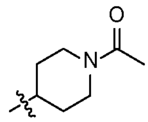,

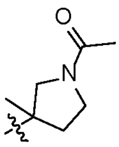, 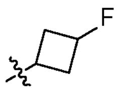, 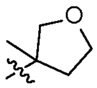, 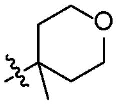, 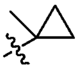, 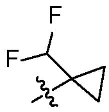.

In another preferred embodiment, $R_5$ is hydrogen; $R_6$ is $-SF_5$; $R_4$ is methyl; $R_3$ is methyl; and $R_2$ is hydrogen.

In another preferred embodiment, $R_1$ is

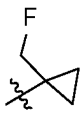, 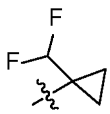, 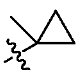, 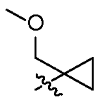, 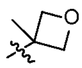,

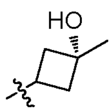, 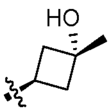, 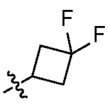, 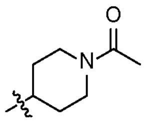, 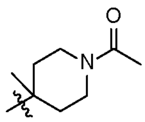, 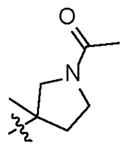, 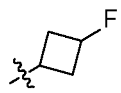, 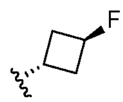,

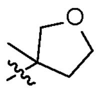, 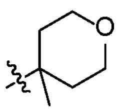.

In another preferred embodiment,

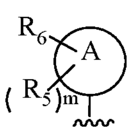 is 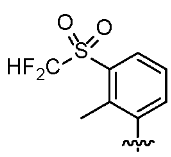, 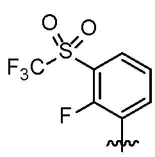,

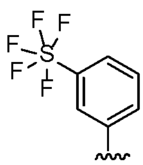, 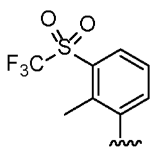, 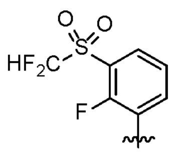, 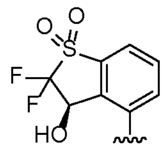, 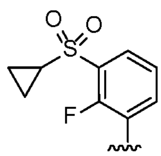, 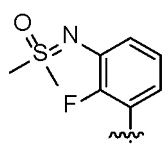,

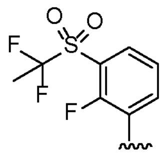,
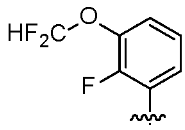.

In another preferred embodiment, $R_2$ is hydrogen or a substituent selected from: halogen, $C_1$-$C_6$ alkyl, 3- to 6-membered cycloalkyl; preferably, $R_2$ is hydrogen or halogen; more preferably, $R_2$ is hydrogen.

In another preferred embodiment, $R_3$ is hydrogen, $C_1$-$C_6$ alkyl or $C_1$-$C_6$ haloalkyl; preferably, $R_3$ is $C_1$-$C_6$ alkyl; more preferably, $R_3$ is methyl.

In another preferred embodiment, the pyridopyrimidinone derivative (compound) comprises:

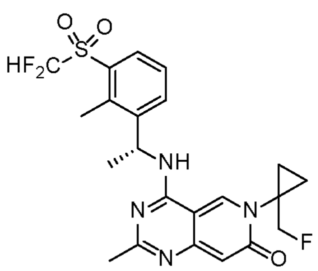
I-1

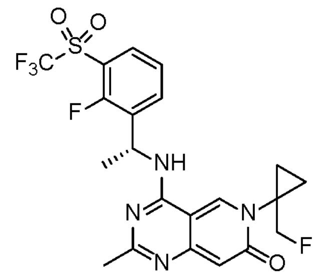
I-2

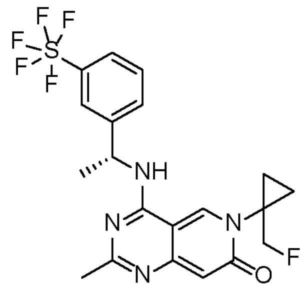
I-3

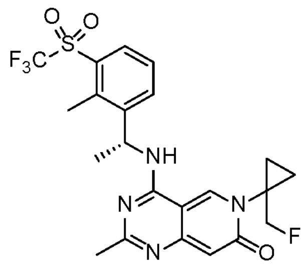
I-4

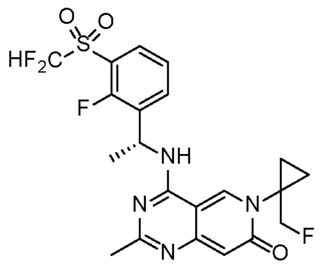
I-5

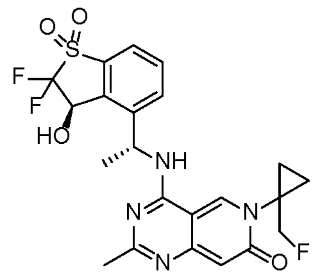
I-6

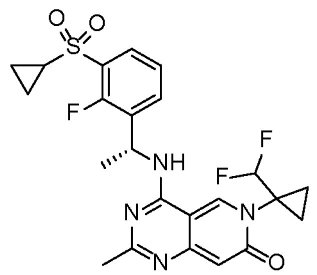
I-7

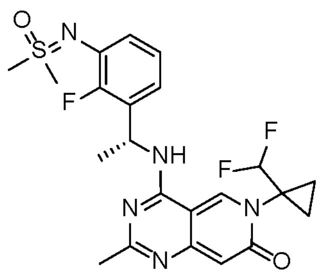
I-8

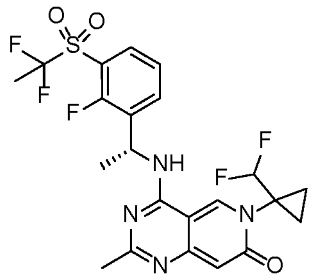
I-9

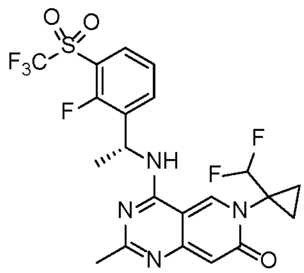
I-10

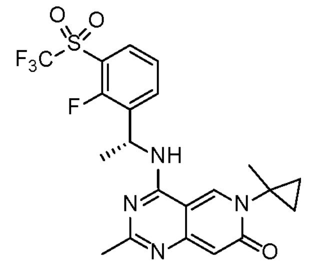
I-11

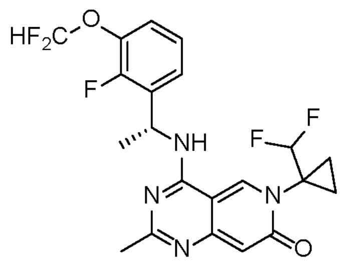
I-12

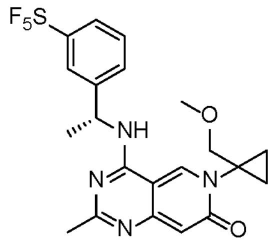
I-13

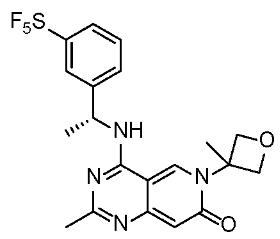
I-14

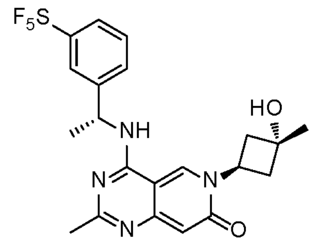
I-15

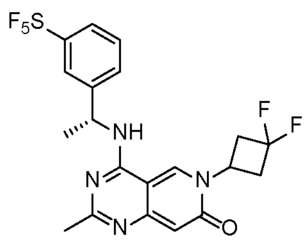
I-16

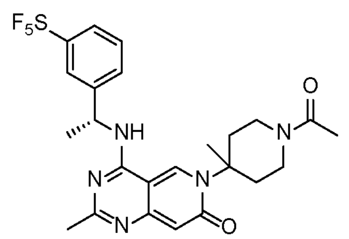
I-17

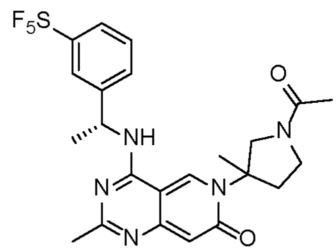
I-18

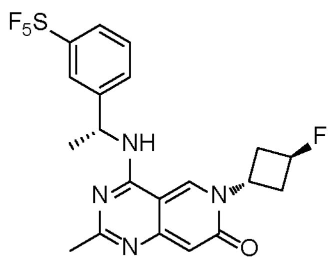
I-19

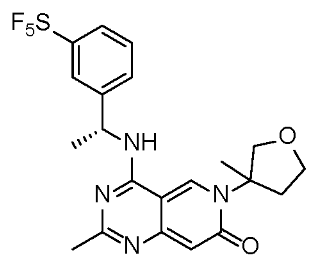
I-20

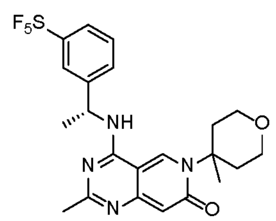
I-21

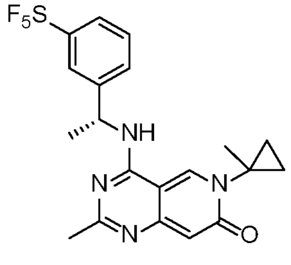
I-22

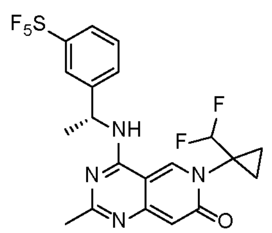
I-23.

A second aspect of the present disclosure provides an intermediate represented by formula B-1, a tautomer

thereof, a stereoisomer thereof, and a salt thereof,

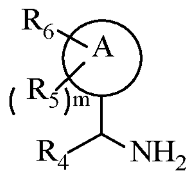

B-1;

wherein ring A, $R_4$, $R_5$ and $R_6$ are as defined in the first aspect; m is 1 or 2.

In another preferred embodiment, the intermediate represented by formula B-1, the tautomer thereof, the stereoisomer thereof, and the salt thereof have a structure of

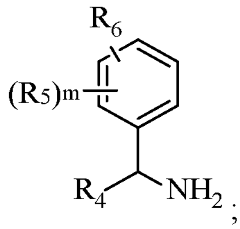

wherein ring A, $R_4$, $R_5$, $R_6$ and m are as defined in formula B-1 of the second aspect;
preferably, $R_4$ is methyl or $-CH_2F$; more preferably, $R_4$ is methyl.

In another preferred embodiment, $R_5$ is hydrogen, fluorine or methyl; m is 1 or 2; preferably, m is 1; $R_6$ is $-SF_5$,

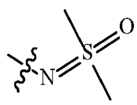 , 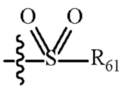 or 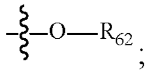 ;

$R_{61}$ and $R_{62}$ are each independently $C_1$-$C_6$ alkyl substituted by one or more than one F, or 3- to 6-membered cycloalkyl;

preferably, $R_{61}$ is $-CH_2F$, $-CHF_2$, $-CF_3$, $-CF_2CH_3$ or cyclopropyl;
preferably, $R_{62}$ is $-CH_2F$, $-CHF_2$, $-CF_3$; more preferably, $R_{62}$ is $-CHF_2$.

In another preferred embodiment, ring A together with $R_6$ and $R_5$ forms a group moiety

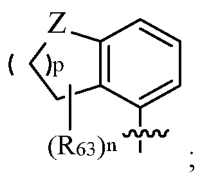

wherein Z is

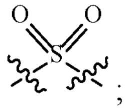

p is 1, n is 2 or 3, $R_{63}$ is fluorine or hydroxyl, and when there is more than one $R_{63}$, the $R_{63}$ are the same or different; preferably, the group moiety

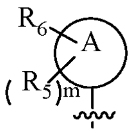

has a structure of

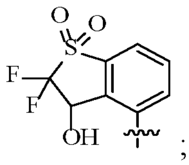

more preferably,

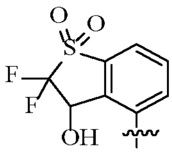 is 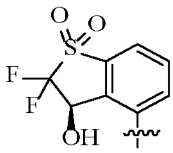 .

In another preferred embodiment, the intermediate represented by formula B-1, the tautomer thereof, the stereoisomer thereof, and the salt thereof comprise:

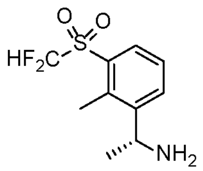

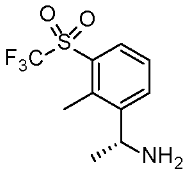

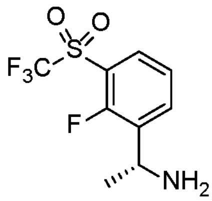

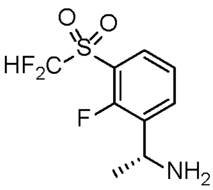

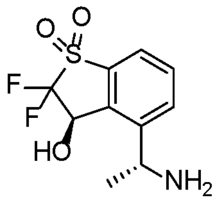

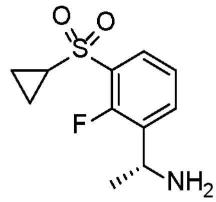

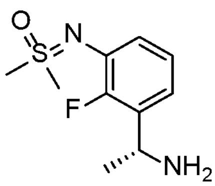

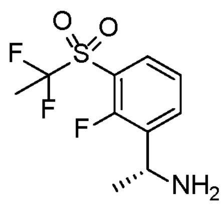

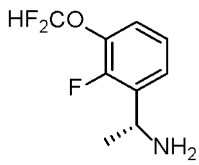

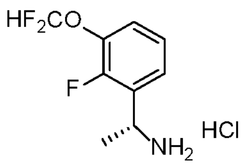

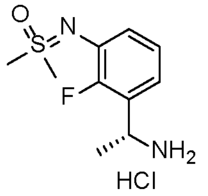

.

In another preferred embodiment, the intermediate represented by formula B-1, the tautomer thereof, the stereoisomer thereof, and the salt thereof are not

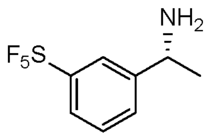

,

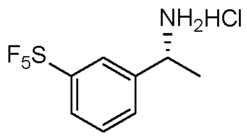

,

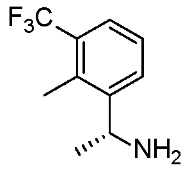

,

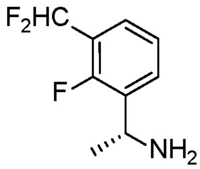

,

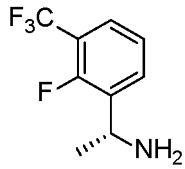

,

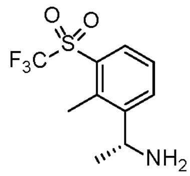

,

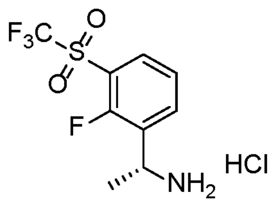

.

A third aspect of the present disclosure provides a method for preparing the pyridopyrimidinone derivative (compound) as represented by formula I, the tautomer thereof, the stereoisomer thereof, the hydrate thereof, the solvate thereof, the pharmaceutically acceptable salt thereof or the prodrug thereof as described in the first aspect, and the method comprises:

1) reacting an intermediate B-1 with an intermediate B-2 to obtain the pyridopyrimidinone derivative (compound) as represented by formula I,

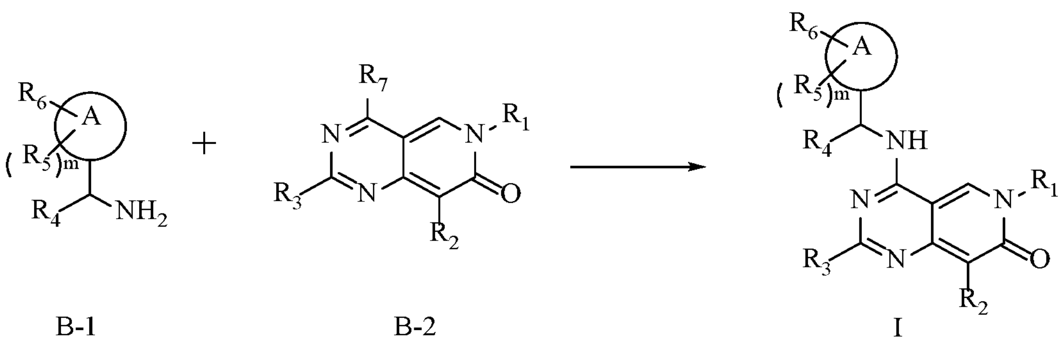

B-1 B-2 I

.

;

wherein ring A, $R_1$, $R_2$, $R_3$, $R_4$, $R_5$, $R_6$, m, p and n are as defined in the first aspect;
$R_7$ is hydroxyl, chlorine, bromine, iodine or sulfonate; preferably, $R_7$ is hydroxyl;

preferably, the sulfonate is $-SO_3R_{71}$, wherein $R_{71}$ is methyl, $-CF_3$, phenyl or 2,4,6-trimethylbenzene.

In another preferred embodiment, ring A is phenyl; preferably, a group moiety

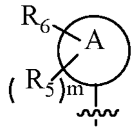

has a structure of

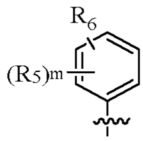

.

In another preferred embodiment, $R_5$ is hydrogen, fluorine or methyl; m is 1 or 2; preferably, m is 1; $R_6$ is $-SF_5$,

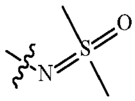

,

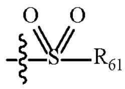

or

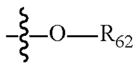

;

$R_{61}$ and $R_{62}$ are each independently $C_1$-$C_6$ alkyl substituted by one or more than one F, or 3- to 6-membered cycloalkyl;

or, ring A together with $R_6$ and $R_5$ forms a group moiety

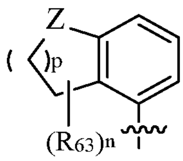

;

wherein Z is

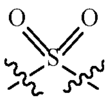

;

p is 1, n is 2 or 3, $R_{63}$ is fluorine or hydroxyl, and when there is more than one $R_{63}$, the $R_{63}$ are the same or different; preferably, a group moiety

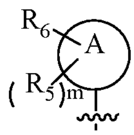

has a structure of

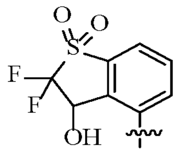;

more preferably,

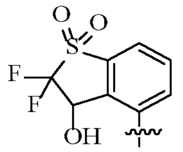 is 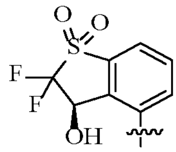.

preferably, $R_{61}$ is $-CH_2F$, $-CHF_2$, $-CF_3$, $-CF_2CH_3$ or cyclopropyl;
preferably, $R_{62}$ is $-CH_2F$, $-CHF_2$, $-CF_3$; more preferably, $R_{62}$ is $-CHF_2$.

In a preferred embodiment of the present disclosure, the method further comprises: 2) converting the intermediate B-1 to an amine salt of B-1 and then reacting with the intermediate B-2 to obtain the pyridopyrimidinone derivative (compound) as represented by formula I. The amine salt may be an amine salt obtained by reacting the pharmaceutically acceptable inorganic acid or organic acid described in the present disclosure with $-NH_2$ in the intermediate B-1, including but not limited to: amine hydrochloride, amine sulfate, amine nitrate, amine phosphate.

In a preferred embodiment of the present disclosure, the method further comprises: 3) reacting the intermediate B-1 with the intermediate B-2 and then preparing the pyridopyrimidinone derivative (compound) as represented by formula I under an acidic preparation condition B. The acidic preparation condition B includes: Welch, Ultimate C18 column, 10 μm, 21.2 mm × 250 mm, mobile phase A is a 1‰ solution of formic acid in pure water, and mobile phase B is an acetonitrile solution. Gradient conditions: mobile phase A was kept at 90% (0 to 3 min), then reduced from 90% to 5% by gradient elution (3 to 18 min), and kept at 5% (18 to 22 min).

According to the difference of each group in the compound as represented by formula I, different synthetic routes and intermediates can be selected. When there is an active group (such as carboxyl, amino, hydroxyl, etc.) in the substituent, the active group may be protected by a protecting group as required before participating in the reaction, and the protecting group is deprotected after the reaction is completed. The compounds in which one or more than one reactive site is blocked by one or more than one protecting group (also referred to as protective groups) are "protected derivatives (compounds)" of the compound as represented by formula I in the present disclosure. For example, suitable carboxyl protecting groups include benzyl, tert-butyl, isotopes, etc. Suitable amino and amido protecting groups include acetyl, trifluoroacetyl, *tert*-butoxycarbonyl, benzyloxycarbonyl, etc. Suitable hydroxyl protecting groups include benzyl, etc. Other suitable protecting groups are well known to those skilled in the art.

In some embodiments, the reaction requires the protection of an inert gas, and the inert gas includes, but is not limited to: nitrogen, helium, neon, and argon.

The reaction of each step of the present disclosure is preferably carried out in an inert solvent, and the inert solvent includes, but is not limited to: toluene, benzene, water, methanol, ethanol, isopropanol, ethylene glycol, *N*-methylpyrrolidone, dimethyl sulfoxide, tetrahydrofuran dichloromethane, trichloromethane, 1,2-dichloroethane, acetonitrile, *N,N*-dimethylformamide, *N,N*-dimethylacetamide, dioxane, or combinations thereof.

A fourth aspect of the present disclosure provides a pharmaceutical composition, comprising: the pyridopyrimidinone derivative (compound) as represented by formula I, the tautomer thereof, the stereoisomer thereof, the hydrate thereof, the solvate thereof, the pharmaceutically acceptable salt thereof or the prodrug thereof according to the first aspect of the present disclosure; and a pharmaceutically acceptable carrier. In addition, a pharmaceutical composition is provided, which comprises: the pyridopyrimidinone derivative (compound) as represented by formula I, the tautomer thereof, the stereoisomer thereof, the hydrate thereof, the solvate thereof, the pharmaceutically acceptable salt thereof or the prodrug thereof according to the first aspect of the present disclosure; and at least one other pharmacologically active inhibitor. Preferably, the other pharmacologically active inhibitor is an inhibitor of MEK and/or a mutant thereof. Preferably, the other pharmacologically active inhibitor is trametinib.

A fifth aspect of the present disclosure provides a use of the pyridopyrimidinone derivative (compound) as represented by formula I, the tautomer thereof, the stereoisomer thereof, the hydrate thereof, the solvate thereof, the pharmaceutically acceptable salt thereof or the prodrug thereof according to the first aspect of the present disclosure, or a use of the pharmaceutical composition according to the fourth aspect of the present disclosure, the use comprises:

inhibiting an interaction between SOS1 and RAS family proteins; and/or, preventing and/or treating a disease related to SOS 1 and RAS family proteins; and/or, preparing a medicament, pharmaceutical composition or formulation for inhibiting an interaction between SOS1 and RAS family proteins, and/or preventing and/or treating a disease related to (or mediated by) SOS1 and RAS family proteins. Preferably, the use comprises a combined use of the pyridopyrimidinone derivative (compound) as represented by formula I, the tautomer thereof, the stereoisomer thereof, the hydrate thereof, the solvate thereof, the pharmaceutically acceptable salt thereof or the prodrug thereof with trametinib.

Preferably, the disease related to (or mediated by) SOS1 and RAS family proteins includes, but is not limited to: cancer and RASopathies. The RASopathies comprise Noonan syndrome, cardio-facio-cutaneous syndrome, hereditary gingival fibromatosis type 1, neurofibromatosis type 1, capillary malformation-arteriovenous malformation syndrome, Costello syndrome and Legius syndrome.

The fifth aspect of the present disclosure also provides a use of the pyridopyrimidinone derivative (compound) as represented by formula I, the tautomer thereof, the stereoisomer thereof, the hydrate thereof, the solvate thereof, the pharmaceutically acceptable salt thereof or the prodrug thereof according to the first aspect of the present disclosure, or a use of the pharmaceutical composition according to the fourth aspect of the present disclosure in the preparation of a medicament (pharmaceutical composition or formulation); the medicament can be used for the prevention and/or treatment of the disease mediated by SOS1 and RAS family proteins; or, the medicament can be used for the prevention and/or treatment of cancer and RASopathies. The RASopathies or diseases mediated by SOS 1 and RAS family proteins comprise Noonan syndrome, cardio-facio-cutaneous syndrome, hereditary gingival fibromatosis type 1, neurofibromatosis type 1, capillary malformation-arteriovenous malformation syndrome, Costello syndrome and Legius syndrome. Preferably, the medicament comprises the pyridopyrimidinone derivative (compound) as represented by formula I, the tautomer thereof, the stereoisomer thereof, the hydrate thereof, the solvate thereof, the pharmaceutically acceptable salt thereof or the prodrug thereof and trametinib.

Preferably, the cancer is selected from melanoma, skin cancer, liver cancer, kidney cancer, lung cancer, nasopharyngeal cancer, gastric cancer, esophageal cancer, colorectal cancer, gallbladder carcinoma, cholangiocarcinoma, choriocarcinoma, pancreatic cancer, polycythemia vera, pediatric tumor, cervical cancer, ovarian cancer, breast cancer, bladder cancer, urothelial carcinoma, ureteral tumor, prostate cancer, seminoma, testicular cancer, leukemia, head and neck tumor, endometrial cancer, thyroid cancer, lymphoma, sarcoma, osteoma, neuroblastoma, neuroblastoma, brain tumor, myeloma, astrocytoma, glioblastoma and glioma; the liver cancer is preferably hepatocellular carcinoma; the head and neck tumor is preferably head and neck squamous cell carcinoma; the sarcoma is preferably osteosarcoma; and the colorectal cancer is preferably colon cancer or rectal cancer.

The RASopathies are preferably neurofibromatosis type 1 (NF1); the lung cancer is preferably non-small cell lung cancer, further preferably metastatic non-small cell lung cancer; the leukemia is preferably chronic lymphocytic leukemia or acute myeloid leukemia; the lymphoma is preferably diffuse large B-cell lymphoma; the myeloma is preferably multiple myeloma; the osteoma is preferably osteochondroma; the liver cancer is preferably hepatocellular carcinoma; the head and neck tumor is preferably head and neck squamous cell carcinoma; the sarcoma is preferably osteosarcoma; and the colorectal cancer is preferably colon cancer or rectal cancer.

The RAS family protein may be KRAS, such as KRAS G12C.

A sixth aspect of the present disclosure provides a method for inhibiting SOS1 and RAS family proteins, or preventing and/or treating a disease related to (or mediated by) SOS 1 and RAS family proteins, comprising the steps of: administering to a subject in need thereof the pyridopyrimidinone derivative (compound) as represented by formula I, the tautomer thereof, the stereoisomer thereof, the hydrate thereof, the solvate thereof, the pharmaceutically acceptable salt thereof or the prodrug thereof according to the first aspect of the present disclosure. Preferably, the method comprises using the pyridopyrimidinone derivative (compound) as represented by formula I, the tautomer thereof, the stereoisomer thereof, the hydrate thereof, the solvate thereof, the pharmaceutically acceptable salt thereof or the prodrug thereof in combination with trametinib.

The additional aspects and advantages of the present disclosure will be partly given in the following description, and part of them will become apparent from the following description, or can be understood through the implementation of the present disclosure.

## Term definitions and explanations

Unless otherwise specified, the definitions of groups and terms described in the description and claims include definitions thereof as examples, exemplary definitions, preferred definitions, definitions recorded in tables, and definitions of specific compounds in the embodiments, etc., which can be arbitrarily combined and integrated with each other. Such combined and integrated definitions of groups and compound structures should fall within the scope of the description of the present disclosure.

Unless otherwise defined, all scientific and technological terms of the present disclosure have the same meanings as those commonly understood by those skilled in the art to which the subjects of the claims belong. Unless otherwise

specified, all granted patents, patent applications, and published documents cited in the present disclosure are incorporated herein by reference in their entireties. If a term has multiple definitions in the present disclosure, the definitions in this section shall prevail.

It should be understood that the foregoing brief description and the following detailed description are exemplary and for explanatory purposes only, and do not limit the subject matter of the present disclosure in any way. In the present disclosure, the use of the singular also includes the plural unless specifically stated otherwise. It must be noted that the singular form used in the present description and claims includes the plural form of the object referred to unless clearly stated otherwise. It should also be noted that the use of "or" and "alternatively" indicates "and/or" unless otherwise specified. Furthermore, the use of the term "including" as well as other forms, such as "comprising", "includes" and "included," is not limiting.

Definitions of standard chemical terms can be found in references (including Carey and Sundberg "ADVANCED ORGANIC CHEMISTRY 4TH ED." Vols. A(2000) and B (2001), Plenum Press, New York). Unless otherwise specified, conventional methods in the technical scope of the art, such as mass spectrometry, NMR, IR and UV/Vis spectroscopy, and pharmacological methods are used. Unless specific definitions are provided, the terms used herein in the relevant descriptions of analytical chemistry, synthetic organic chemistry, and pharmaceuticals and medicinal chemistry are known in the art Standard techniques can be used in chemical synthesis, chemical analysis, drug preparation, formulation and delivery, and treatment of patients. For example, reaction and purification can be carried out according to the manufacturer's instructions for use of the kit or in a manner known in the art or the description of the present disclosure. Generally, the above-mentioned techniques and methods can be implemented according to the descriptions in a number of summary and more specific documents cited and discussed in the present disclosure according to conventional methods well-known in the art. In the present description, groups and substituents thereof can be selected by those skilled in the art to provide stable structural moieties and compounds.

When a substituent is described by a conventional chemical formula written from left to right, the substituent also includes the chemically equivalent substituent obtained when the structural formula is written from right to left. For example, $CH_2O$ is equivalent to $OCH_2$. As used herein,

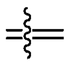 or 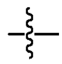

indicates the connection site of the group. As used herein, "Ri", "R1" and "$R^1$" have the same meaning and can be interchanged. For other symbols such as $R_2$, similar definitions have the same meaning.

The section headings used herein are for organizational purposes only and should not be construed as limiting the subject matter described. All documents, or portions of documents, cited in the present disclosure, including but not limited to patents, patent applications, articles, books, manuals and treatises, are incorporated herein by reference in their entireties.

In addition to the foregoing, when used in the description and claims of the present disclosure, the following terms have the meanings shown below unless otherwise specified.

When the numerical range described in the description and claims of the present disclosure is understood as "integers", it should be understood as recording the two endpoints of the range and all integers in the range. For example, an "integer from 1 to 6" should be understood as recording every integer of 0, 1, 2, 3, 4, 5 and 6.

In the present disclosure, the term "halogen" alone or as part of another substituent refers to fluorine, chlorine, bromine, iodine; preferably fluorine.

As used herein, the term "amino" alone or as part of another substituent means $-NH_2$.

As used herein, the term "alkyl" alone or as part of another substituent refers to a linear or branched hydrocarbon chain group consisting solely of carbon atoms and hydrogen atoms, free of unsaturated bonds, having, for example, 1 to 6 carbon atoms, and connected to the rest of the molecule by a single bond. Examples of alkyl include, but are not limited to, methyl, ethyl, *n*-propyl, isopropyl, *n*-butyl, isobutyl, *tert*-butyl, pentyl, isopentyl, neopentyl and hexyl. Alkyl may be unsubstituted or substituted by one or more than one suitable substituent. Alkyl may also be an isotopic isomer of naturally abundant alkyl rich in isotopes of carbon and/or hydrogen (i.e., deuterium or tritium). As used herein, the term "alkenyl" refers to an unbranched or branched monovalent hydrocarbon chain that contains one or more than one carbon-carbon double bond. As used herein, the term "alkynyl" refers to an unbranched or branched monovalent hydrocarbon chain that contains one or more than one carbon-carbon triple bond.

The term "$C_1$-$C_6$ alkyl", alone or as part of another substituent, should be understood to mean a linear or branched saturated monovalent hydrocarbon radical having 1, 2, 3, 4, 5 or 6 carbon atoms. The alkyl is, for example, methyl, ethyl, propyl, butyl, pentyl, hexyl, isopropyl, isobutyl, *sec*-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or 1,2-dimethylbutyl, etc. or isomers thereof. In particular, the group has 1, 2 or 3 carbon atoms ("$C_1$-$C_3$ alkyl"), such as methyl, ethyl, *n*-propyl or isopropyl.

The term "cycloalkyl" or "carbocyclyl" alone or as part of another substituent refers to a cyclic alkyl group. The term "m- to n-membered cycloalkyl" or "$C_m$-$C_n$ cycloalkyl" should be understood to mean a saturated, unsaturated or partially saturated carbocyclic ring having m to n atoms. For example, "3- to 15-membered cycloalkyl" or "$C_3$-$C_{15}$ cycloalkyl" refers to a cyclic alkyl group containing 3 to 15, 3 to 9, 3 to 6 or 3 to 5 carbon atoms, which may contain 1 to 4 rings. "3- to 10-membered cycloalkyl" contains 3 to 10 carbon atoms. It includes a monocyclic, bicyclic, tricyclic, spiro or bridged ring. Examples of unsubstituted cycloalkyl include, but are not limited to, cyclopropyl, cyclobutyl, cyclopentyl, cyclohexyl and adamantyl, or bicyclic hydrocarbyl such as decahydronaphthalene ring. Cycloalkyl may be substituted by one or more than one substituent. In some embodiments, cycloalkyl may be aryl- or heteroaromatic ring group-fused cycloalkyl.

The term "spiro ring" alone or as part of another substituent refers to a polycyclic group that shares one carbon atom (called spiro atom) between monocyclic rings, which may contain one or more than one double bond, but none of the rings has a fully conjugated $\pi$-electron system. Spirocycloalkyl can be divided into monospirocycloalkyl, bispirocycloalkyl or multispirocycloalkyl according to the number of spiro atoms shared between rings, preferably monospirocycloalkyl and bispirocycloalkyl. Non-limiting examples of spirocycloalkyl include:

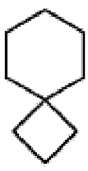 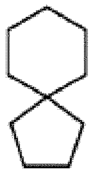 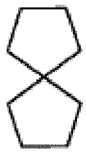 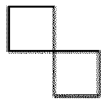 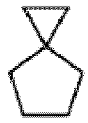

Spirocycloalkyl in which monospirocycloalkyl shares a spiro atom with heterocycloalkyl is also included. Non-limiting examples include:

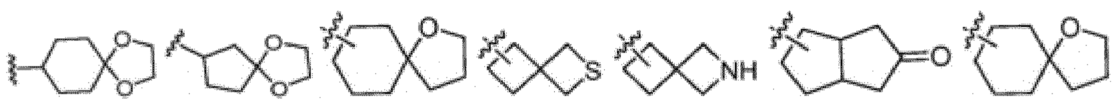

and

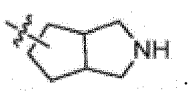

The term "bridged ring" refers to a cyclic hydrocarbon in which any two rings in a compound share two carbon atoms that are not directly connected, and can be divided into bicyclic hydrocarbon, tricyclic hydrocarbon, tetracyclic hydrocarbon, etc. according to the number of constituent rings. Non-limiting examples include:

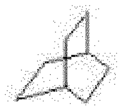 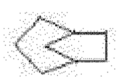 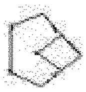 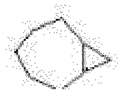

The term "heterocycloalkyl" or "heterocyclyl" or "heterocyclic ring" alone or as part of another substituent refers to cycloalkyl in which one or more than one (in some embodiments, 1 to 3) carbon atom is substituted by heteroatoms such as, but not limited to, N, O, S, and P. The term "m- to n-membered heterocycloalkyl" or "$C_m$-$C_n$ heterocycloalkyl" should be understood to mean a saturated, unsaturated or partially saturated ring having m to n atoms, wherein heterocyclic atom is selected from N, O, S, P, preferably selected from N, O or S. For example, the term "4- to 8-membered heterocycloalkyl" or "$C_4$-$C_8$ heterocycloalkyl" should be understood to mean a saturated, unsaturated or partially saturated ring having 4 to 8 atoms, wherein 1, 2, 3 or 4 ring atoms are selected from N, O, S, P, preferably selected from N, O or S. The term "9- to 11-membered heterocyclyl" refers to a saturated, unsaturated or partially saturated ring having 9 to 11 atoms. In some embodiments, heterocycloalkyl may be aromatic ring group- or heteroaromatic ring group-fused heterocycloalkyl. When a prefix such as 9- to 11-membered is used to indicate heterocycloalkyl, the number of carbons is also meant to include heteroatoms. It includes a monocyclic, bicyclic, tricyclic, spiro or bridged ring.

The term "heteroaromatic ring group" alone or as part of another substituent refers to a monocyclic or polycyclic aromatic ring system, and in some embodiments, 1 to 3 atoms in the ring system are heteroatoms, i.e., elements other than carbon, including but not limited to N, O, S or P. For example, furanyl, imidazolyl, dihydroindolyl, pyrrolidinyl, pyrimidinyl, tetrazolyl, thienyl, pyridinyl, pyrrolyl, *N*-methylpyrrolyl, quinolinyl and isoquinolinyl. The heteroaromatic ring group may optionally be fused with benzene ring, and may also include a monocyclic, bicyclic, tricyclic, spiro or bridged ring.

The term "9- to 11-membered heteroaromatic ring group" or "$C_9$-$C_{11}$ heteroaromatic ring group", alone or as part of another substituent, should be understood as a monovalent monocyclic, bicyclic or tricyclic aromatic ring group having 9 to 11 ring atoms and containing 1 to 3 heteroatoms independently selected from N, O and S, and should be understood as a monovalent monocyclic, bicyclic or tricyclic aromatic ring group having 9, 10 or 11 ring atoms and containing heteroatoms independently selected from N, O and S, and additionally, in each case may be benzofused. Alone or as part of another substituent, "9- to 11-membered heteroaromatic ring group" or "$C_9$-$C_{11}$ heteroaromatic ring group" may be attached through carbon or nitrogen, wherein the $-CH_2-$ group is optionally substituted by -C(O)-; and wherein, unless otherwise stated to the contrary, a ring nitrogen atom or ring sulfur atom is optionally oxidized to form an N-oxide or S-oxide, or the ring nitrogen atom is optionally quaternized; wherein -NH in the ring is optionally substituted by acetyl, formyl, methyl or methanesulfonyl; and wherein the ring is optionally substituted by one or more than one halogen. It should be understood that when the total number of S and O atoms in the heterocyclyl exceeds 1, these heteroatoms are not adjacent to each other. If the heterocyclyl is bicyclic or tricyclic, at least one ring may optionally be a heteroaromatic or aromatic ring, provided that at least one ring is non-heteroaromatic. If the heterocyclyl is monocyclic, it must not be aromatic.

The term "haloalkyl" alone or as part of another substituent refers to branched and linear saturated aliphatic hydrocarbyl having a specific number of carbon atoms and substituted by one or more than one halogen (e.g. -CvFw, where v = 1 to 3 and w = 1 to (2v + 1)). Examples of haloalkyl include, but are not limited to, trifluoromethyl, trichloromethyl, pentafluoroethyl, pentachloroethyl, 2,2,2-trifluoroethyl, heptafluoropropyl and heptachloropropyl.

The compounds provided herein, including intermediates useful in the preparation of the compounds provided herein, contain reactive functional groups (such as, but not limited to, carboxyl, hydroxyl and amino moieties), and also include protected derivatives (compounds) thereof. "Protected derivatives (compounds)" are those compounds in which one or more than one reactive site is blocked by one or more than one protecting group (also referred to as protective group). Suitable carboxyl protecting groups include benzyl, *tert*-butyl, etc., as well as isotopes, etc. Suitable amino and amido protecting groups include acetyl, trifluoroacetyl, *tert*-butoxycarbonyl, benzyloxycarbonyl, etc. Suitable hydroxyl protecting groups include benzyl, etc. Other suitable protecting groups are well known to those skilled in the art.

In the present disclosure, "optional" or "optionally" means that the subsequently described event or circumstance may or may not occur, and the description includes both the occurrence and non-occurrence of the event or circumstance. For example, "optionally substituted aryl" means that the aryl is substituted or unsubstituted, and the description includes both substituted and unsubstituted aryl.

In the present disclosure, the term "salt" or "pharmaceutically acceptable salt" includes a pharmaceutically acceptable acid addition salt and a pharmaceutically acceptable base addition salt. The term "pharmaceutically acceptable" refers to the compounds, materials, compositions and/or dosage forms that are suitable for use in contact with human and animal tissues without excess toxicity, irritation, allergic reactions or other problems or complications within the scope of reliable medical judgment, and are commensurate with a reasonable benefit/risk ratio.

"Pharmaceutically acceptable acid addition salt" refers to a salt formed with an inorganic or organic acid that retains the biological effectiveness of the free base without other side effects. "Pharmaceutically acceptable base addition salt" refers to a salt formed with an inorganic or organic base that retains the biological effectiveness of the free acid without other side effects. In addition to the pharmaceutically acceptable salts, other salts may be adopted in the present disclosure. The other salts can serve as intermediates in the purification of compounds or in the preparation of other pharmaceutically acceptable salts or can be used for identifying, characterizing, or purifying the compounds of the present disclosure.

The term "amine salt" refers to the product obtained by neutralizing alkyl primary amine, secondary amine or tertiary amine with an acid. The acid includes the inorganic acid or the organic acid as described in the present disclosure.

The term "stereoisomer" refers to an isomer produced by a different spatial arrangement of atoms in the molecule, including cis-trans isomers, enantiomers, diastereomers, and conformational isomers.

Depending on the selected raw materials and methods, the compounds of the present disclosure may exist in the form of one of the possible isomers or a mixture thereof, for example, as pure optical isomers, or as a mixture of isomers such as a mixture of racemic isomer and diastereoisomer, depending on the number of asymmetric carbon atoms. When describing optically active compounds, the prefixes D and L or R and S are used to denote the absolute configurations of the molecule with respect to chiral center(s) in the molecule. The prefixes D and L or (+) and (-) are symbols used to specify a rotation of plane-polarized light caused by a compound, where (-) or L indicates that the compound is levorotatory. The prefix (+) or D indicates that the compound is dextrorotatory.

When the bond with a chiral carbon in the formula of the present disclosure is depicted in a straight line, it should be understood that the two configurations (R) and (S) of the chiral carbon and both the resulting enantiomerically pure compound and mixture are included in the scope defined by the general formula. The graphical representation of the racemically or enantiomerically pure compound herein is from Maehr, J. Chem. Ed. 1985, 62: 114-120. The absolute configuration of a stereocenter is represented by wedge-shaped bonds and dashed-line bonds.

The term "tautomer" refers to an isomer of a functional group resulting from a rapid movement of an atom between two positions in a molecule. The compound of the present disclosure may exhibit tautomerism. Tautomeric compounds can be present in two or more mutually convertible species. Prototropic tautomer is resulted from a migration of covalently bonded hydrogen atoms between two atoms. The tautomer generally exist in an equilibrium form, and when trying to separate a single tautomer, a mixture is usually produced, the physical and chemical properties of which are consistent with the mixture of compounds. The position of equilibrium depends on the intramolecular chemical properties. For example, for many aliphatic aldehydes and ketones, such as acetaldehyde, the ketonic form is dominant; and for phenols, the enol form is dominant. All tautomeric forms of the compounds are included in the present disclosure.

The term "pharmaceutical composition" of the present disclosure refers to a formulation of the compound of the present disclosure with a medium generally accepted in the art for delivering a biologically active compound to a mammal (e.g., a human). The medium includes a pharmaceutically acceptable carrier. The purpose of the pharmaceutical composition is to promote the administration to an organism, facilitate the absorption of the active ingredient and thus exert its biological activity.

In the present disclosure, "pharmaceutically acceptable carrier" includes, but is not limited to, any acceptable adjuvants, carriers, excipients, glidants, sweeteners, diluents, preservatives, dyes/coloring agents, flavoring agents, surfactants, wetting agents, dispersants, suspending agents, stabilizers, isotonic agents, solvents or emulsifiers for humans or livestocks as licensed by relevant governmental administrations.

The term "solvate" refers to the compound of the present disclosure or a salt thereof including a stoichiometric or non-stoichiometric solvent bonded through an intermolecular non-covalent force. When the solvent is water, the solvate is a hydrate.

The term "prodrug" can be converted into the compound of the present disclosure having biological activity under physiological conditions or through solvolysis. The prodrug of the present disclosure is prepared by modifying the functional groups in the compound, and the modification can be removed by conventional operations or *in vivo,* so as to obtain the parent compound. The prodrug includes a compound formed by attaching a hydroxyl or amino group in the compound of the present disclosure to any group. When the prodrug of the compound of the present disclosure is administered to a mammal individual, the prodrug is dissociated to form a free hydroxyl group and a free amino group respectively.

The term "excipient" refers to a pharmaceutically acceptable inert ingredient. Examples of categories of the term "excipient" include, but are not limited to, binders, disintegrants, lubricants, glidants, stabilizers, fillers, diluents, etc. Excipients can enhance operation properties of the pharmaceutical formulation, i.e., allowing the formulation to be more suitable for direct compression by increasing fluidity and/or adhesion.

As used herein, the term "treatment" and other similar synonyms include the following meanings:

(i) preventing the occurrence of a disease or condition in mammals, particularly when such mammals are susceptible to the disease or condition but have not been diagnosed as having the disease or condition;
(ii) inhibiting the disease or condition, i.e., restraining its development;
(iii) ameliorating the disease or condition, i.e., causing the disease or condition to subside; or
(iv) alleviating the symptoms caused by the disease or condition.

For the reaction of each step, the reaction temperature can be appropriately selected according to the solvent, starting material, reagent, etc., and the reaction time can also be appropriately selected according to the reaction temperature, solvent, starting material, reagent, etc. After the reaction of each step, the target compound can be separated and purified from the reaction system by common methods, such as filtration, extraction, recrystallization, washing, silica gel column chromatography and other methods. Without affecting the next reaction step, the target compound can also be directly used in the next reaction step without separation and purification.

The above preferred conditions can be combined arbitrarily to obtain preferred embodiments of the present disclosure without violating common knowledge in the art. The reagents and raw materials used in the present disclosure are all commercially available.

**Beneficial effects**

After extensive and in-depth research, the present inventors unexpectedly developed a pyridopyrimidinone derivative (compound) or a pharmaceutically acceptable salt thereof, a preparation method therefor and a use thereof.

The present disclosure provides the pyridopyrimidinone derivative (compound) as represented by formula I, the tautomer thereof, the stereoisomer thereof, the hydrate thereof, the solvate thereof, the pharmaceutically acceptable salt thereof or the prodrug thereof, the compound as represented by formula I shows a significant inhibitory effect on KRAS G12C::SOS1 binding, a significant inhibitory effect on KRAS G12C-SOS1, a significant inhibitory effect on the ERK phosphorylation level in DLD-1 cells, and a strong inhibitory effect on the 3D proliferation of H358 cells; the compound of the present disclosure exhibits good hepatic metabolic stability, slow metabolism in the human body, and high exposure; no inhibitory effect on CYP3A4 enzymes, low risk of potential drug-drug interactions, excellent pharmacokinetic properties, high safety and druggability, and is more suitable for combination medication. Experiments indicate that the compound of the present disclosure, alone or in combination with trametinib, has a significant inhibitory effect on Mia Paca-2 tumor growth, and the combined use is more effective than the single use.

The present disclosure provides a method for preparing the pyridopyrimidinone derivative as represented by formula I, the tautomer thereof, the stereoisomer thereof, the hydrate thereof, the solvate thereof, the pharmaceutically acceptable salt thereof or the prodrug thereof, and the intermediate thereof. The method is simple in operation, high in yield and high in purity, and can be used in the industrialized production of medicines.

BRIEF DESCRIPTION OF THE DRAWINGS

FIG. 1 shows the tumor inhibitory ability of representative compounds at the tumor volume level (tumor volume ($mm^3$) at the end of treatment) tested in Mia Paca-2 pancreatic cancer *in vivo* efficacy experiment.

FIG. 2 shows the tumor inhibitory ability of representative compounds at the tumor weight level (tumor volume ($mm^3$) at the end of treatment) tested in LOVO colorectal cancer *in vivo* efficacy experiment.

DETAILED DESCRIPTION OF THE PREFERRED EMBODIMENT

The present disclosure is further illustrated below in conjunction with specific embodiments. It should be understood that the following description is only the most preferred embodiment of the present disclosure and should not be construed as limiting the scope of protection of the present disclosure. On the basis of a full understanding of the present disclosure, the experimental methods without indication of specific conditions in the following embodiments shall be implemented usually in accordance with conventional conditions or the conditions suggested by the manufacturer. Those skilled in the art can make non-essential modifications to the technical solutions of the present disclosure, and such modifications should be considered to be included in the scope of protection of the present disclosure.

**The abbreviations in the present disclosure are defined as follows:**

Symbols or units:

$IC_{50}$: half inhibitory concentration, which refers to a concentration at which half of the maximum inhibitory effect is reached

M: mol/L, for example, n-butyllithium (14.56 mL, 29.1 mmol, 2.5 M n-hexane) means a solution of n-butyllithium in n-hexane with a molar concentration of 2.5 mol/L

N: equivalent concentration, for example, 2 N hydrochloric acid means 2 mol/L hydrochloric acid solution

Reagents:

DCM: dichloromethane

DIPEA: also referred to as DIEA, diisopropylethylamine, i.e., *N,N*-diisopropylethylamine

DMF: *N*,*N*-dimethylformamide

DMSO: dimethyl sulfoxide

EA: ethyl acetate

$Et_3N$: triethylamine

MeOH: methanol

PE: petroleum ether

THF: tetrahydrofuran

Test or detection methods:
HPLC: high performance liquid chromatography
SFC: supercritical fluid chromatography

Acidic preparation condition B:

Welch, Ultimate C18 column, 10 μm, 21.2 mm × 250 mm. Mobile phase A is a 1‰ solution of formic acid in pure water, and mobile phase B is an acetonitrile solution. Gradient conditions: mobile phase A was kept at 90% (0 to 3 min), then reduced from 90% to 5% by gradient elution (3 to 18 min), and kept at 5% (18 to 22 min).

Intermediate **A1**: Preparation of intermediate **A1**

The synthetic route is as follows:

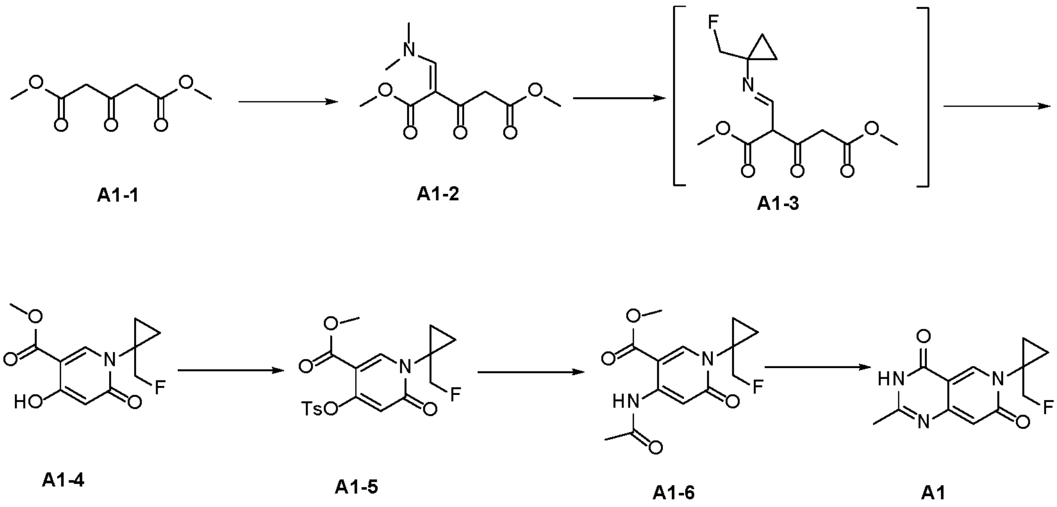

Step 1: Methyl (Z)-2-((dimethylamino)methylene)-3-oxoglutarate (**A1-2**)

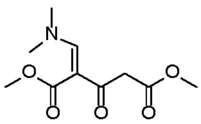

A1-2

To 2-methyltetrahydrofuran (100 mL) was added compound methyl 3-oxoglutarate (10.0 g, 57.4 mmol) at room temperature, then DMF-DMA (6.8 g, 57.1 mmol) was added thereto, and the reaction mixture was stirred at room temperature for 4 h. The reaction mixture was concentrated, and the residue was purified by silica gel column chromatography (petroleum ether:ethyl acetate (V/V) = 1:1) to obtain a crude product of the title compound methyl (Z)-2-((dimethylamino)methylene)-3-oxoglutarate **(A1-2)** (12 g, yield: 91.1%) as a yellow liquid.

LC-MS, M/Z (ESI): 230.2 [M+H]$^+$.

Step 2: Methyl 1-(1-(fluoromethyl)cyclopropyl)-4-hydroxy-6-oxo-1,6-dihydroxypyridine-3-carboxylate (**A1-4**)

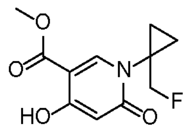

**A1-4**

To 2-methyltetrahydrofuran (30 mL) was added compound methyl (*Z*)-2-((dimethylamino)methylene)-3-oxoglutarate (2.4 g, 10.4 mmol) at room temperature, then 4 N hydrochloric acid (10 mL) was added thereto, and the reaction mixture was stirred for 3 h. The liquid was separated, and the aqueous phase was extracted with ethyl acetate (100 mL × 3). The organic phases were combined, dried over anhydrous sodium sulfate, filtered and concentrated, added with methanol (30 mL), then added with 1-(fluoromethyl)cyclopropane-1-amine hydrochloride (1.0 g, 8.0 mmol), and the mixture was stirred at room temperature for 16 h. The system was added with sodium methoxide (1.3 g, 24.0 mmol), and stirred for 2 h. The reaction mixture was added with concentrated hydrochloric acid to adjust the pH to 2, and filtered to obtain a crude product of the title compound methyl 1-(1-(fluoromethyl)cyclopropyl)-4-hydroxy-6-oxo-1,6-dihydroxypyridine-3-carboxylate (**A1-4**) (2.0 g, yield: 79.1%) as a brown solid.

LC-MS, M/Z (ESI): 242.2 $[M+H]^+$.

Step 3: Methyl 1-(1-(fluoromethyl)cyclopropyl)-6-oxo-4-(p-toluenesulfonyloxy)-1,6-dihydroxypyridine-3-carboxylate (**A1-5**)

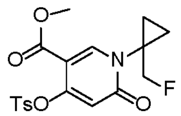

**A1-5**

To acetonitrile (20 mL) was added raw material methyl 1-(1-(fluoromethyl)cyclopropyl)-4-hydroxy-6-oxo-1,6-dihydroxypyridine-3-carboxylate (2.0 g, 8.3 mmol) at room temperature, the reaction mixture was cooled to 0°C, added with triethylamine (1.68 g, 16.6 mmol) and TsCl (1.58 g, 8.3 mmol), heated to room temperature, and stirred for 2 h. The reaction mixture was concentrated and purified by silica gel column chromatography (petroleum ether:ethyl acetate (V/V) = 5:1 to 1:1) to obtain the title compound methyl 1-(1-(fluoromethyl)cyclopropyl)-6-oxo-4-(p-toluenesulfonyloxy)-1,6-dihydroxypyridine-3-carboxylate (**A1-5**) (1.2 g, yield: 36.6%) as a white solid.

LC-MS, M/Z (ESI): 396.3 $[M+H]^+$.

Step 4: Methyl 4-acetamido-1-(1-(fluoromethyl)cyclopropyl)-6-oxo-1,6-dihydroxypyridine-3-carboxylate (**A1-6**)

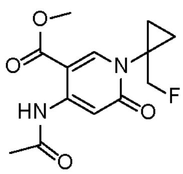

**A1-6**

To dioxane (50 mL) was added raw material methyl 1-(1-(fluoromethyl)cyclopropyl)-6-oxo-4-(*p*-toluenesulfonyloxy)-1,6-dihydroxypyridine-3-carboxylate (1.2 g, 3.0 mmol) at room temperature, the reaction mixture was added with potassium phosphate (700 mg, 3.3 mmol), Xantphos (173 mg, 0.3 mmol) and palladium (II) ($\pi$-cinnamyl) chloride dimer (212 mg, 0.3 mmol), heated and stirred at reflux for 2 h under nitrogen atmosphere. The reaction mixture was cooled to room temperature, concentrated and purified by silica gel column chromatography (petroleum ether: ethyl acetate (V/V) = 3:1 to 1:1) to obtain the title compound methyl 4-acetamido-1-(1-(fluoromethyl)cyclopropyl)-6-oxo-1,6-dihydroxypyridine-3-carboxylate (**A1-6**) (680 mg, yield: 79.3%) as a white solid.

LC-MS, M/Z (ESI): 283.2 [M+H]+.

Step 5: 6-(1-(Fluoromethyl)cyclopropyl)-4-hydroxy-2-methylpyrido[4,3-d]pyrimidin-7(6H)-one (**A1**)

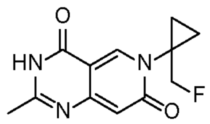

**A1**

To a 7 mol/L solution of ammonia in methanol (10 mL) was added raw material methyl 4-acetamido-1-(1-(fluoromethyl)cyclopropyl)-6-oxo-1,6-dihydroxypyridine-3-carboxylate (680 mg, 2.41 mmol) at room temperature, and the reaction mixture was stirred at room temperature for 5 d. The reaction mixture was concentrated to 3 mL and filtered to obtain the title compound 6-(1-(fluoromethyl)cyclopropyl)-4-hydroxy-2-methylpyrido[4,3-d]pyrimidin-7(6H)-one (460 mg, yield: 16.4%) as a white solid.
$^1$H NMR (400 MHz, DMSO-*$d_6$*) δ 11.8 (s, 1H), 8.36 (s, 1H), 6.17 (s, 1H), 4.62 (d, 2H), 2.24 (s, 3H), 1.27 (s, 4H).
LC-MS, M/Z (ESI): 250.2 [M+H]+.

Intermediate **A2:** Preparation of intermediate **A2**

The synthetic route is as follows:

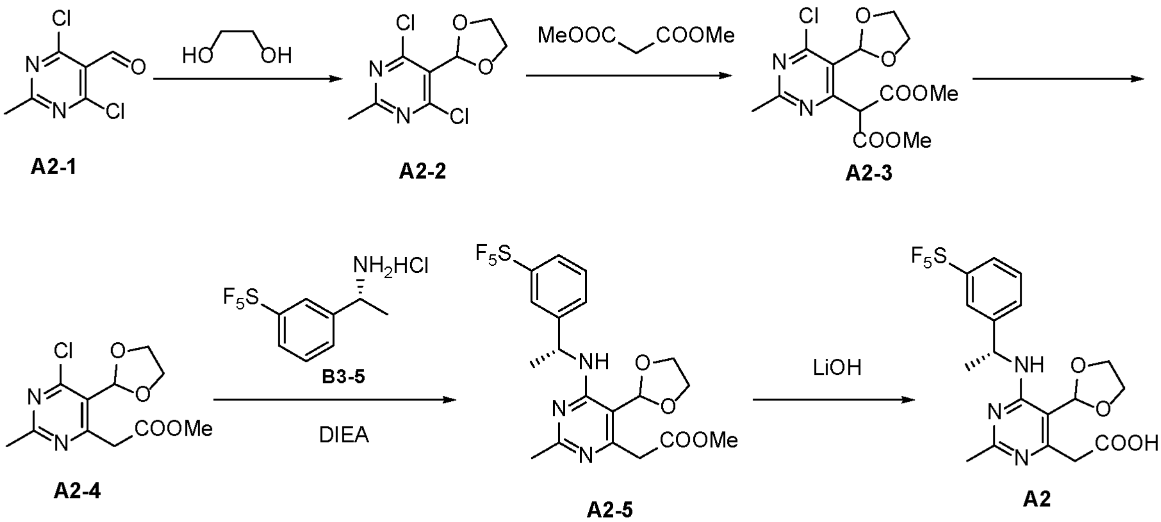


Step 1: Synthesis of 4,6-dichloro-5-(1,3-dioxolan-2-yl)-2-methylpyrimidine **(A2-2)**

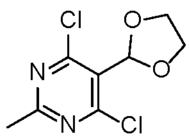

**A2-2**

To toluene (200 mL) was added 4,6-dichloro-2-methylpyrimidine-5-carbaldehyde (20 g, 105 mmol), the reaction mixture was added with ethylene glycol (5.84 mL) and *p*-toluenesulfonic acid (2 g, 10.5 mmol), and refluxed at 120°C for 12 h. After the reaction was completed, the reaction mixture was concentrated, added with dichloromethane (200 mL), and washed with saturated sodium bicarbonate solution (200 mL × 3). The organic phase was concentrated, and

the residue was purified by silica gel column chromatography (petroleum ether:ethyl acetate (V/V) = 10:1) to obtain the title compound 4,6-dichloro-5-(1,3-dioxolan-2-yl)-2-methylpyrimidine **(A2-2)** (11 g, yield: 44.6%).
LC-MS, M/Z (ESI): 235.0 $[M+H]^+$.

Step 2: Synthesis of dimethyl 2-(6-chloro-5-(1,3-dioxolan-2-yl)-2-methylpyrimidin-4-yl)malonate **(A2-3)**

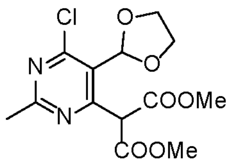

**A2-3**

To dimethyl sulfoxide (50 mL) was added 4,6-dichloro-5-(1,3-dioxolan-2-yl)-2-methylpyrimidine (10 g, 42.5 mmol), the reaction mixture was added with cesium carbonate (27.7 g, 85 mmol) and dimethyl malonate (6.18 g, 46.8 mmol), and stirred at 80°C for 12 h. After the reaction was completed, the reaction mixture was diluted with ethyl acetate (200 mL), and washed with water (200 mL × 3) and saturated brine (200 mL × 3). The organic phase was concentrated, and the residue was purified by silica gel column chromatography (petroleum ether:ethyl acetate (V/V) = 3:1) to obtain the title compound dimethyl 2-(6-chloro-5-(1,3-dioxolan-2-yl)-2-methylpyrimidin-4-yl)malonate **(A2-3)** (10 g, yield: 71%).
LC-MS, M/Z (ESI): 331.1 $[M+H]^+$.

Step 3: Synthesis of methyl 2-(6-chloro-5-(1,3-dioxolan-2-yl)-2-methylpyrimidin-4-yl)acetate **(A2-4)**

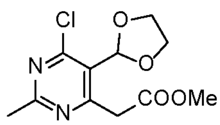

**A2-4**

To dimethyl sulfoxide (30 mL) was added dimethyl 2-(6-chloro-5-(1,3-dioxolan-2-yl)-2-methylpyrimidin-4-yl)malonate (10.2 g, 30.8 mmol), the reaction mixture was added with lithium chloride (5.23 g, 123 mmol), and stirred at 120°C for 12 h. After the reaction was completed, the reaction mixture was diluted with ethyl acetate (100 mL), and washed with water (200 mL × 3) and saturated brine (200 mL × 3). The organic phase was concentrated, and the residue was purified by silica gel column chromatography (petroleum ether:ethyl acetate (V/V) = 3:1) to obtain the title compound methyl 2-(6-chloro-5-(1,3-dioxolan-2-yl)-2-methylpyrimidin-4-yl)acetate **(A2-4)** (6 g, yield: 71.3%).
LC-MS, M/Z (ESI): 273.1 $[M+H]^+$.

Step 4: Synthesis of methyl (R)-2-(5-(1,3-dioxolan-2-yl)-2-methyl-6-((1-(3-(pentafluoro-$\lambda^6$-sulfanyl)phenyl)ethyl)amino)pyrimidin-4-yl)acetate **(A2-5)**

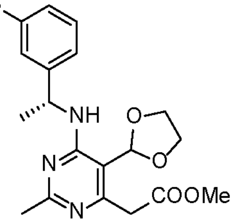

**A2-5**

To dimethyl sulfoxide (25 mL) was added methyl 2-(6-chloro-5-(1,3-dioxolan-2-yl)-2-methylpyrimidin-4-yl)acetate (2.5 g, 9.17 mmol), the reaction mixture was added with *N,N*-diisopropylethylamine (4.8 mL, 27.5 mmol) and (R)-1-(3-(pentafluorosulfanyl)phenyl)ethan-1-amine hydrochloride (3.12 g, 11 mmol), and stirred at 80°C for 6 h. After the reaction was completed, the reaction mixture was diluted with ethyl acetate (100 mL), and washed with water (100 mL $\times$ 3) and saturated brine (100 mL $\times$ 3). The organic phase was concentrated, and the residue was purified by silica gel column chromatography (petroleum ether:ethyl acetate (V/V) = 1:1) to obtain the title compound methyl (R)-2-(5-(1,3-dioxolan-2-yl)-2-methyl-6-((1-(3-(pentafluorosulfanyl)phenyl)ethyl)amino)pyrimidin-4-yl)acetate **(A2-5)** (2.3 g, yield: 51.9%).

LC-MS, M/Z (ESI): 484.1 $[M+H]^+$.

Step 5: Synthesis of (R)-2-(5-(1,3-dioxolan-2-yl)-2-methyl-6-((1-(3-(pentafluorosulfanyl)phenyl)ethyl)amino)pyrimidin-4-yl)acetic acid **(A2)**

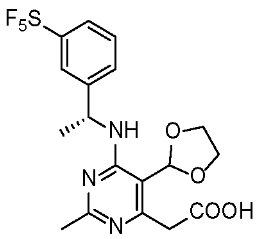

**A2**

To a mixed solution of dimethyl sulfoxide (25 mL) and acetonitrile (1 mL) was added methyl (R)-2-(5-(1,3-dioxolan-2-yl)-2-methyl-6-((1-(3-(pentafluorosulfanyl)phenyl)ethyl)amino)pyrimidin-4-yl)acetate (250 mg, 0.52 mmol), the reaction mixture was added with sodium hydroxide (83 mg, 2.07 mmol), and stirred at room temperature for 1 h. After the reaction was completed, the reaction mixture was directly concentrated and dried to obtain methyl (R)-2-(5-(1,3-dioxolan-2-yl)-2-methyl-6-((1-(3-(pentafluorosulfanyl)phenyl)ethyl)amino)pyrimidin-4-yl)acetic acid **(A2)** (243 mg, yield: 100%).

LC-MS, M/Z (ESI): 470.1 $[M+H]^+$.

**Embodiment 1: Synthesis of compound I-1**

The synthetic route is as follows:

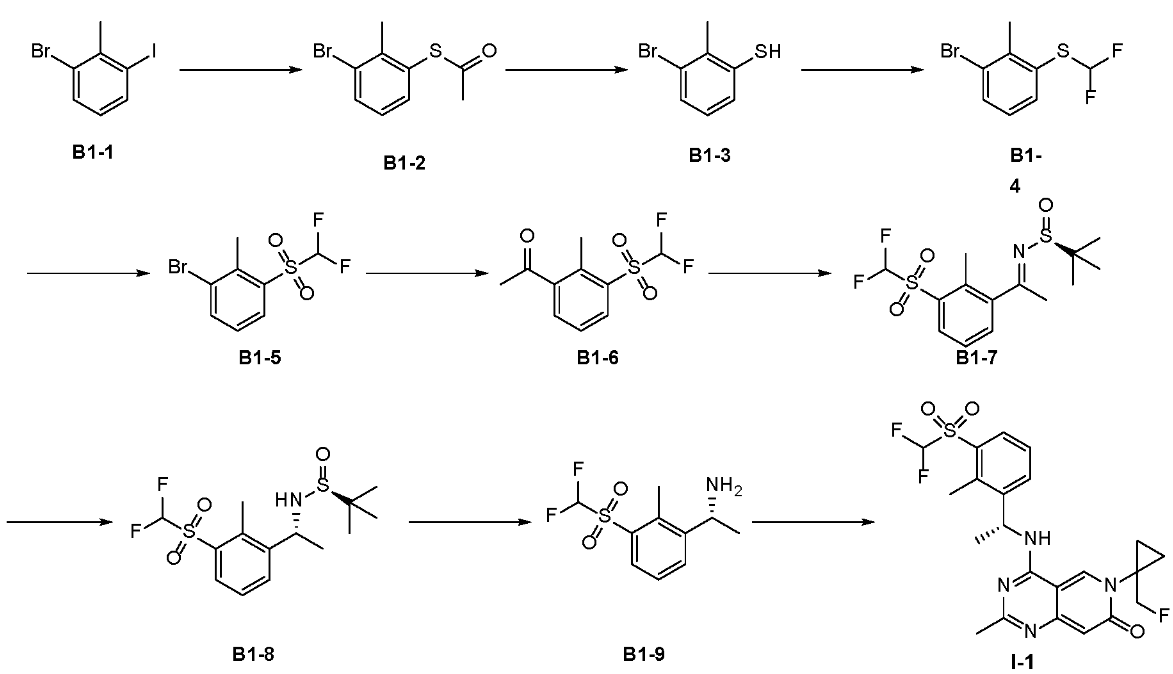

B1-1 B1-2 B1-3 B1-4

B1-5 B1-6 B1-7

B1-8 B1-9 I-1

Step 1: Synthesis of S-(3-bromo-2-methylphenyl)acetylmercaptoester (B1-2)

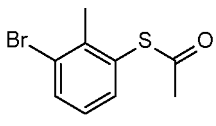

B1-2

To anhydrous toluene (20 mL) was added 1-bromo-3-iodo-2-toluene (2.0 g, 6.7 mmol) at room temperature, the reaction mixture was added with potassium thioacetate (1.2 g, 10.5 mmol), 1,10-phenanthroline (120 mg, 0.67 mmol) and cuprous iodide (260 mg, 1.4 mmol), heated to 100°C under nitrogen atmosphere, and stirred for 3 h. The reaction mixture was cooled to room temperature, added with water (50 mL), extracted with EA (80 mL × 3), and the liquid was separated. The organic phases were combined, dried over anhydrous sodium sulfate, filtered, concentrated, and the residue was purified by silica gel column chromatography (petroleum ether) to obtain S-(3-bromo-2-methylphenyl)acetylmercaptoester (B1-2, a crude product as colorless liquid, 1.0 g, yield: 42.0%).

LC-MS, M/Z (ESI): 245.0 $[M+H]^+$.

Step 2: Synthesis of 3-bromo-2-methylbenzenethiol (B 1-3)

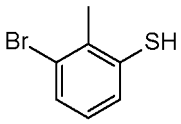

B1-3

To methanol (20 mL) and THF (20 mL) were added S-(3-bromo-2-methylphenyl)acetylmercaptoester (2.44 g, 10.0 mmol) at room temperature, the reaction mixture was added with potassium hydroxide (670 mg, 12.0 mmol), and stirred at room temperature for 0.5 h. The reaction mixture was concentrated to obtain 3-bromo-2-methylbenzenethiol (B1-3, a crude product as yellow solid, 2.10 g).

LC-MS, M/Z (ESI): 202.9 $[M+H]^+$.

Step 3: Synthesis of (3-bromo-2-methylphenyl)(difluoromethyl)sulfide (B1-4)

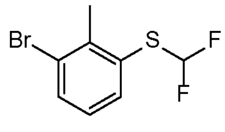

**B1-4**

To acetonitrile (20 mL) was added 3-bromo-2-methylbenzenethiol (2.1 g, 10.0 mmol) at room temperature, the reaction mixture was added with potassium carbonate (2.8 g, 20.0 mmol) and sodium bromodifluoroacetate (3.0 g, 15.0 mmol), heated to 100°C and stirred for 1 h. The reaction mixture was cooled to room temperature and filtered to obtain a solution of compound B1-4.
$^{19}F$ NMR (400 MHz, $CDCl_3$) $\delta$ -92.36.
LC-MS, M/Z (ESI): 252.9 $[M+H]^+$.

Step 4: Synthesis of 1-bromo-3-((difluoromethyl)sulfonyl)-2-methylbenzene (B1-5)

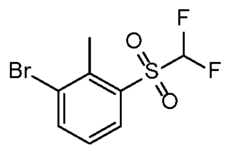

**B1-5**

To acetonitrile (40 mL), carbon tetrachloride (40 mL) and water (80 mL) were added the solution of compound B1-4 at room temperature, the reaction mixture was added with ruthenium trichloride (2.1 g, 10.0 mmol) and sodium periodate (6.5 g, 30.0 mmol), and stirred for 16 h. The reaction mixture was diluted with water (400 mL), extracted with DCM (100 mL × 3), and the liquid was separated. The organic phases were combined, dried over anhydrous sodium sulfate, filtered, concentrated, and the residue was purified by silica gel column chromatography (petroleum ether:ethyl acetate (V/V) = 10:1) to obtain 1-bromo-3-((difluoromethyl)sulfonyl)-2-methylbenzene (B1-5, colorless liquid, 710 mg, yield: 24.9%).
LC-MS, M/Z (ESI): 284.9 $[M+H]^+$.

Step 5: Synthesis of 1-(3-((difluoromethyl)sulfonyl)-2-methylphenyl)ethan-1-one (B1-6)

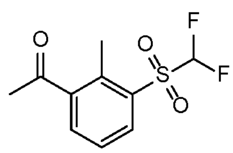

**B1-6**

To dioxane (50 mL) was added compound 1-bromo-3-((difluoromethyl)sulfonyl)-2-methylbenzene (700 mg, 3.07 mmol) at room temperature, the reaction mixture was added with bis(triphenylphosphine)palladium(II) chloride (431 mg, 0.61 mmol) and tributyl(1-ethoxyvinyl)tin (2.21 g, 6.14 mmol), heated to 90°C under nitrogen atmosphere, and stirred for 14 h. The reaction mixture was cooled to room temperature, added with 2 N hydrochloric acid (30 mL), and stirred for 4 h. The reaction mixture was extracted with ethyl acetate (50 mL × 3), and the liquid was separated. The organic phases were combined, dried over anhydrous sodium sulfate, filtered, concentrated, and the residue was purified by silica gel column chromatography (petroleum ether: ethyl acetate (V/V) = 10:1) to obtain 1-(3-((difluoromethyl)sulfonyl)-2-methylphenyl)ethan-1-one (B1-6, colorless liquid, 450 mg, yield: 74.6%).
LC-MS, M/Z (ESI): 249.0 $[M+H]^+$.

Step 6: Synthesis of (S,E)-N-(1-(3-((difluoromethyl)sulfonyl)-2-methylphenyl)ethylidene)-2-methylpropane-2-sulfinamide (B1-7)

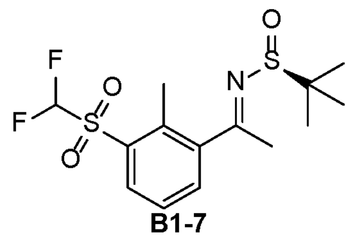

**B1-7**

To THF (100 mL) was added compound 1-(3-((difluoromethyl)sulfonyl)-2-methylphenyl)ethan-1-one (650 mg, 2.60 mmol) at room temperature, the reaction mixture was added with (S)-*tert*-butylsulfinamide (475 mg, 3.93 mmol) and tetraethyl titanate (1.18 g, 5.20 mmol), heated to 70°C and stirred for 16 h. The reaction mixture was cooled to room temperature, diluted with water (200 mL), extracted with ethyl acetate (100 mL × 3), and the liquid was separated. The organic phases were combined, dried over anhydrous sodium sulfate, filtered, concentrated, and the residue was purified by silica gel column chromatography (petroleum ether: ethyl acetate (V/V) = 4:1) to obtain (S,E)-N-(1-(3-((difluoromethyl)sulfonyl)-2-methylphenyl)ethylidene)-2-methylpropane-2-sulfinamide (B1-7, white solid, 900 mg, yield: 100%).
LC-MS, M/Z (ESI): 352.3 $[M+H]^+$.

Step 7: Synthesis of (S)-N-((R)-1-(3-((difluoromethyl)sulfonyl)-2-methylphenyl)ethyl)-2-methylpropane-2-sulfinamide (B1-8)

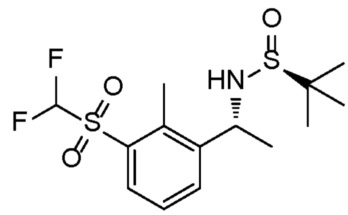

**B1-8**

To methanol (20 mL) was added raw material (S,E)-N-(1-(3-((difluoromethyl)sulfonyl)-2-methylphenyl)ethylidene)-2-methylpropane-2-sulfinamide (900 mg, 2.56 mmol) at room temperature, and the reaction mixture was cooled to 0°C. The $NaBH_4$ (474 mg, 12.8 mmol) was added into methanol in batches, and the reaction mixture was heated to room temperature and stirred for 3 h. The reaction mixture was concentrated and purified by preparative thin-layer chromatography to obtain (S)-N-((R)-1-(3-((difluoromethyl)sulfonyl)-2-methylphenyl)ethyl)-2-methylpropane-2-sulfinamide (B1-8, white solid, 500 mg, yield: 55.3%).
LC-MS, M/Z (ESI): 354.1 $[M+H]^+$.

Step 8: Synthesis of (R)-1-(3-((difluoromethyl)sulfonyl)-2-methylphenyl)ethan-1-amine hydrochloride (B1-9)

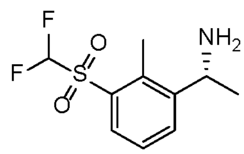

**B1-9**

To a 4 mol/L solution of hydrochloric acid in dioxane (1 mL) was added raw material (S)-N-((R)-1-(3-((difluoromethyl)sulfonyl)-2-methylphenyl)ethyl)-2-methylpropane-2-sulfinamide (350 mg, 1.0 mmol) at room temperature, and the reaction mixture was stirred for 4 h. The reaction mixture was concentrated, added with methyl tert-butyl ether (20 mL), stirred for 1 h, and filtered to obtain (R)-1-(3-((difluoromethyl)sulfonyl)-2-methylphenyl)ethan-1-amine hydrochloride (B1-9, white solid, 260 mg, yield: 100%).

LC-MS, M/Z (ESI): 250.2 $[M+H]^+$.

Step 9: Synthesis of (R)-4-((1-(3-((difluoromethyl)sulfonyl)-2-methylphenyl)ethyl)amino)-6-(1-(fluoromethyl)cyclopropyl)-2-methylpyrido[4,3-d]pyrimidin-7(6H)-one (I-1)

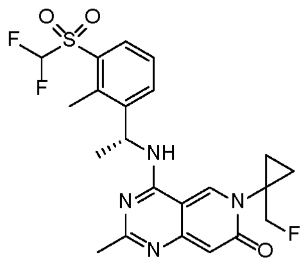

**I-1**

To acetonitrile (20 mL) was added raw material 6-(1-(fluoromethyl)cyclopropyl)-4-hydroxy-2-methylpyrido[4,3-d]pyrimidin-7(6H)-one (**A1**) (200 mg, 0.80 mmol) at room temperature, the reaction mixture was added with potassium phosphate (678 mg, 3.20 mmol) and phosphonitrilic chloride trimer (416 mg, 1.20 mmol), and stirred at room temperature for 16 h. To DCM (10 mL) was added raw material (R)-1-(3-((difluoromethyl)sulfonyl)-2-methylphenyl)ethan-1-amine hydrochloride (200 mg, 0.87 mmol), the reaction mixture was added with DIPEA (2 mL), and stirred for 0.5 h. The above system was added with the reaction mixture, and stirred at room temperature for 6 h. The reaction mixture was concentrated and prepared under acidic preparation condition B to obtain (R)-4-((1-(3-((difluoromethyl)sulfonyl)-2-methylphenyl)ethyl)amino)-6-(1-(fluoromethyl)cyclopropyl)-2-methylpyrido[4,3-d]pyrimidin-7(6H)-one (I-1, white solid, 26 mg, yield: 6.7%).

$^1H$ NMR (400 MHz, DMSO-$d_6$) δ 9.17 (s, 1H), 9.03 (d, 1H), 7.92 (t, 2H),7.59 (t, 1H), 7.46 (t, 1H), 6.05 (s, 1H), 5.67 (q, 1H), 4.73 (t, 2H), 2.83 (s, 3H), 2.14 (s, 3H), 1.56 (d, 3H), 1.33 (s, 4H).

LC-MS, M/Z (ESI): 481.2 $[M+H]^+$.

**Embodiment 2: Synthesis of compound I-2**

The synthetic route is as follows:

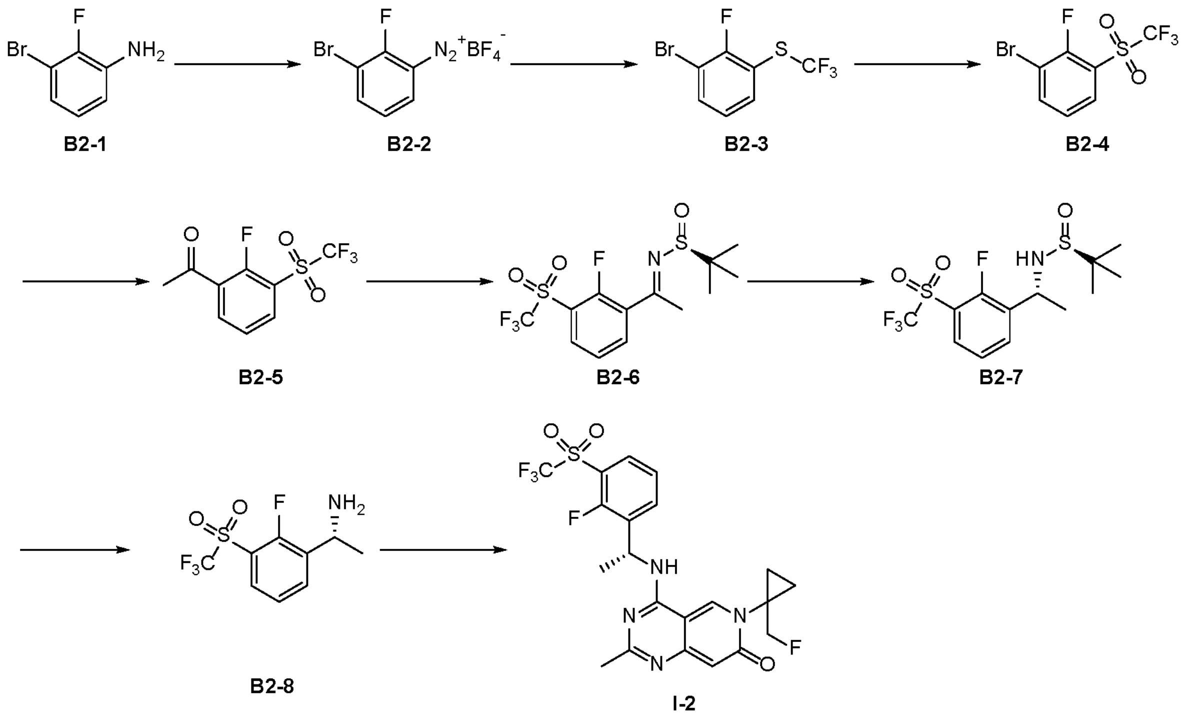

B2-1 B2-2 B2-3 B2-4

B2-5 B2-6 B2-7

B2-8 I-2

Step 1: Synthesis of 3-bromo-2-fluorobenzene-1-diazonium tetrafluoroborate (B2-2)

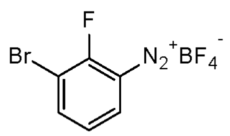

B2-2

To 50% tetrafluoroboric acid aqueous solution (21 mL) was added 3-bromo-2-fluoroaniline (10.3 g, 54.2 mmol) at room temperature, the reaction mixture was cooled to 0°C, and stirred for 1 h. A solution of sodium nitrite (3.8 g, 55 mmol) dissolved in water (6 mL) was added dropwise thereto at 0°C, and the reaction mixture was stirred continuously at low temperature for 1 h. The reaction mixture was filtered and dried to obtain 3-bromo-2-fluorobenzene-1-diazonium tetrafluoroborate (B2-2, a crude product as yellow solid, 13.0 g, yield: 83.2%).

Step 2: Synthesis of (3-bromo-2-fluorophenyl)(trifluoromethyl)sulfide (B2-3)

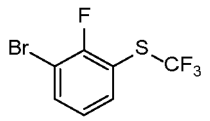

B2-3

To acetonitrile (130 mL) was added 3-bromo-2-fluorobenzene-1-diazonium tetrafluoroborate (13 g, 45.1 mmol) at room temperature, the reaction mixture was added with cesium carbonate (30 g, 91.0 mmol), sodium thiocyanate (5.5 g, 67.9 mmol) and cuprous thiocyanate (2.8 g, 23.0 mmol), stirred for 0.5 h, then added with trifluoromethyltrimethylsilane (12.8 g, 90 mmol), and stirred for 16 h. The reaction mixture was filtered to obtain a solution of compound B2-3.

LC-MS, M/Z (ESI): 274.9 $[M+H]^+$.

Step 3: Synthesis of 1-bromo-2-fluoro-3-((trifluoromethyl)sulfonyl)benzene (B2-4)

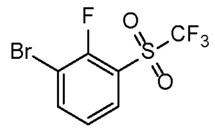

**B2-4**

To acetonitrile (200 mL), carbon tetrachloride (200 mL) and water (400 mL) were added the solution of compound B2-3 from the previous step at room temperature, the reaction mixture was added with ruthenium trichloride (9.3 g, 45.0 mmol) and sodium periodate (28.8 g, 134.5 mmol), and stirred for 16 h. The reaction mixture was diluted with water (800 mL), extracted with DCM (400 mL $\times$ 3), and the liquid was separated. The organic phases were combined, dried over anhydrous sodium sulfate, filtered, concentrated, and the residue was purified by silica gel column chromatography (petroleum ether: ethyl acetate (V/V) = 10:1) to obtain 1-bromo-2-fluoro-3-((trifluoromethyl)sulfonyl)benzene (B2-4, colorless liquid, 6.5 g, yield: 46.9%).

LC-MS, M/Z (ESI): 306.9 $[M+H]^+$.

Step 4: Synthesis of 1-(2-fluoro-3-((trifluoromethyl)sulfonyl)phenyl)ethan-1-one (B2-5)

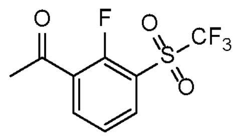

**B2-5**

To dioxane (150 mL) was added compound 1-bromo-2-fluoro-3-((trifluoromethyl)sulfonyl)benzene (6.5 g, 21.2 mmol) at room temperature, the reaction mixture was added with bis(triphenylphosphine)palladium(II) chloride (1.5 g, 2.12 mmol) and tributyl(1-ethoxyvinyl)tin (11.5 g, 31.7 mmol), heated to 90°C under nitrogen atmosphere, and stirred for 14 h. The reaction mixture was cooled to room temperature, added with 2 N hydrochloric acid (100 mL), and stirred for 4 h. The reaction mixture was extracted with ethyl acetate (200 mL $\times$ 3), and the liquid was separated. The organic phases were combined, dried over anhydrous sodium sulfate, filtered, concentrated, and the residue was purified by silica gel column chromatography (petroleum ether: ethyl acetate (V/V) = 10:1) to obtain 1-(2-fluoro-3-((trifluoromethyl)sulfonyl)phenyl)ethan-1-one (B2-5, colorless liquid, 5.0 g, yield: 87.7%).

LC-MS, M/Z (ESI): 271.0 $[M+H]^+$.

Step 5: Synthesis of (S,E)-N-(1-(2-fluoro-3-((trifluoromethyl)sulfonyl)phenyl)ethylidene)-2-methylpropane-2-sulfmamide (B2-6)

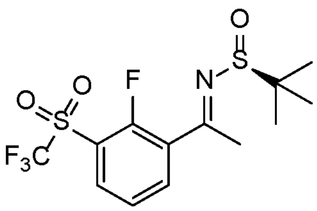

**B2-6**

To THF (150 mL) was added compound 1-(2-fluoro-3-((trifluoromethyl)sulfonyl)phenyl)ethan-1-one (2.7 g, 10.0 mmol) at room temperature, the reaction mixture was added with (S)-tert-butylsulfinamide (1.82 g, 15.0 mmol) and tetraethyl titanate (4.56 g, 20.0 mmol), heated to 70°C and stirred for 16 h. The reaction mixture was cooled to room

temperature, diluted with water (300 mL), extracted with ethyl acetate (200 mL × 3), and the liquid was separated. The organic phases were combined, dried over anhydrous sodium sulfate, filtered, concentrated, and the residue was purified by silica gel column chromatography (petroleum ether:ethyl acetate (V/V) = 4:1) to obtain (S,E)-N-(1-(2-fluoro-3-((trifluoromethyl)sulfonyl)phenyl)ethylidene)-2-methylpropane-2-sulfmamide (B2-6, white solid, 4.0 g, yield: 100%).

LC-MS, M/Z (ESI): 374.1 $[M+H]^+$.

Step 6: Synthesis of (S)-N-((R)-1-(2-fluoro-3-((trifluoromethyl)sulfonyl)phenyl)ethyl)-2-methylpropane-2-sulfinamide (B2-7)

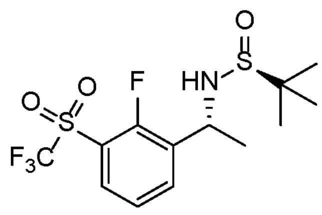

**B2-7**

To methanol (30 mL) was added raw material (S,E)-N-(1-(2-fluoro-3-((trifluoromethyl)sulfonyl)phenyl)ethylidene)-2-methylpropane-2-sulfinamide (1.5 g, 4.0 mmol) at room temperature, and the reaction mixture was cooled to 0°C. $NaBH_4$ (744 mg, 20.1 mmol) was added to methanol in batches, and the reaction mixture was heated to room temperature and stirred for 3 h. The reaction mixture was concentrated and purified by preparative thin-layer chromatography to obtain (S)-N-((R)-1-(2-fluoro-3-((trifluoromethyl)sulfonyl)phenyl)ethyl)-2-methylpropane-2-sulfinamide (B2-7, white solid, 600 mg, yield: 40.0%).

LC-MS, M/Z (ESI): 376.1 $[M+H]^+$.

Step 7: Synthesis of (R)-1-(2-fluoro-3-((trifluoromethyl)sulfonyl)phenyl)ethan-1-amine hydrochloride (B2-8)

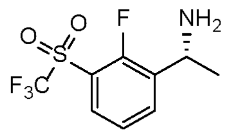

**B2-8**

To a 4 mol/L solution of hydrochloric acid in dioxane (1 mL) was added raw material (S)-N-((R)-1-(2-fluoro-3-((trifluoromethyl)sulfonyl)phenyl)ethyl)-2-methylpropane-2-sulfinamide (250 mg, 0.67 mmol) at room temperature, and the reaction mixture was stirred for 4 h. The reaction mixture was concentrated, added with methyl *tert*-butyl ether (20 mL), stirred for 1 h, and filtered to obtain (R)-1-(2-fluoro-3-((trifluoromethyl)sulfonyl)phenyl)ethan-1-amine hydrochloride (B2-8, white solid, 163 mg, yield: 79.6%).

LC-MS, M/Z (ESI): 272.2 $[M+H]^+$.

Step 8: Synthesis of (R)-4-((1-(2-fluoro-3-((trifluoromethyl)sulfonyl)phenyl)ethyl)amino)-6-(1-(fluoromethyl)cyclopropyl)-2-methylpyrido[4,3-d]pyrimidin-7(6H)-one (I-2)

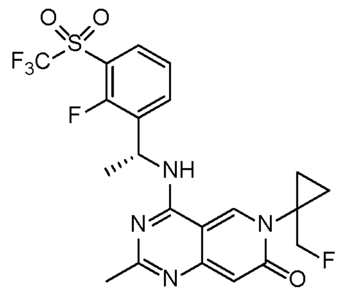

I-2

To acetonitrile (20 mL) was added raw material 6-(1-(fluoromethyl)cyclopropyl)-4-hydroxy-2-methylpyrido[4,3-d]pyrimidin-7(6H)-one (**A1**) (200 mg, 0.80 mmol) at room temperature, the reaction mixture was added with potassium phosphate (678 mg, 3.20 mmol) and phosphonitrilic chloride trimer (416 mg, 1.20 mmol), and stirred at room temperature for 16 h. To DCM (10 mL) was added raw material (R)-1-(2-fluoro-3-((trifluoromethyl)sulfonyl)phenyl)ethan-1-amine hydrochloride (163 mg, 0.53 mmol), the reaction mixture was added with DIPEA (2 mL), and stirred for 0.5 h. The above system was added with the reaction mixture, and stirred at room temperature for 6 h. The reaction mixture was concentrated and prepared under acidic preparation condition B to obtain (R)-4-((1-(2-fluoro-3-((trifluoromethyl)sulfonyl)phenyl)ethyl)amino)-6-(1-(fluoromethyl)cyclopropyl)-2-methylpyrido[4,3-d]pyrimidin-7(6H)-one (I-2, white solid, 25 mg, yield: 6.2%).

$^1$H NMR (400 MHz, DMSO-$d_6$) δ 9.18 (s, 1H), 9.06 (d, 1H), 8.10 (t, 1H), 7.98 (t, 1H), 7.62 (t, 1H), 6.08 (s, 1H), 5.64 (q, 1H), 4.64 (t, 2H), 2.11 (s, 3H), 1.62 (d, 3H), 1.33 (s, 4H).

LC-MS, M/Z (ESI): 503.4 [M+H]$^+$.

**Embodiment 3: Synthesis of compound I-3**

The synthetic route is as follows:

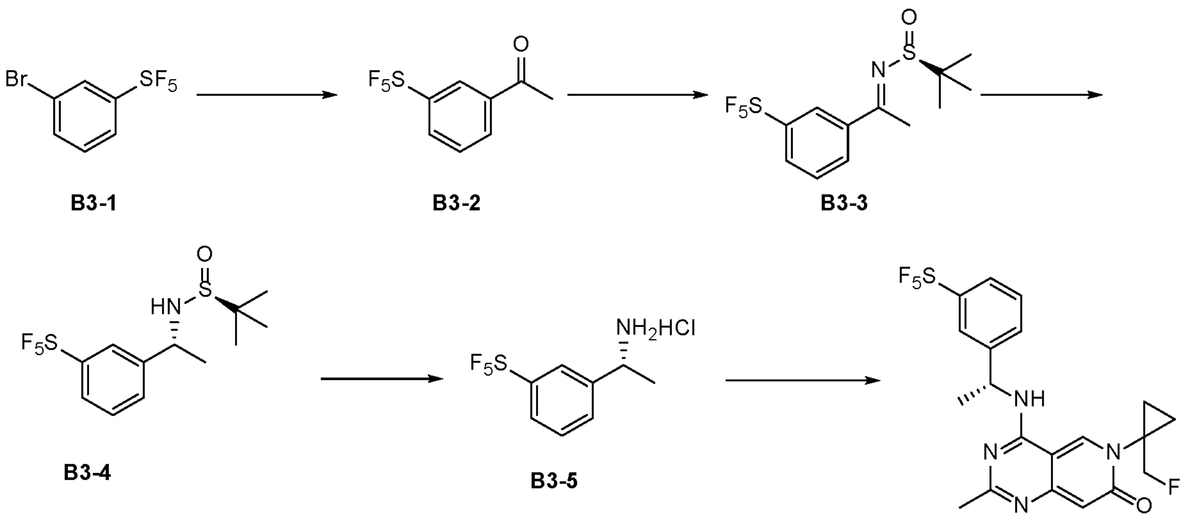

B3-1 B3-2 B3-3 B3-4 B3-5 I-3

Step 1: Synthesis of 1-(3-(pentafluorosulfanyl)phenyl)ethan-1-one (B3-2)

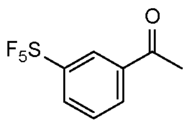

B3-2

To dioxane (100 mL) was added compound 3-bromo-(pentafluorosulfanyl)benzene (3.00 g, 10.6 mmol) at room temperature, the reaction mixture was added with bis(triphenylphosphine)palladium(II) chloride (744 mg, 1.06 mmol) and tributyl(1-ethoxyvinyl)tin (4.20 g, 11.7 mmol), heated to 90°C under nitrogen atmosphere, and stirred for 14 h. The reaction mixture was cooled to room temperature, added with 2 N hydrochloric acid (100 mL), and stirred for 4 h. The reaction mixture was extracted with ethyl acetate (200 mL × 3), and the liquid was separated. The organic phases were combined, dried over anhydrous sodium sulfate, filtered, concentrated, and the residue was purified by silica gel column chromatography (petroleum ether: ethyl acetate (V/V) = 8:1) to obtain 1-(3-(pentafluorosulfanyl)phenyl)ethan-1-one (B3-2, yellow liquid, 2.4 g, yield: 89%).

LC-MS, M/Z (ESI): 247.0 $[M+H]^+$.

Step 2: Synthesis of (S,E)-2-methyl-N-(1-(3-(pentafluorosulfanyl)phenyl)ethylidene)propane-2-sulfinamide (B3-3)

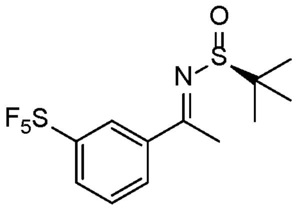

**B3-3**

To THF (150 mL) was added compound 1-(3-(pentafluorosulfanyl)phenyl)ethan-1-one (1.0 g, 4.06 mmol) at room temperature, the reaction mixture was added with (S)-*tert*-butylsulfinamide (492 mg, 4.06 mmol) and tetraethyl titanate (1.14 g, 5.0 mmol), heated to 70°C and stirred for 16 h. The reaction mixture was cooled to room temperature, diluted with water (100 mL), extracted with ethyl acetate (100 mL × 3), and the liquid was separated. The organic phases were combined, dried over anhydrous sodium sulfate, filtered, concentrated, and the residue was purified by silica gel column chromatography (petroleum ether:ethyl acetate (V/V) = 4:1) to obtain (S,E)-2-methyl-N-(1-(3-(pentafluorosulfanyl)phenyl)ethylidene)propane-2-sulfinamide (B3-3, white solid, 1.42 g, yield: 100%).

LC-MS, M/Z (ESI): 350.2 $[M+H]^+$.

Step 3: Synthesis of (S)-2-methyl-N-((R)-1-(3-(pentafluorosulfanyl)phenyl)ethyl)propane-2-sulfinamide (B3-4)

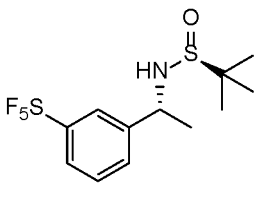

**B3-4**

To methanol (30 mL) was added raw material (S,E)-2-methyl-N-(1-(3-(pentafluorosulfanyl)phenyl)ethylidene)propane-2-sulfinamide (1.5 g, 4.3 mmol) at room temperature, and the reaction mixture was cooled to 0°C. $NaBH_4$ (744 mg, 20.1 mmol) was added to methanol in batches, and the reaction mixture was heated to room temperature and stirred for 3 h. The reaction mixture was concentrated and purified by preparative thin-layer chromatography to obtain (S)-2-methyl-N-((R)-1-(3-(pentafluorosulfanyl)phenyl)ethyl)propane-2-sulfinamide (B3-4, white solid, 600 mg, yield: 40.0%).

LC-MS, M/Z (ESI): 352.1 $[M+H]^+$.

Step 4: Synthesis of (R)-1-(3-(pentafluorosulfanyl)phenyl)ethan-1-amine hydrochloride (B3-5)

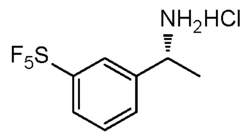

**B3-5**

To a 4 mol/L solution of hydrochloric acid in dioxane (10 mL) was added raw material (R)-2-methyl-N-((R)-1-(3-(pentafluorosulfanyl)phenyl)ethyl)propane-2-sulfinamide (600 mg, 1.70 mmol) at room temperature, and the reaction mixture was stirred for 4 h. The reaction mixture was concentrated, added with methyl tert-butyl ether (20 mL), stirred for 1 h, and filtered to obtain (R)-1-(3-(pentafluorosulfanyl)phenyl)ethan-1-amine hydrochloride (B3-5, white solid, 350 mg, yield: 72.7%).

LC-MS, M/Z (ESI): 248.2 $[M+H]^+$.

Step 5: Synthesis of (R)-6-(1-(fluoromethyl)cyclopropyl)-2-methyl-4-((1-(3-(pentafluorosulfanyl)phenyl)ethyl)amino)pyrido[4,3-d]pyrimidin-7(6H)-one (I-3)

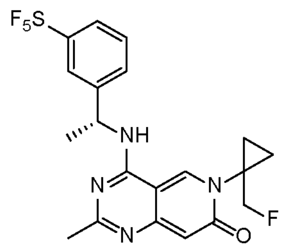

**I-3**

To acetonitrile (20 mL) was added raw material 6-(1-(fluoromethyl)cyclopropyl)-4-hydroxy-2-methylpyrido[4,3-d]pyrimidin-7(6H)-one (**A1**) (200 mg, 0.80 mmol) at room temperature, the reaction mixture was added with potassium phosphate (678 mg, 3.20 mmol) and phosphonitrilic chloride trimer (416 mg, 1.20 mmol), and stirred at room temperature for 16 h. To DCM (10 mL) was added raw material (R)-1-(3-(pentafluorosulfanyl)phenyl)ethan-1-amine hydrochloride (160 mg, 0.56 mmol), the reaction mixture was added with DIPEA (2 mL), and stirred for 0.5 h. The above system was added into the reaction mixture, and stirred at room temperature for 6 h. The reaction mixture was concentrated and prepared under acidic preparation condition B to obtain (R)-6-(1-(fluoromethyl)cyclopropyl)-2-methyl-4-((1-(3-(pentafluorosulfanyl)phenyl)ethyl)amino)pyrido[4,3-d]pyrimidin-7(6H)-one (I-3, white solid, 44 mg, yield: 16.4%).

$^1H$ NMR (400 MHz, DMSO-$d_6$) δ 9.18 (s, 1H), 8.87 (d, 1H), 7.96 (s, 1H), 7.78 (d, 1H), 7.71 (d, 1H), 7.60 (t, 1H), 6.08 (s, 1H), 5.60 (q, 1H), 4.68 - 4.56 (m, 2H), 2.21 (s, 3H), 1.61 (d, 3H), 1.32 - 1.28 (m, 4H).

LC-MS, M/Z (ESI): 479.4 $[M+H]^+$.

**Embodiment 4: Synthesis of compound I-4**

Compound **I-4** was synthesized with reference to the synthetic method of compound 1-1 by replacing sodium bromodifluoroacetate with iodotrifluoromethane in step 3 to obtain (R)-6-(1-(fluoromethyl)cyclopropyl)-2-methyl-4-((1-(2-methyl-3-((trifluoromethyl)sulfonyl)phenyl)ethyl)amino)pyrido[4,3-d]pyrimidin-7(6H)-one (I-4). LC-MS, M/Z (ESI): 499.1 $[M+H]^+$.

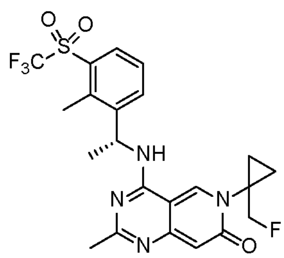

**I-4**

### Embodiment 5: Synthesis of compound I-5

Compound **I-5** was synthesized with reference to the synthetic method of compound **I-1** with the starting material replaced by 1-bromo-2-fluoro-3-iodobenzene to obtain (R)-4-((1-(3-((difluoromethyl)sulfonyl)-2-fluorophenyl)ethyl)amino)-6-(1-(fluoromethyl)cyclopropyl)-2-methylpyrido[4,3-d]pyrimidin-7(6H)-one (I-5). $^1$H NMR (400 MHz, DMSO-$d_6$) δ 9.17 (s, 1H), 8.94 (d, 1H), 7.98 (t, 1H), 7.83 (t, 1H), 7.53 (d, 1H), 7.41 (t, 1H), 6.08 (s, 1H), 5.65 - 5.68 (m, 1H), 4.55 - 4.60 (m, 2H), 2.15 (s, 3H), 1.60 (d, 3H), 1.78 (d, 3H), 1.23 - 1.34 (m, 4H). LC-MS, M/Z (ESI): 485.0 [M+H]$^+$.

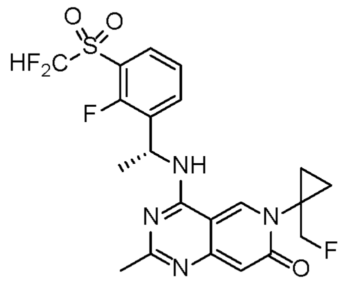

**I-5**

### Embodiment 6: Synthesis of compound I-6

The synthetic route is as follows:

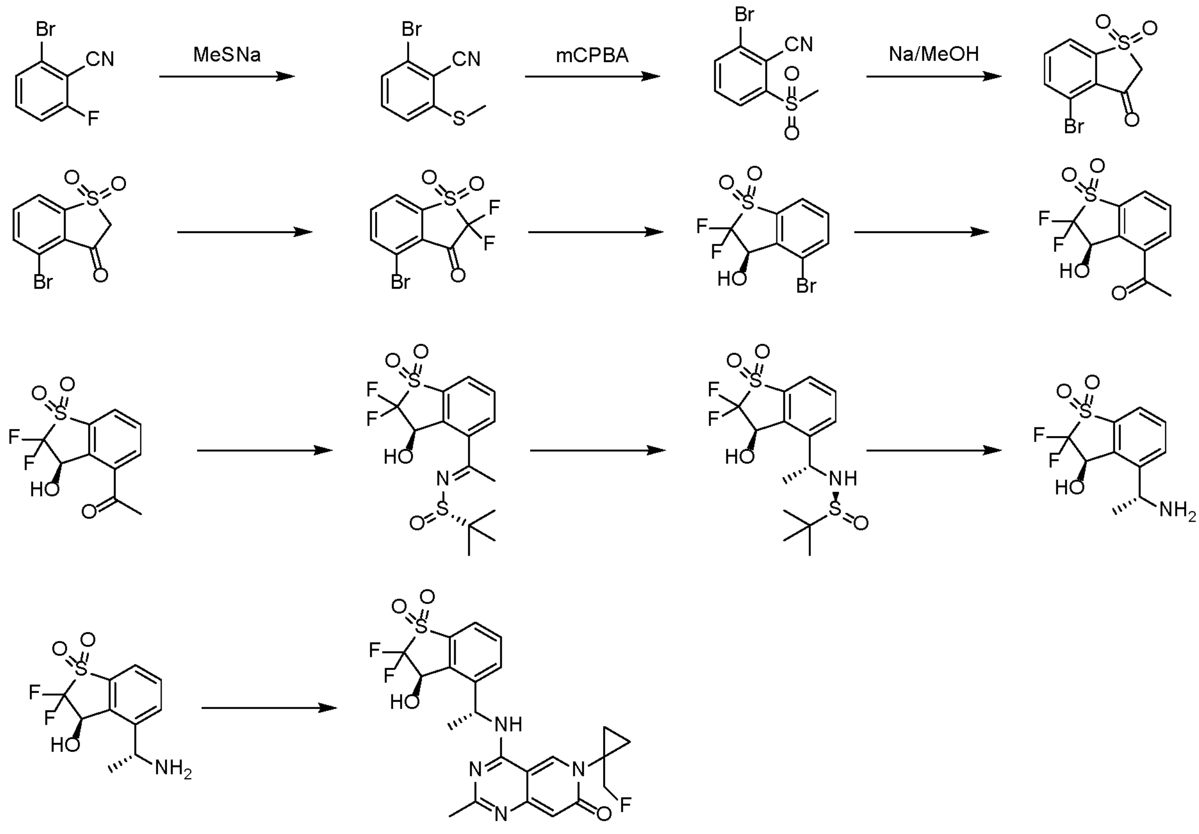


**I-6**

Compound 4-(((R)-1-((R)-2,2-difluoro-3-hydroxy-1,1-dioxo-2,3-dihydrobenzo[b]thiophen-4-yl)ethyl)amino)-6-(1-(fluoromethyl)cyclopropyl)-2-methylpyrido[4,3-d]pyrimidin-7(6H)-one (I-6) was obtained. LC-MS, M/Z (ESI): 495.2 [M+H]$^+$.

## Embodiment 7: Synthesis of compound I-7

The synthetic route is as follows:

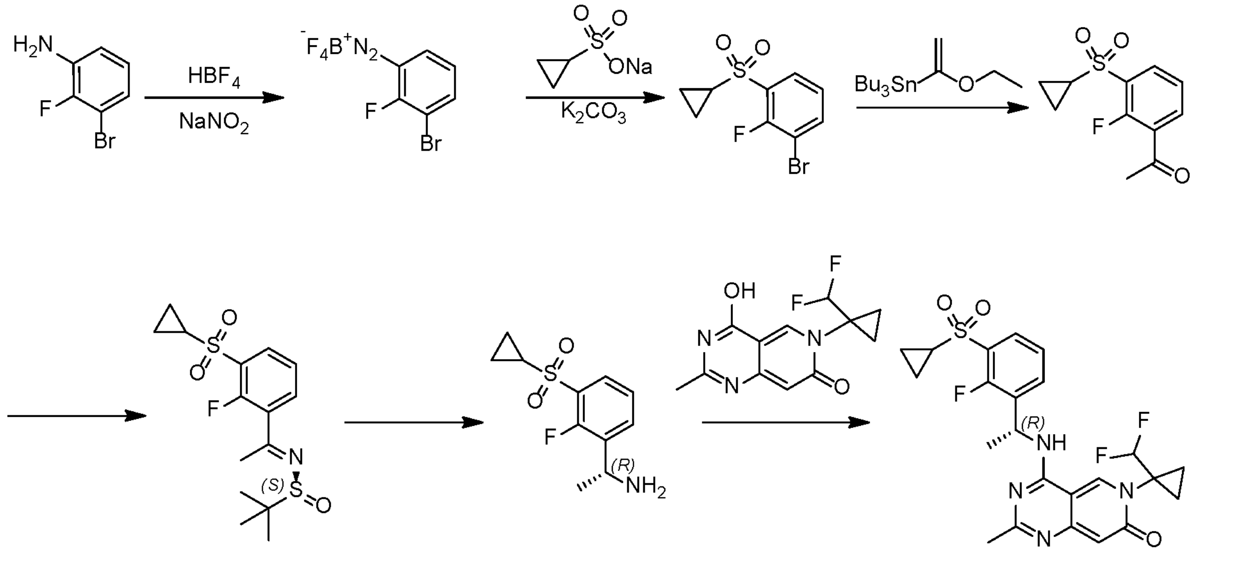


I-7

Compound (R)-4-((1-(3-(cyclopropylsulfonyl)-2-fluorophenyl)ethyl)amino)-6-(1-(difluoromethyl)cyclopropyl)-2-methylpyrido[4,3-d]pyrimidin-7(6H)-one (I-7) was obtained. LC-MS, M/Z (ESI): 493.2 $[M+H]^+$.

## Embodiment 8: Synthesis of compound I-8

The synthetic route is as follows:

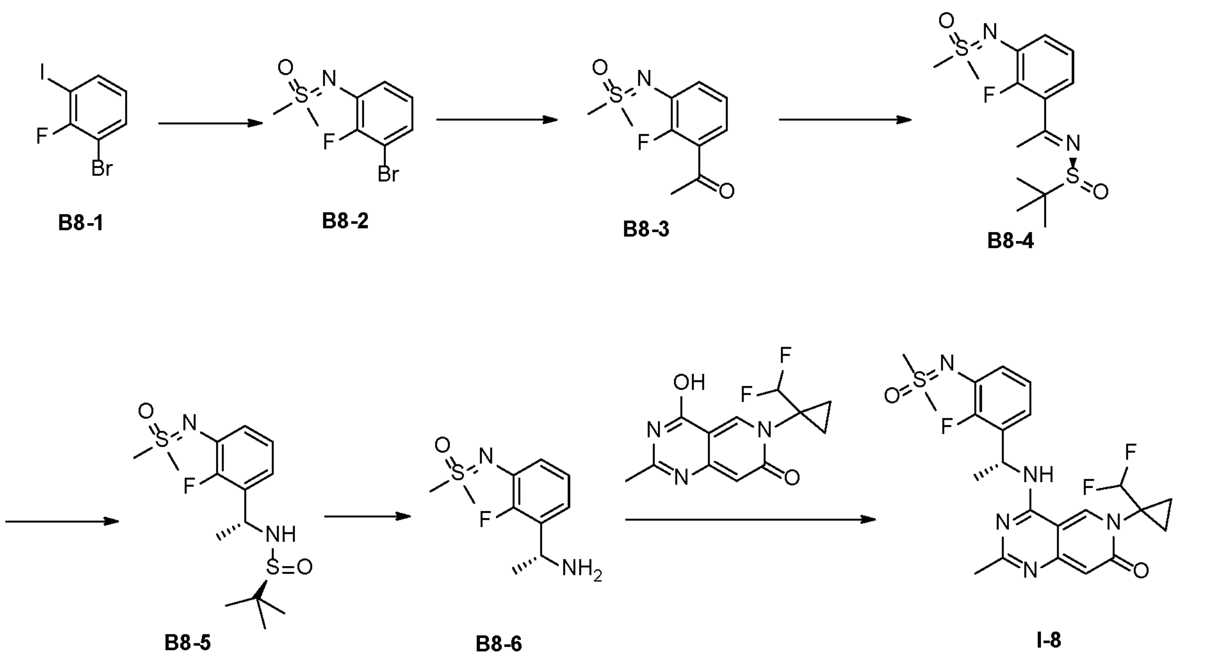

B8-1 B8-2 B8-3 B8-4 B8-5 B8-6 I-8

Step 1: ((3-Bromo-2-fluorophenyl)imino)dimethylsulfanone **(B8-2)**

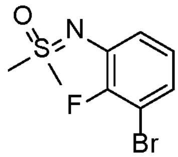

**B8-2**

To dioxane (50 mL) was added compound 1-bromo-2-fluoro-3-iodobenzene (1.00 g, 3.32 mmol) at room temperature, and the reaction mixture was added with iminodimethylsulfanone (370 mg, 4.00 mmol), cesium carbonate (3.25 g, 11.7 mmol), tris(dibenzylideneacetone)dipalladium (607 mg, 0.664 mmol) and 4,5-bis(diphenylphosphino)-9,9-dimethylxanthene (384 mg, 0.664 mmol). The reaction mixture was heated to 105°C under nitrogen atmosphere, and stirred for 3 h. The reaction mixture was cooled to room temperature, added with water (100 mL), extracted with ethyl acetate (200 mL × 3), and the liquid was separated. The organic phases were combined, dried over anhydrous sodium sulfate, filtered, concentrated, and the residue was purified by silica gel column chromatography (petroleum ether: ethyl acetate (V/V) = 5:1) to obtain the title compound ((3-bromo-2-fluorophenyl)imino)dimethylsulfanone **(B8-2)** (500 mg, yield: 56.6%) as a yellow solid.

Step 2: ((3-Acetyl-2-fluorophenyl)imino)dimethylsulfanone **(B8-3)**

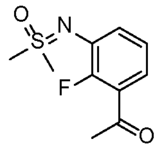

**B8-3**

To dioxane (30 mL) was added compound ((3-bromo-2-fluorophenyl)imino)dimethylsulfanone (460 mg, 1.73 mmol) at room temperature, the reaction mixture was added with bis(triphenylphosphine)palladium(II) chloride (122 mg, 0.173 mmol) and tributyl(1-ethoxyvinyl)tin (628 g, 1.73 mmol), heated to 90°C under nitrogen atmosphere, and stirred for 14 h. The reaction mixture was cooled to room temperature, added with 2 N hydrochloric acid (10 mL), and stirred for 4 h. The reaction mixture was extracted with ethyl acetate (50 mL × 3), and the liquid was separated. The organic phases were combined, dried over anhydrous sodium sulfate, filtered, concentrated, and the residue was purified by silica gel column chromatography (petroleum ether:ethyl acetate (V/V) = 5:1) to obtain the title compound ((3-acetyl-2-fluorophenyl)imino)dimethylsulfanone **(B8-3)** (340 mg, yield: 85%) as a yellow liquid.

Step 3: (S,E)-N-(1-(3-((dimethyl(oxo)sulfonyl)amino)-2-fluorophenyl)ethylidene)-2-methylpropane-2-sulfinamide **(B8-4)**

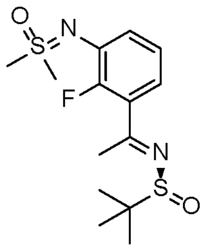

**B8-4**

To THF (15 mL) was added compound ((3-acetyl-2-fluorophenyl)imino)dimethylsulfanone (340 mg, 1.50 mmol) at room temperature, the reaction mixture was added with (S)-*tert*-butylsulfinamide (856 mg, 2.25 mmol) and tetraethyl titanate (273 mg, 3.75 mmol), heated to 70°C and stirred for 16 h. The reaction mixture was cooled to room temperature, diluted with water (100 mL), extracted with ethyl acetate (50 mL × 3), and the liquid was separated. The organic phases were combined, dried over anhydrous sodium sulfate, filtered, concentrated, and the residue was purified by silica gel column chromatography (petroleum ether: ethyl acetate (V/V) = 1:1) to obtain the title compound (S,E)-N-(1-(3-((dimethyl(oxo)sulfonyl)amino)-2-fluorophenyl)ethylidene)-2-methylpropane-2-sulfinamide **(B8-4)** (600 mg, yield > 100%) as a yellow solid.

LC-MS, M/Z (ESI): 333.4 $[M+H]^+$.

Step 4: (S)-N-((R)-1-(3-((dimethyl(oxo)sulfonyl)amino)-2-fluorophenyl)ethyl)-2-methylpropane-2-sulfinamide **(B8-5)**

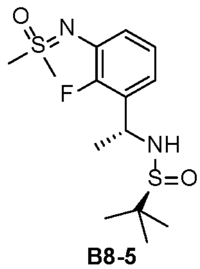

**B8-5**

To methanol (30 mL) was added raw material (S,E)-N-(1-(3-((dimethyl(oxo)sulfonyl)amino)-2-fluorophenyl)ethylidene)-2-methylpropane-2-sulfinamide (600 mg, 1.8 mmol) at room temperature, and the reaction mixture was cooled to 0°C. Sodium borohydride (96 mg, 2.5 mmol) was added to methanol in batches, and the reaction mixture was heated to room temperature and stirred for 3 h. The reaction mixture was concentrated and purified by preparative thin-layer chromatography (petroleum ether:ethyl acetate (V/V) = 1:1) to obtain the title compound (S)-N-((R)-1-(3-((dimethyl(oxo)sulfonyl)amino)-2-fluorophenyl)ethyl)-2-methylpropane-2-sulfinamide **(B8-5)** (200 mg, yield: 33.0%) as a white solid.

Step 5: (R)-((3-(1-aminoethyl)-2-fluorophenyl)imino)dimethylsulfanone hydrochloride **(B8-6)**

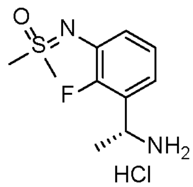

**B8-6**

To a 4 mol/L solution of hydrochloric acid in dioxane (10 mL) was added raw material (S)-N-((R)-1-(3-((dimethyl(oxo)sulfonyl)amino)-2-fluorophenyl)ethyl)-2-methylpropane-2-sulfinamide (200 mg, 0.60 mmol) at room temperature, and the reaction mixture was stirred for 4 h. The reaction mixture was concentrated, added with methyl *tert*-butyl ether (20 mL), stirred for 1 h, and filtered to obtain the title compound (R)-((3-(1-aminoethyl)-2-fluorophenyl)imino)dimethylsulfanone hydrochloride **(B8-6)** (100 mg, yield: 62.9%) as a white solid.

LC-MS, M/Z (ESI): 231.2 $[M+H]^+$.

Step 6: (R)-6-(1-(difluoromethyl)cyclopropyl)-4-((1-(3-((dimethyl(oxo)sulfonyl)amino)-2-fluorophenyl)ethyl)amino)-2-methylpyrido[4,3-d]pyrimidin-7(6H)-one

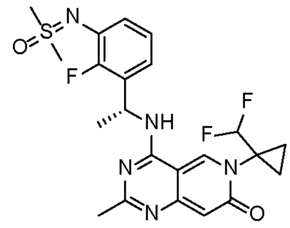

I-8

To acetonitrile (20 mL) was added raw material 6-(1-(fluoromethyl)cyclopropyl)-4-hydroxy-2-methylpyrido[4,3-d]pyrimidin-7(6H)-one (200 mg, 0.80 mmol) at room temperature, the reaction mixture was added with potassium phosphate (678 mg, 3.20 mmol) and phosphonitrilic chloride trimer (416 mg, 1.20 mmol), and stirred at room temperature for 16 h. To DCM (10 mL) was added raw material (R)-((3-(1-aminoethyl)-2-fluorophenyl)imino)dimethylsulfanone hydrochloride (149 mg, 0.56 mmol), the reaction mixture was added with DIPEA (2 mL), and stirred for 0.5 h. The above system was added with the reaction mixture, and stirred at room temperature for 6 h. The reaction mixture was concentrated and purified by silica gel column chromatography (dichloromethane:methanol (V/V) = 10:1) to obtain the title compound (R)-6-(1-(difluoromethyl)cyclopropyl)-4-((1-(3-((dimethyl(oxo)sulfonyl)amino)-2-fluorophenyl)ethyl)amino)-2-methylpyrido[4,3-d]pyrimidin-7(6H)-one (9 mg, yield: 3.35%) as a white solid.

$^1$H NMR (400 MHz, DMSO-$d_6$) δ 9.17 (s, 1H), 8.79 (d, 1H), 7.04 - 7.01 (m, 1H), 6.97 - 6.95 (m, 1H), 6.33 (t, 1H), 6.08 (s, 1H), 5.79 - 5.75 (m, 1H), 3.32 (s, 3H), 3.21 (s, 3H), 2.20 (s, 3H), 1.53 (d, 3H), 1.37 - 1.23 (m, 4H).

LC-MS, M/Z (ESI): 480.4 [M+H]$^+$.

**Embodiment 9: Synthesis of compound 1-9**

The synthetic route is as follows:

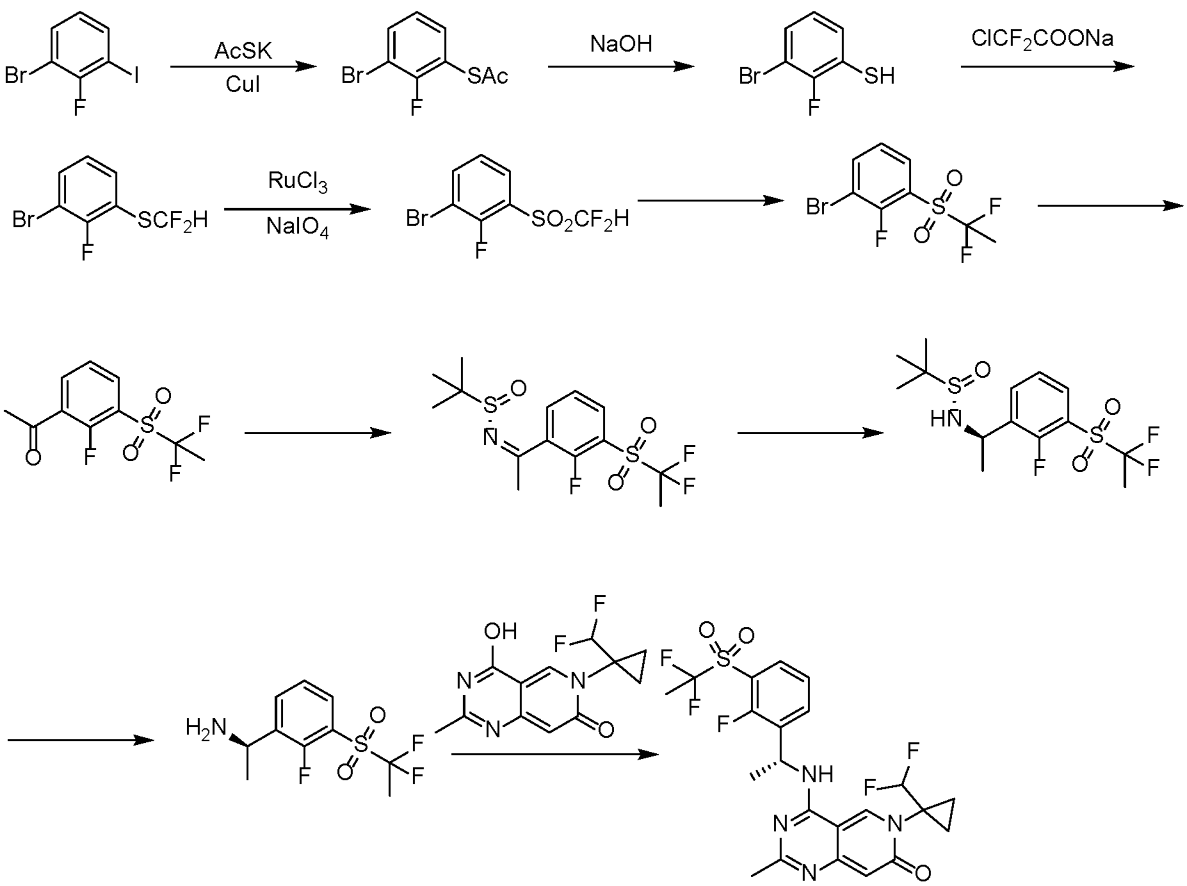


I-9

Compound (R)-4-((1-(3-((1,1-difluoroethyl)sulfonyl)-2-fluorophenyl)ethyl)amino)-6-(1-(difluoromethyl)cyclopropyl)-2-methylpyrido[4,3-d]pyrimidin-7(6H)-one (I-9) was obtained. LC-MS, M/Z (ESI): 517.2 [M+H]$^+$.

**Embodiment 10: Synthesis of compound I-10**

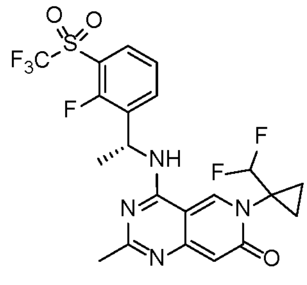

**I-10**

Compound I-10 was synthesized with reference to the synthetic method of compound I-9 to obtain compound (R)-6-(1-(difluoromethyl)cyclopropyl)-4-((1-(2-fluoro-3-((trifluoromethyl)sulfonyl)phenyl)ethyl)amino)-2-methylpyrido[4,3-d]pyrimidin-7(6H)-one (I-10). LC-MS, M/Z (ESI): 521.2 [M+H]$^+$.

**Embodiment 11: Synthesis of compound I-11**

The synthetic route is as follows:

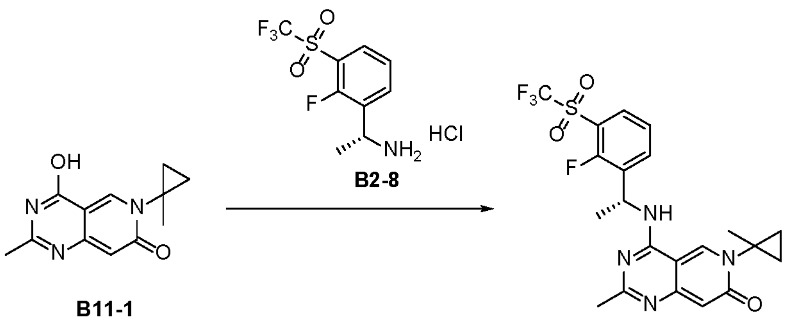


B11-1 B2-8 I-11

Intermediate **B11-1** was synthesized with reference to the synthesis of intermediate **A1** by replacing 1-(fluoromethyl)cyclopropane-1-amine hydrochloride with raw material 1-methylcyclopropylamine hydrochloride.
To acetonitrile (10 mL) was added raw material 4-hydroxy-2-methyl-6-(1-methylcyclopropyl)pyrido[4,3-d]pyrimidin-7(6H)-one **(B11-1)** (100 mg, 0.43 mmol), the reaction mixture was added with compound (R)-1-(2-fluoro-3((trifluoromethyl)sulfonyl)phenyl)ethane-1-ethylamine hydrochloride **(B2- 8)** (146 mg, 0.48 mmol) and anhydrous potassium phosphate (229 mg, 1.08 mmol), stirred at room temperature for 24 h, then added with phosphonitrilic chloride trimer (150 mg, 0.43 mmol) and triethylamine (137 mg, 1.29 mmol), and stirred continuously at room temperature for 24 h. The reaction mixture was concentrated under reduced pressure and purified by silica gel column chromatography (petroleum ether:ethyl acetate = 10:1 to 1:1) to obtain (R)-4-((1-(2-fluoro-3-((trifluoromethyl)sulfonyl)phenyl)ethyl)amino)-2-methyl-6-(1-methylcyclopropyl)pyrido[4,3-d]pyrimidin-7(6H)-one **(I-11)** (48 mg, yield: 22.9%) as a white solid.
$^1$H NMR (400 MHz, DMSO) $\delta$ 9.40 (s, 1H), 9.24 (s, 1H), 8.18 (s, 2H), 7.91 - 8.07 (s, 1H), 7.58 (m, 1H), 5.49 - 5.77 (m, 1H), 1.93 - 2.25 (m, 3H), 1.61 (d, *J* = 7.1 Hz, 3H), 1.38 - 1.55 (m, 3H), 1.19 (s, 2H), 0.88 - 1.08 (m, 2H).
LC-MS, M/Z (ESI): 485.5 [M+H]$^+$.

**Embodiment 12: Synthesis of compound I-12**

The synthetic route is as follows:

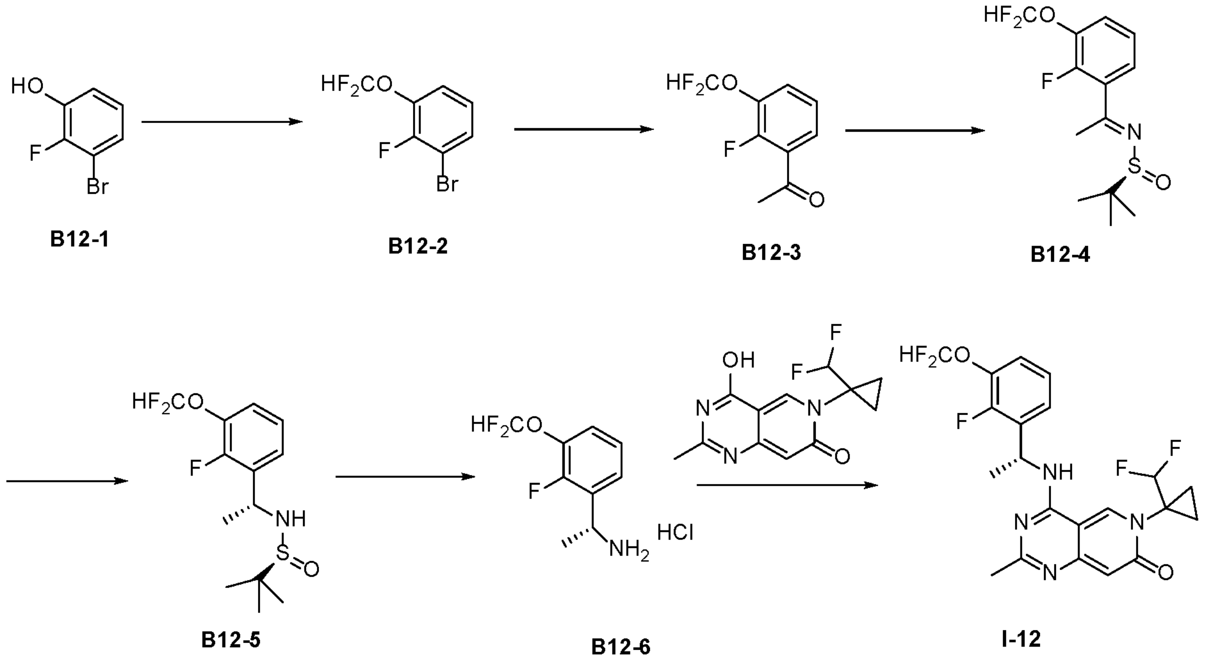

B12-1 B12-2 B12-3 B12-4

B12-5 B12-6 I-12

Step 1: 1-Bromo-3-(difluoromethoxy)-2-fluorobenzene (B12-2)

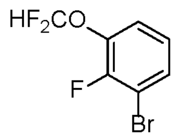

**B12-2**

To DMF (100 mL) was added compound 3-bromo-2-fluorophenol (7.20 g, 37.6 mmol) at room temperature, the reaction mixture was added with cesium carbonate (26.7 g, 75.2 mmol) and sodium bromodifluoroacetate (8.89 g, 45.1 mmol), heated to 100°C and stirred for 2 h. The reaction mixture was cooled to room temperature, added with water (300 mL), extracted with ethyl acetate (200 mL × 3), and the liquid was separated. The organic phases were combined, dried over anhydrous sodium sulfate, filtered, concentrated, and the residue was purified by silica gel column chromatography (petroleum ether:ethyl acetate (V/V) = 8:1) to obtain the title compound 1-bromo-3-(difluoromethoxy)-2-fluorobenzene (035B) (5.0 g, yield: 55.2%) as a yellow liquid.

Step 2: 1-(3-(Difluoromethoxy)-2-fluorophenyl)ethan-1-one **(B12-3)**

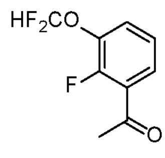

**B12-3**

To dioxane (100 mL) was added compound 1-bromo-3-(difluoromethoxy)-2-fluorobenzene (4.60 g, 19.0 mmol) at room temperature, the reaction mixture was added with bis(triphenylphosphine)palladium(II) chloride (2.13 g, 1.52 mmol) and tributyl(1-ethoxyvinyl)tin (6.85 g, 19.0 mmol), heated to 90°C under nitrogen atmosphere, and stirred for 14

h. The reaction mixture was cooled to room temperature, added with 2 N hydrochloric acid (100 mL), and stirred for 4 h. The reaction mixture was extracted with ethyl acetate (200 mL × 3), and the liquid was separated. The organic phases were combined, dried over anhydrous sodium sulfate, filtered, concentrated, and the residue was purified by silica gel column chromatography (petroleum ether: ethyl acetate (V/V) = 5:1) to obtain the title compound 1-(3-(difluoromethoxy)-2-fluorophenyl)ethan-1-one (2.40 g, yield: 72%) as a yellow liquid.

Step 3: (S,E)-N-(1-(3-(difluoromethoxy)-2-fluorophenyl)ethylidene)-2-methylpropane-2-sulfinamide

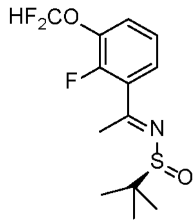

**B12-4**

To THF (150 mL) was added compound 1-(3-(difluoromethoxy)-2-fluorophenyl)ethan-1-one (5.0 g, 25.0 mmol) at room temperature, the reaction mixture was added with (S)-*tert*-butylsulfinamide (4.55 g, 37.5 mmol) and tetraethyl titanate (14.3 g, 62.5 mmol), heated to 70°C and stirred for 16 h. The reaction mixture was cooled to room temperature, diluted with water (300 mL), extracted with ethyl acetate (200 mL × 3), and the liquid was separated. The organic phases were combined, dried over anhydrous sodium sulfate, filtered, concentrated, and the residue was purified by silica gel column chromatography (petroleum ether: ethyl acetate (V/V) = 1:1) to obtain the title compound (S,E)-N-(1-(3-(difluoromethoxy)-2-fluorophenyl)ethylidene)-2-methylpropane-2-sulfinamide (8.50 g, yield: 100%) as a yellow solid.

LC-MS, M/Z (ESI): 308.2 $[M+H]^+$.

Step 4: (S)-N-((R)-1-(3-(difluoromethoxy)-2-fluorophenyl)ethyl)-2-methylpropane-2-sulfinamide **(B12-5)**

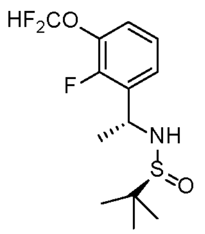

**B12-5**

To methanol (100 mL) was added raw material (S,E)-N-(1-(3-(difluoromethoxy)-2-fluorophenyl)ethylidene)-2-methylpropane-2-sulfinamide (8.5 g, 27.6 mmol) at room temperature, and the reaction mixture was cooled to 0°C. Sodium borohydride (1.50 g, 39.3 mmol) was added to methanol in batches, and the reaction mixture was heated to room temperature and stirred for 3 h. The reaction mixture was concentrated and purified by preparative thin-layer chromatography (petroleum ether:ethyl acetate (V/V) = 1:1) to obtain the title compound (S)-N-((R)-1-(3-(difluoromethoxy)-2-fluorophenyl)ethyl)-2-methylpropane-2-sulfinamide (2.0 g, yield: 23.5%) as a colorless solid.

$^1H$ NMR (400 MHz, DMSO-$d_6$) δ 7.38 - 7.20 (m, 3H), 7.25 (t, 1H), 5.53 (d, 1H), 4.71 - 4.68 (m, 1H), 1.49 (d, 3H), 1.09 (s, 9H).

LC-MS, M/Z (ESI): 310.1 $[M+H]^+$.

Step 5: (R)-1-(3-(difluoromethoxy)-2-fluorophenyl)ethan-1-amine hydrochloride

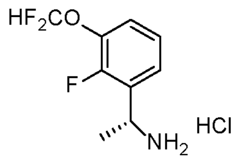

**B12-6**

To a 4 mol/L solution of hydrochloric acid in dioxane (50 mL) was added raw material (S)-N-((R)-1-(3-(difluoromethoxy)-2-fluorophenyl)ethyl)-2-methylpropane-2-sulfinamide (2.0 g, 6.45 mmol) at room temperature, and the reaction mixture was stirred for 4 h. The reaction mixture was concentrated, added with methyl *tert*-butyl ether (50 mL), stirred for 1 h, and filtered to obtain the title compound (R)-1-(3-(difluoromethoxy)-2-fluorophenyl)ethan-1-amine hydrochloride (1.0 g, yield: 64.1%) as a white solid.
LC-MS, M/Z (ESI): 206.2 $[M+H]^+$.

Step 6: (R)-4-((1-(3-(difluoromethoxy)-2-fluorophenyl)ethyl)amino)-6-(1-(fluoromethyl)cyclopropyl)-2-methylpyrido[4,3-d]pyrimidin-7(6H)-one

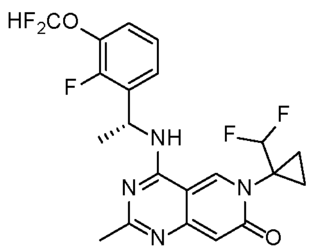

**I-12**

To acetonitrile (20 mL) was added raw material 6-(1-(fluoromethyl)cyclopropyl)-4-hydroxy-2-methylpyrido[4,3-d]pyrimidin-7(6H)-one (200 mg, 0.80 mmol) at room temperature, the reaction mixture was added with potassium phosphate (678 mg, 3.20 mmol) and phosphonitrilic chloride trimer (416 mg, 1.20 mmol), and stirred at room temperature for 16 h. To DCM (10 mL) was added raw material (R)-1-(3-(difluoromethoxy)-2-fluorophenyl)ethan-1-amine hydrochloride (181 mg, 0.75 mmol), the reaction mixture was added with DIPEA (2 mL), and stirred for 0.5 h. The above system was added with the reaction mixture, and stirred at room temperature for 6 h. The reaction mixture was concentrated and purified by silica gel column chromatography (dichloromethane:methanol = 50:1 to 10:1) to obtain the title compound (R)-4-((1-(3-(difluoromethoxy)-2-fluorophenyl)ethyl)amino)-6-(1-(difluoromethyl)cyclopropyl)-2-methylpyrido[4,3-d]pyrimidin-7(6H)-one (15 mg, yield: 4.4%) as a white solid.
$^1H$ NMR (400 MHz, DMSO-$d_6$) δ 9.16 (s, 1H), 8.88 (d, 1H), 7.36 - 7.19 (m, 4H), 7.27 (t, 1H), 6.33 (t, 1H), 6.09 (s, 1H), 5.75 - 5.72 (m, 1H), 2.18 (s, 3H), 1.58 (d, 3H), 1.48 - 1.32 (m, 4H).
LC-MS, M/Z (ESI): 455.3 $[M+H]^+$.

**Embodiment 13: Synthesis of compound 1-13**

The synthetic route is as follows:

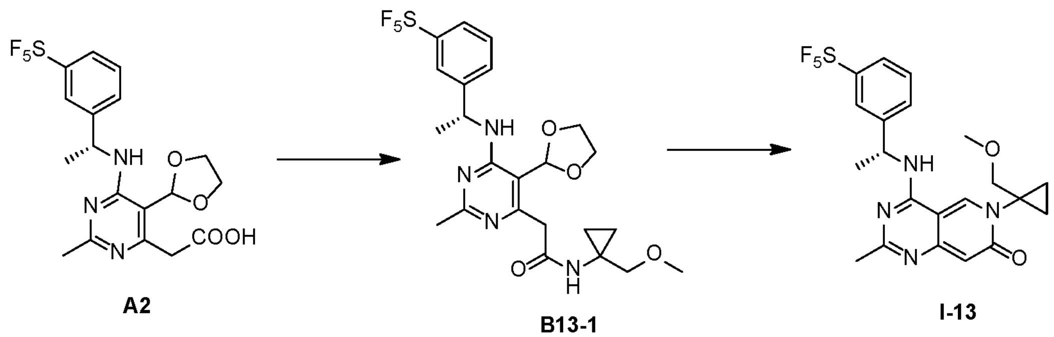

A2

B13-1

I-13

Step 1: Synthesis of (R)-2-(5-(1,3-dioxolan-2-yl)-2-methyl-6-((1-(3-(pentafluorosulfanyl)phenyl)ethyl)amino)pyrimidin-4-yl)-N-(1-(methoxymethyl)cyclopropyl)acetamide **(B13-1)**

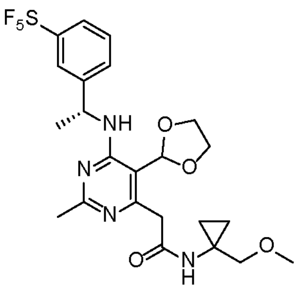

B13-1

(R)-2-(5-(1,3-dioxolan-2-yl)-2-methyl-6-((1-(3-(pentafluorosulfanyl)phenyl)ethyl)amino)pyrimidin-4-yl)acetic acid (243 mg, 0.52 mmol) was dissolved in a mixed solution of dimethyl sulfoxide (2 mL) and acetonitrile (1 mL), the reaction mixture was added with triethylamine (0.14 mL, 1.03 mmol), 2-(7-azobenzotriazole)-N,N,N',N'-tetramethyluronium hexafluorophosphate (295 mg, 0.78 mmol) and 1-(methoxymethyl)cyclopropanamine hydrochloride (107 mg, 0.78 mmol), and stirred at room temperature for 12 h. After the reaction was completed, the reaction mixture was diluted with ethyl acetate (10 mL), and washed with water (10 mL × 3) and saturated brine (10 mL × 3). The organic phase was concentrated, and the residue was purified by silica gel column chromatography (petroleum ether:ethyl acetate (V/V) = 1:3) to obtain the title compound (R)-2-(5-(1,3-dioxolan-2-yl)-2-methyl-6-((1-(3-(pentafluorosulfanyl)phenyl)ethyl)amino)pyrimidin-4-yl)-N-(1-(methoxymethyl)cyclopropyl)acetamide **(B13-1)** (70 mg, yield: 24.5%).

LC-MS, M/Z (ESI): 553.2 $[M+H]^+$.

Step 2: Synthesis of (R)-6-(1-(methoxymethyl)cyclopropyl)-2-methyl-4-((1-(3-(pentafluorosulfanyl)phenyl)ethyl)amino)pyrido[4,3-d]pyrimidin-7(6H)-one **(I-13)**

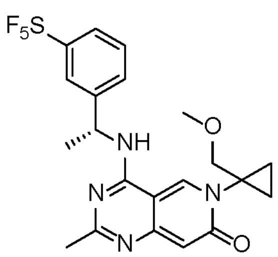

I-13

(R)-2-(5-(1,3-dioxolan-2-yl)-2-methyl-6-((1-(3-(pentafluorosulfanyl)phenyl)ethyl)amino)pyrimidin-4-yl)-N-(1-(methoxymethyl)cyclopropyl)acetamide (70 mg, 0.13 mmol) was dissolved in isopropanol (2 mL), the reaction

mixture was added with 2 M hydrochloric acid (1 mL), and stirred at 50°C for 4 h. After the reaction was completed, the reaction mixture was directly concentrated, and the residue was purified by silica gel column chromatography (dichloromethane:methanol (V/V) = 10:1) to obtain the title compound (R)-6-(1-(methoxymethyl)cyclopropyl)-2-methyl-4-((1-(3-(pentafluorosulfanyl)phenyl)ethyl)amino)pyrido[4,3-d]pyrimidin-7(6H)-one (I-13, 40 mg, yield: 56.3%).
$^1$H NMR (400 MHz, $CDCl_3$) $\delta$ 8.28 (s, 1H), 7.85 (s, 1H), 7.65 - 7.68 (m, 1H), 7.57 (d, 1H), 7.26 - 7.47 (m, 1H), 6.51 (s, 1H), 6.16 (d, 1H), 5.60 - 5.68 (m, 2H), 3.61 (s, 2H), 3.26 (s, 3H), 1.68 (d, 3H), 1.20 (d, 4H).
LC-MS, M/Z (ESI): 491.2 $[M+H]^+$.

**Embodiment 14: Synthesis of compound I-14**

The synthetic route is as follows:

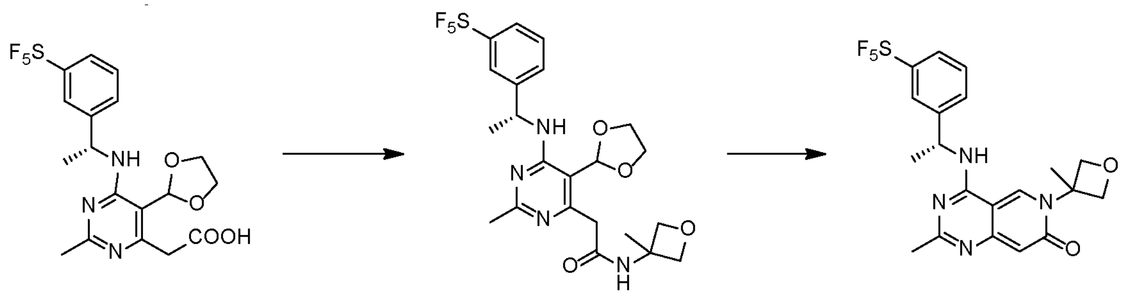

A2 B14-1 I-14

Step 1: Synthesis of (R)-2-(5-(1,3-dioxolan-2-yl)-2-methyl-6-((1-(3-(pentafluorosulfanyl)phenyl)ethyl)amino)pyrimidin-4-yl)-N-(3-methyloxetan-3-yl)acetamide (**B14-1**)

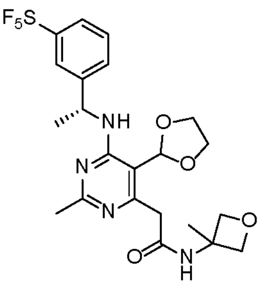

B14-1

(R)-2-(5-(1,3-dioxolan-2-yl)-2-methyl-6-((1-(3-(pentafluorosulfanyl)phenyl)ethyl)amino)pyrimidin-4-yl)acetic acid (583 mg, 1.24 mmol) was dissolved in a mixed solution of dimethyl sulfoxide (2 mL) and acetonitrile (1 mL), the reaction mixture was added with triethylamine (0.35 mL, 2.48 mmol), 2-(7-azobenzotriazole)-N,N,N',N'-tetramethyluronium hexafluorophosphate (708 mg, 1.86 mmol) and 3-trimethoprim-3-amine hydrochloride (199 mg, 1.61 mmol), and stirred at room temperature for 12 h. After the reaction was completed, the reaction mixture was diluted with ethyl acetate (10 mL), and washed with water (10 mL × 3) and saturated brine (10 mL × 3). The organic phase was concentrated, and the residue was purified by silica gel column chromatography (petroleum ether: ethyl acetate (V/V) = 1:3) to obtain the title compound (R)-2-(5-(1,3-dioxolan-2-yl)-2-methyl-6-((1-(3-(pentafluorosulfanyl)phenyl)ethyl)amino)pyrimidin-4-yl)-N-(3-methyloxetan-3-yl)acetamide **(B14-1)** (300 mg, yield: 44.9%).
LC-MS, M/Z (ESI): 539.1 $[M+H]^+$.

Step 2: (R)-2-methyl-6-(3-methyloxetan-3-yl)-4-((1-(3-(pentafluorosulfanyl)phenyl)ethyl)amino)pyrido[4,3-d]pyrimidin-7(6H)-one (I-14)

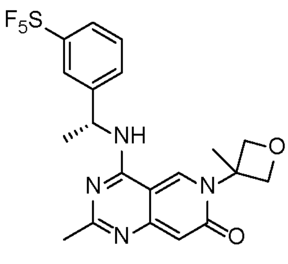

I-14

(R)-2-(5-(1,3-dioxolan-2-yl)-2-methyl-6-((1-(3-(pentafluorosulfanyl)phenyl)ethyl)amino)pyrimidin-4-yl)-N-(3-methyloxetan-3-yl)acetamide (300 mg, 0.56 mmol) was dissolved in isopropanol (2 mL), the reaction mixture was added with 2 M hydrochloric acid (1 mL), and stirred at 50°C for 4 h. After the reaction was completed, the reaction mixture was directly concentrated, and the residue was purified by silica gel column chromatography (dichloromethane:methanol (V/V) = 10:1) to obtain the title compound (R)-2-methyl-6-(3-methyloxetan-3-yl)-4-((1-(3-(pentafluorosulfanyl)phenyl)ethyl)amino)pyrido[4,3-d]pyrimidin-7(6H)-one **(I-14,** 150 mg, yield: 56.6%).

$^1$H NMR (400 MHz, $CDCl_3$) δ 11.01 (d, 1H), 10.25 (d, 1H), 8.75 (s, 1H), 7.96 (d, 1H), 7.79 (d, 1H), 7.62 - 7.75 (m, 2H), 7.41 - 7.49 (m, 1H), 6.99 (s, 1H), 5.59 - 5.64 (m, 1H), 5.03 - 5.05 (m, 1H), 4.64 (d, 1H), 4.42 - 4.45 (m, 1H), 3.73 - 3.76 (m, 1H), 2.54 (d, 3H), 1.73 - 1.81 (m, 6H).

LC-MS, M/Z (ESI): 477.1 $[M+H]^+$.

**Embodiment 15: Synthesis of compound I-15**

The synthetic route is as follows:

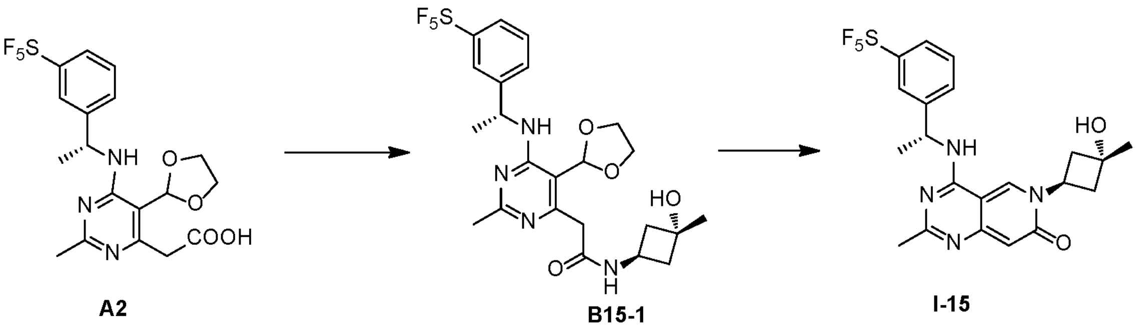

A2 B15-1 I-15

Step 1: Synthesis of 2-(5-(1,3-dioxolan-2-yl)-2-methyl-6-(((R)-1-(3-(pentafluorosulfanyl)phenyl)ethyl)amino)pyrimidin-4-yl)-N-((1r,3R)-3-hydroxy-3-methylcyclobutyl)acetamide **(B15-1)**

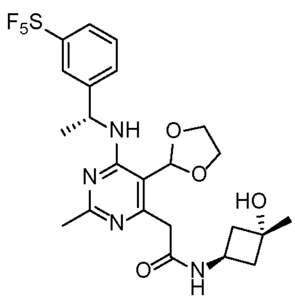

B15-1

(R)-2-(5-(1,3-dioxolan-2-yl)-2-methyl-6-((1-(3-(pentafluorosulfanyl)phenyl)ethyl)amino)pyrimidin-4-yl)acetic acid (400 mg, 0.83 mmol) was dissolved in a mixed solution of dimethyl sulfoxide (2 mL) and acetonitrile (1 mL), the reaction mixture was added with triethylamine (0.23 mL, 1.65 mmol), 2-(7-azobenzotriazole)-N,N,N',N'-tetramethyluronium hexafluorophosphate (472 mg, 1.24 mmol) and (1r,3r)-3-amino-1-methylcyclobutanol (148 mg, 1.07 mmol), and stirred at room temperature for 12 h. After the reaction was completed, the reaction mixture was diluted with ethyl acetate (10 mL), and washed with water (10 mL × 3) and saturated brine (10 mL × 3). The organic phase was concentrated, and the residue was purified by silica gel column chromatography (petroleum ether: ethyl acetate (V/V) = 1:3) to obtain the title compound 2-(5-(1,3-dioxolan-2-yl)-2-methyl-6-(((R)-1-(3-(pentafluorosulfanyl)phenyl)ethyl)amino)pyrimidin-4-yl)-N-((1r,3R)-3-hydroxy-3-methylcyclobutyl)acetamide **(B15-1)** (300 mg, yield: 65.6%).

LC-MS, M/Z (ESI): 553.1 $[M+H]^+$.

Step 2: Synthesis of 6-((1r,3R)-3-hydroxy-3-methylcyclobutyl)-2-methyl-4-(((R)-1-(3-(pentafluorosulfanyl)phenyl)ethyl)amino)pyrido[4,3-d]pyrimidin-7(6H)-one (I-15)

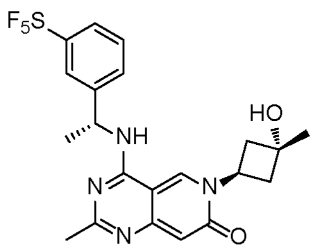

**I-15**

2-(5-(1,3-dioxolan-2-yl)-2-methyl-6-(((R)-1-(3-(pentafluorosulfanyl)phenyl)ethyl)amino)pyrimidin-4-yl)-N-((1r,3R)-3-hydroxy-3-methylcyclobutyl)acetamide (300 mg, 0.54 mmol) was dissolved in isopropanol (2 mL), the reaction mixture was added with 2 M hydrochloric acid (1 mL), and stirred at 50°C for 4 h. After the reaction was completed, the reaction mixture was directly concentrated, and the residue was purified by silica gel column chromatography (dichloromethane:methanol (V/V) = 10:1) to obtain the title compound 6-((1r,3R)-3-hydroxy-3-methylcyclobutyl)-2-methyl-4-(((R)-1-(3-(pentafluorosulfanyl)phenyl)ethyl)amino)pyrido[4,3-d]pyrimidin-7(6H)-one (I-15, 140 mg, yield: 52.6%).

$^1H$ NMR (400 MHz, DMSO-$d_6$) δ 8.94 (s, 1H), 8.87 (d, 1H), 7.93 (s, 1H), 7.77 (d, 1H), 7.73 (d, 1H), 7.56 - 7.60 (m, 1H), 6.05 (s, 1H), 5.59 - 5.62 (m, 1H), 5.23 (s, 1H), 4.55 - 4.63 (m, 1H), 2.52 - 2.55 (m, 2H), 2.47 - 2.49 (m, 2H), 2.19 (s, 3H), 1.60 (d, 3H), 1.36 (s, 3H).

LC-MS, M/Z (ESI): 491.1 $[M+H]^+$.

**Embodiment 16: Synthesis of compound 1-16**

The synthetic route is as follows:

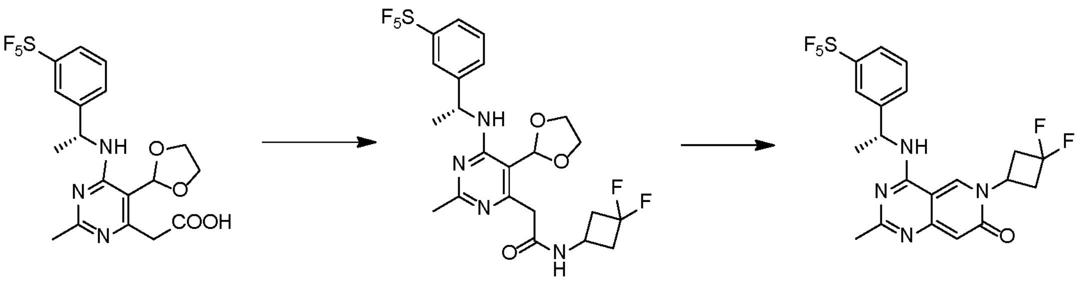

A2 B16-1 I-16

Step 1: Synthesis of (R)-2-(5-(1,3-dioxolan-2-yl)-2-methyl-6-((1-(3-(pentafluorosulfanyl)phenyl)ethyl)amino)pyrimidin-4-yl)-N-(3,3-difluorocyclobutyl)acetamide (**B16-1**)

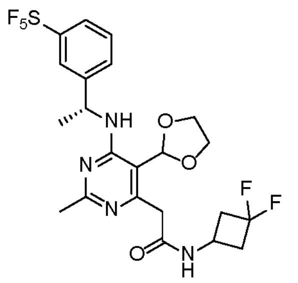

B16-1

(R)-2-(5-(1,3-dioxolan-2-yl)-2-methyl-6-((1-(3-(pentafluorosulfanyl)phenyl)ethyl)amino)pyrimidin-4-yl)acetic acid (291 mg, 0.62 mmol) was dissolved in a mixed solution of dimethyl sulfoxide (2 mL) and acetonitrile (1 mL), the reaction mixture was added with triethylamine (0.17 mL, 1.24 mmol), 2-(7-azobenzotriazole)-N,N,N',N'-tetramethyluronium hexafluorophosphate (354 mg, 0.93 mmol) and 3,3-difluorocyclobutanamine hydrochloride (116 mg, 0.80 mmol), and stirred at room temperature for 12 h. After the reaction was completed, the reaction mixture was diluted with ethyl acetate (10 mL), and washed with water (10 mL × 3) and saturated brine (10 mL × 3). The organic phase was concentrated, and the residue was purified by silica gel column chromatography (petroleum ether: ethyl acetate (V/V) = 1:3) to obtain the title compound (R)-2-(5-(1,3-dioxolan-2-yl)-2-methyl-6-((1-(3-(pentafluorosulfanyl)phenyl)ethyl)amino)pyrimidin-4-yl)-N-(3,3-difluorocyclobutyl)acetamide (B16-1) (300 mg, yield: 86.4%).

LC-MS, M/Z (ESI): 559.1 $[M+H]^+$.

Step 2: Synthesis of (R)-6-(3,3-difluorocyclobutyl)-2-methyl-4-((1-(3-(pentafluorosulfanyl)phenyl)ethyl)amino)pyrido[4,3-d]pyrimidin-7(6H)-one (**I-16**)

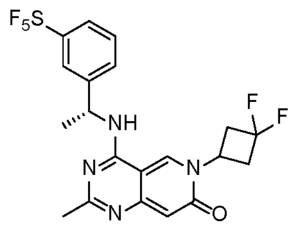

I-16

(R)-2-(5-(1,3-dioxolan-2-yl)-2-methyl-6-((1-(3-(pentafluorosulfanyl)phenyl)ethyl)amino)pyrimidin-4-yl)-N-(3,3-difluorocyclobutyl)acetamide (300 mg, 0.54 mmol) was dissolved in isopropanol (2 mL), the reaction mixture was added with 2 M hydrochloric acid (1 mL), and stirred at 50°C for 4 h. After the reaction was completed, the reaction mixture was directly concentrated, and the residue was purified by silica gel column chromatography (dichloromethane:methanol (V/V) = 10:1) to obtain the title compound (R)-6-(3,3-difluorocyclobutyl)-2-methyl-4-((1-(3-(pentafluorosulfanyl)phenyl)ethyl)amino)pyrido[4,3-d]pyrimidin-7(6H)-one (**I-16**, 150 mg, yield: 56.2%).

$^1H$ NMR (400 MHz, $CDCl_3$) δ 8.89 (s, 1H), 8.10 (s, 1H), 7.77 (s, 1H), 7.55 - 7.77 (m, 2H), 7.26 - 7.39 (m, 1H), 6.40 (s, 1H), 5.73 (t, 1H), 5.11 - 5.16 (m, 1H), 3.00 - 3.13 (m, 2H), 2.81 - 2.90 (m, 2H), 2.42 (s, 3H), 1.61 (d, 3H).

LC-MS, M/Z (ESI): 497.1 $[M+H]^+$.

**Embodiment 17: Synthesis of compound 1-17**

The synthetic route is as follows:

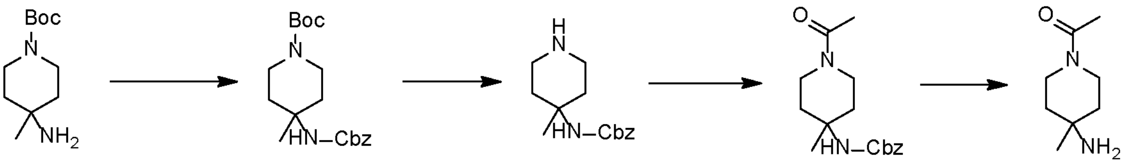

B17-1 B17-2 B17-3 B17-4 B17-5

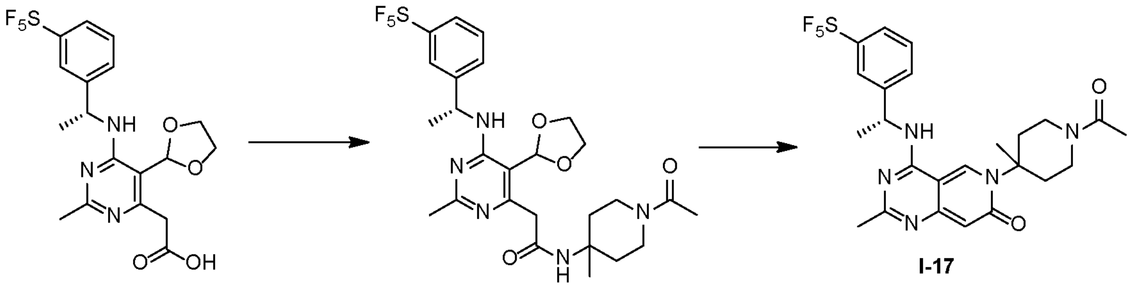

A2 B17-6 I-17

Step 1: Synthesis of tert-butyl 4-(((benzyloxy)carbonyl)amino)-4-methylpiperidine-1-carboxylate (**B17-2**)

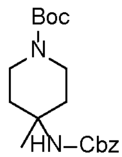

**B17-2**

tert-Butyl 4-amino-4-methylpiperidine-1-carboxylate (4 g, 18.67 mmol) was dissolved in a mixed solution of dioxane (20 mL) and water (20 mL), the reaction mixture was added with sodium bicarbonate (4.7 g, 56.0 mmol) and benzyl(2,5-dioxopyrrolidin-1-yl)carbonate (9.3 g, 37.3 mmol), and stirred at room temperature for 3 h. After the reaction was completed, the reaction mixture was diluted with ethyl acetate (100 mL), and washed with water (100 mL × 3) and saturated brine (100 mL × 3). The organic phase was concentrated, and the residue was purified by silica gel column chromatography (petroleum ether:ethyl acetate (V/V) = 10: 1) to obtain the title compound tert-butyl 4-(((benzyloxy)carbonyl)amino)-4-methylpiperidine-1-carboxylate (**B17-2**) (6.4 g, yield: 98%).

LC-MS, M/Z (ESI): 349.2 $[M+H]^+$.

Step 2: Synthesis of benzyl(4-methylpiperidin-4-yl)carbamate (**B17-3**)

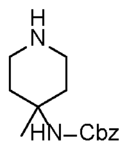

**B17-3**

*tert*-Butyl 4-(((benzyloxy)carbonyl)amino)-4-methylpiperidine-1-carboxylate (6.4 g, 18.37 mmol) was dissolved in a 4 M solution of hydrogen chloride in dioxane (9.18 mL), and the reaction mixture was stirred at room temperature for 12 h. After the reaction was completed, the reaction mixture was concentrated to obtain the title compound benzyl(4-methylpiperidin-4-yl)carbamate (**B17-3**) (5.23 g, yield: 100%).

LC-MS, M/Z (ESI): 249.1 $[M+H]^+$.

Step 3: Synthesis of benzyl(1-acetyl-4-methylpiperidin-4-yl)carbamate (**B17-4**)

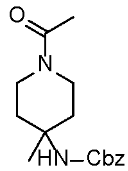

**B17-4**

Benzyl(4-methylpiperidin-4-yl)carbamate (5.23 g, 21.06 mmol) was dissolved in dichloromethane (50 mL), the reaction mixture was added with triethylamine (8.81 mL, 63.2 mmol) and acetic anhydride (3.23 g, 31.6 mmol), and stirred at room temperature for 3 h. After the reaction was completed, the reaction mixture was diluted with dichloromethane (50 mL), and washed with water (100 mL × 3) and saturated brine (100 mL × 3). The organic phase was concentrated, and the residue was purified by silica gel column chromatography (petroleum ether:ethyl acetate (V/V) = 5:1) to obtain the title compound benzyl(1-acetyl-4-methylpiperidin-4-yl)carbamate (**B17-4**) (4 g, yield: 65.3%).

LC-MS, M/Z (ESI): 291.2 $[M+H]^+$.

Step 4: Synthesis of 1-(4-amino-4-methylpiperidin-1-yl)ethan-1-one (**B17-5**)

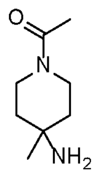

**B17-5**

Benzyl(1-acetyl-4-methylpiperidin-4-yl)carbamate (4 g, 13.78 mmol) was dissolved in methanol (40 mL), the reaction mixture was added with wet palladium on carbon (1 g, 10%), and stirred at room temperature under hydrogen atmosphere for 12 h. After the reaction was completed, the reaction mixture was filtered to obtain the title compound 1-(4-amino-4-methylpiperidin-1-yl)ethan-1-one (**B17-5**) (1.46 g, yield: 100%).

LC-MS, M/Z (ESI): 157.1 $[M+H]^+$.

Step 5: Synthesis of (R)-2-(5-(1,3-dioxolan-2-yl)-2-methyl-6-((1-(3-(pentafluorosulfanyl)phenyl)ethyl)amino)pyrimidin-4-yl)-N-(1-acetyl-4-methylpiperidin-4-yl)acetamide (**B17-6**)

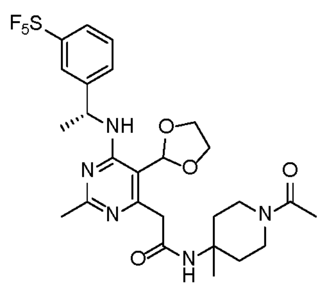

**B17-6**

(R)-2-(5-(1,3-dioxolan-2-yl)-2-methyl-6-((1-(3-(pentafluorosulfanyl)phenyl)ethyl)amino)pyrimidin-4-yl)acetic acid (583 mg, 1.24 mmol) was dissolved in a mixed solution of dimethyl sulfoxide (2 mL) and acetonitrile (1 mL), the reaction mixture was added with triethylamine (0.35 mL, 2.48 mmol), 2-(7-azobenzotriazole)-N,N,N',N'-tetramethyluronium hexafluorophosphate (708 mg, 1.86 mmol) and 1-(4-amino-4-methylpiperidin-1-yl)ethan-1-one (291 mg, 1.86

mmol), and stirred at room temperature for 12 h. After the reaction was completed, the reaction mixture was diluted with ethyl acetate (10 mL), and washed with water (10 mL × 3) and saturated brine (10 mL × 3). The organic phase was concentrated, and the residue was purified by silica gel column chromatography (petroleum ether: ethyl acetate (V/V) = 1:3) to obtain the title compound (R)-2-(5-(1,3-dioxolan-2-yl)-2-methyl-6-((1-(3-(pentafluorosulfanyl)phenyl)ethyl)amino)pyrimidin-4-yl)-N-(1-acetyl-4-methylpiperidin-4-yl)acetamide (**B17-6**) (150 mg, yield: 20.0%).

LC-MS, M/Z (ESI): 608.2 [M+H]$^+$.

Step 6: Synthesis of (R)-6-(1-acetyl-4-methylpiperidin-4-yl)-2-methyl-4-((1-(3-(pentafluorosulfanyl)phenyl)ethyl)amino)pyrido[4,3-d]pyrimidin-7(6H)-one (1-17)

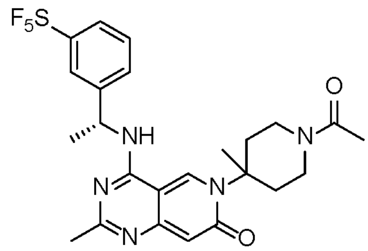

**I-17**

(R)-2-(5-(1,3-dioxolan-2-yl)-2-methyl-6-((1-(3-(pentafluorosulfanyl)phenyl)ethyl)amino)pyrimidin-4-yl)-N-(1-acetyl-4-methylpiperidin-4-yl)acetamide (150 mg, 0.25 mmol) was dissolved in isopropanol (2 mL), the reaction mixture was added with 2 M hydrochloric acid (1 mL), and stirred at 50°C for 4 h. After the reaction was completed, the reaction mixture was directly concentrated, and the residue was purified by silica gel column chromatography (dichloromethane:methanol (V/V) = 10:1) to obtain the title compound (R)-6-(1-acetyl-4-methylpiperidin-4-yl)-2-methyl-4-((1-(3-(pentafluorosulfanyl)phenyl)ethyl)amino)pyrido[4,3-d]pyrimidin-7(6H)-one (I-17, 100 mg, yield: 74.0%).

$^1$H NMR (400 MHz, DMSO-$d_6$) δ 8.79 (d, 1H), 8.72 (s, 1H), 7.94 (s, 1H), 7.70 - 7.77 (m, 2H), 7.55 - 7.59 (m, 1H), 6.04 (s, 1H), 5.59 - 5.64 (m, 1H), 3.87 - 3.97 (m, 1H), 3.40 - 3.41 (m, 1H), 3.39 - 3.41 (m, 1H), 3.31 - 3.37 (m, 1H), 2.48 - 2.50 (m, 1H), 2.19 - 2.25 (m, 3H), 2.18 (s, 3H), 1.98 (s, 3H), 1.72 (s, 3H), 1.59 (d, 3H).

LC-MS, M/Z (ESI): 546.1 [M+H]$^+$.

**Embodiment 18: Synthesis of compound 1-18**

The synthetic route is as follows:

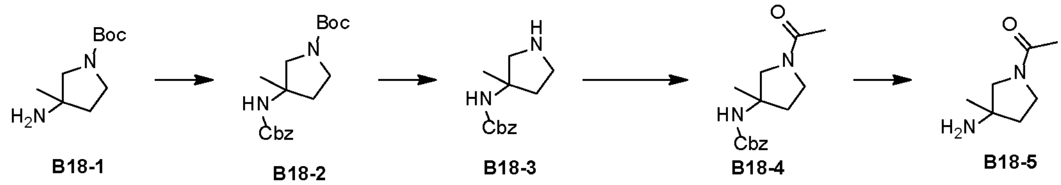

B18-1 B18-2 B18-3 B18-4 B18-5

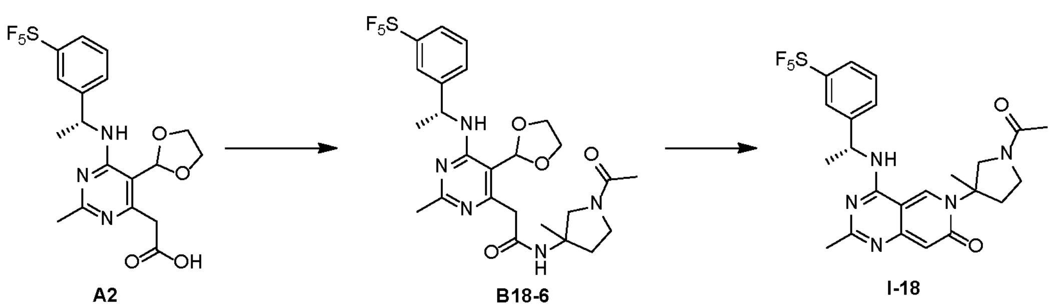

A2 B18-6 I-18

Step 1: Synthesis of *tert*-butyl 3-(((benzyloxy)carbonyl)amino)-3-methylpyrrolidine-1-carboxylate (**B18-2**)

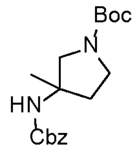

**B18-2**

*tert*-Butyl 3-amino-3-methylpyrrolidine-1-carboxylate (2 g, 9.99 mmol) was dissolved in a mixed solution of dioxane (10 mL) and water (10 mL), the reaction mixture was added with sodium bicarbonate (2.52 g, 30.0 mmol) and benzyl(2,5-dioxopyrrolidin-1-yl)carbonate (4.98 g, 19.97 mmol), and stirred at room temperature for 3 h. After the reaction was completed, the reaction mixture was diluted with ethyl acetate (50 mL), and washed with water (50 mL × 3) and saturated brine (50 mL × 3). The organic phase was concentrated, and the residue was purified by silica gel column chromatography (petroleum ether:ethyl acetate (V/V) = 10:1) to obtain the title compound tert-butyl 3-(((benzyloxy)carbonyl)amino)-3-methylpyrrolidine-1-carboxylate (**B18-2**) (3.3 g, yield: 98.8%).

LC-MS, M/Z (ESI): 335.2 $[M+H]^+$.

Step 2: Synthesis of benzyl(3-methylpyrrolidin-3-yl)carbamate (**B18-3**)

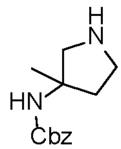

**B18-3**

*tert*-Butyl 3-(((benzyloxy)carbonyl)amino)-3-methylpyrrolidine-1-carboxylate (3.3 g, 9.87 mmol) was dissolved in a 4 M solution of hydrogen chloride in dioxane (8 mL), and the reaction mixture was stirred at room temperature for 12 h. After the reaction was completed, the reaction mixture was concentrated to obtain the title compound benzyl(3-methylpyrrolidin-3-yl)carbamate (**B18-3**) (2.6 g, yield: 97%).

LC-MS, M/Z (ESI): 235.1 $[M+H]^+$.

Step 3: Synthesis of benzyl(1-acetyl-3-methylpyrrolidin-3-yl)carbamate (**B18-4**)

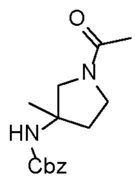

**B18-4**

Benzyl(3-methylpyrrolidin-3-yl)carbamate (2.6 g, 11.10 mmol) was dissolved in dichloromethane (50 mL), the reaction mixture was added with triethylamine (4.64 mL, 33.3 mmol) and acetic anhydride (2.27 g, 22.19 mmol), and stirred at room temperature for 3 h. After the reaction was completed, the reaction mixture was diluted with dichloromethane (50 mL), and washed with water (100 mL × 3) and saturated brine (100 mL × 3). The organic phase was concentrated, and the residue was purified by silica gel column chromatography (petroleum ether:ethyl acetate (V/V) = 5:1) to obtain the title compound benzyl(1-acetyl-3-methylpyrrolidin-3-yl)carbamate (B18-4) (2.4 g, yield: 78.2%).

LC-MS, M/Z (ESI): 277.1 $[M+H]^+$.

Step 4: Synthesis of 1-(3-amino-3-methylpyrrolidin-1-yl)ethan-1-one (**B18-5**)

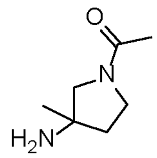

**B18-5**

Benzyl(1-acetyl-3-methylpyrrolidin-3-yl)carbamate (2.4 g, 8.69 mmol) was dissolved in methanol (24 mL), the reaction mixture was added with wet palladium on carbon (240 mg, 10%), and stirred at room temperature under hydrogen atmosphere for 12 h. After the reaction was completed, the reaction mixture was filtered and concentrated to obtain the title compound 1-(3-amino-3-methylpyrrolidin-1-yl)ethan-1-one (B18-5) (1.23 g, yield: 100%).

LC-MS, M/Z (ESI): 143.1 $[M+H]^+$.

Step 5: Synthesis of 2-(5-(1,3-dioxolan-2-yl)-2-methyl-6-(((R)-1-(3-(pentafluorosulfanyl)phenyl)ethyl)amino)pyrimidin-4-yl)-N-(1-acetyl-3-methylpyrrolidin-3-yl)acetamide (**B18-6**)

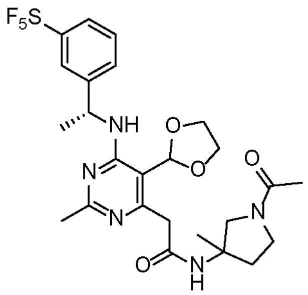

**B18-6**

(R)-2-(5-(1,3-dioxolan-2-yl)-2-methyl-6-((1-(3-(pentafluorosulfanyl)phenyl)ethyl)amino)pyrimidin-4-yl)acetic acid (583 mg, 1.24 mmol) was dissolved in a mixed solution of dimethyl sulfoxide (2 mL) and acetonitrile (1 mL), the reaction mixture was added with triethylamine (0.35 mL, 2.48 mmol), 2-(7-azobenzotriazole)-N,N,N',N'-tetramethyluronium hexafluorophosphate (708 mg, 1.86 mmol) and 1-(3-amino-3-methylpyrrolidin-1-yl)ethan-1-one (176 mg, 1.24 mmol), and stirred at room temperature for 12 h. After the reaction was completed, the reaction mixture was diluted with ethyl acetate (10 mL), and washed with water (10 mL × 3) and saturated brine (10 mL × 3). The organic phase was concentrated, and the residue was purified by silica gel column chromatography (petroleum ether:ethyl acetate (V/V) = 1:3) to obtain the title compound 2-(5-(1,3-dioxolan-2-yl)-2-methyl-6-(((R)-1-(3-(pentafluorosulfanyl)phenyl)ethyl)amino)pyrimidin-4-yl)-N-(1-acetyl-3-methylpyrrolidin-3-yl)acetamide (**B18-6**) (100 mg, yield: 13.6%).

LC-MS, M/Z (ESI): 594.2 $[M+H]^+$.

Step 6: Synthesis of 6-(1-acetyl-3-methylpyrrolidin-3-yl)-2-methyl-4-(((R)-1-(3-(pentafluorosulfanyl)phenyl)ethyl)amino)pyrido[4,3-d]pyrimidin-7(6H)-one (**I-18**)

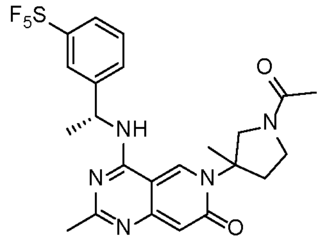

**I-18**

2-(5-(1,3-Dioxolan-2-yl)-2-methyl-6-(((R)-1-(3-(pentafluorosulfanyl)phenyl)ethyl)amino)pyrimidin-4-yl)-N-(1-acetyl-3-methylpyrrolidin-3-yl)acetamide (100 mg, 0.17 mmol) was dissolved in isopropanol (2 mL), the reaction mixture was added with 2 M hydrochloric acid (1 mL), and stirred at 50°C for 4 h. After the reaction was completed, the reaction mixture was directly concentrated, and the residue was purified by silica gel column chromatography (dichloromethane:methanol (V/V) = 10:1) to obtain the title compound 6-(1-acetyl-3-methylpyrrolidin-3-yl)-2-methyl-4-(((R)-1-(3-(pentafluorosulfanyl)phenyl)ethyl)amino)pyrido[4,3-d]pyrimidin-7(6H)-one (**I-18**, 80 mg, yield: 88.9.0%).

$^1$H NMR (400 MHz, DMSO-*d*$_6$) $\delta$ 8.83 (s, 1H), 7.93 (s, 1H), 7.76 (d, 1H), 7.70 (d, 1H), 7.55 - 7.59 (m, 1H), 6.06 (d, 1H), 5.57 - 5.64 (m, 1H), 4.39 - 4.44 (m, 1H), 3.59 - 3.67 (m, 2H), 3.32 - 3.3.33 (m, 1H), 2.70 - 2.73 (m, 1H), 2.48 - 2.52 (m, 1H), 2.20 (d, 3H), 1.94 (q, 3H), 1.60 (d, 3H), 1.54 (q, 3H).

LC-MS, M/Z (ESI): 532.1 [M+H]$^+$.

**Embodiment 19: Synthesis of compound 1-19**

The synthetic route is as follows:

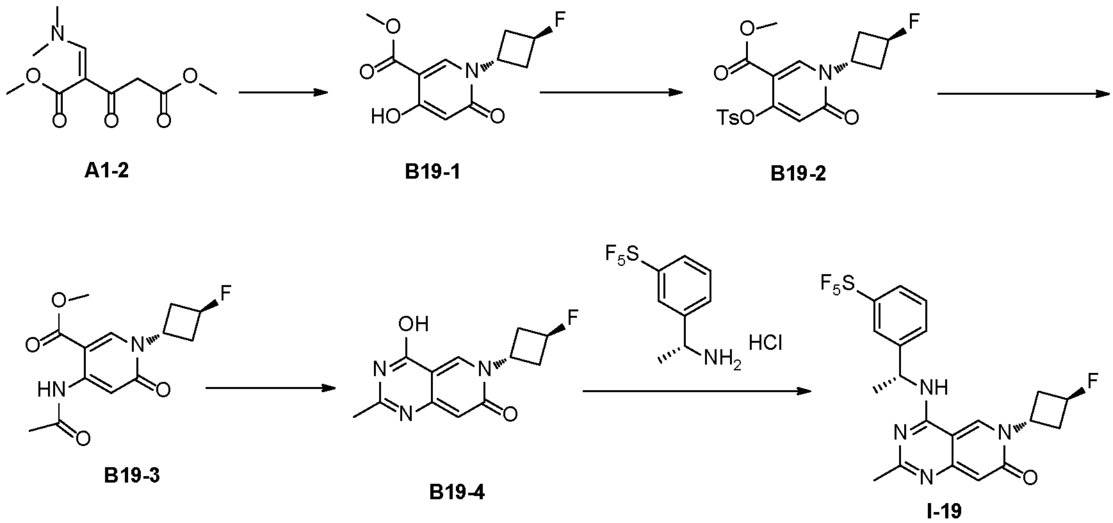

**A1-2** **B19-1** **B19-2**

**B19-3** **B19-4** **I-19**

Step 1: Synthesis of methyl 1-((1r,3r)-3-fluorocyclobutyl)-4-hydroxy-6-oxo-1,6-dihydropyridine-3-carboxylate (**B19-1**)

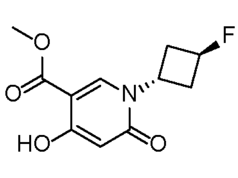

**B19-1**

To 2-methyltetrahydrofuran (30 mL) was added dimethyl (Z)-2-((dimethylamino)methylene)-3-oxoglutarate (1.95 g, 9.65 mmol) at room temperature, the reaction mixture was added with 4 N hydrochloric acid (10 mL), and stirred for 3 h. The reaction mixture was separated, and the aqueous phase was extracted with ethyl acetate (100 mL × 3).

The organic phases were combined, dried over anhydrous sodium sulfate, filtered and concentrated. The reaction mixture was added with methanol (30 mL), then added with (1r,3r)-3-fluorocyclobutanamine hydrochloride (1.21 g, 9.65 mmol), and stirred at room temperature for 16 h. The system was added with sodium methoxide (1.04 g, 19.29 mmol), and stirred for 2 h. The reaction mixture was added with concentrated hydrochloric acid to adjust the pH to 2, and filtered to obtain a crude product of the title compound methyl 1-((1r,3r)-3-fluorocyclobutyl)-4-hydroxy-6-oxo-1,6-dihydropyridine-3-carboxylate (**B19-1**) (1.35 g, yield: 58.2%) as a white solid.

LC-MS, M/Z (ESI): 242.1 $[M+H]^+$.

Step 2: Synthesis of methyl 1-((1r,3r)-3-fluorocyclobutyl)-6-oxo-4-(toluenesulfonyloxy)-1,6-dihydropyridine-3-carboxylate (**B19-2**)

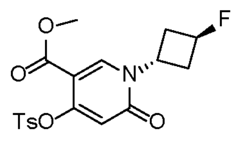

**B19-2**

To acetonitrile (20 mL) was added methyl 1-((1r,3r)-3-fluorocyclobutyl)-4-hydroxy-6-oxo-1,6-dihydropyridine-3-carboxylate (1.35 g, 5.60 mmol) at room temperature, the reaction mixture was cooled to 0°C, added with triethylamine (1.56 mL, 11.19 mmol) and *p*-toluenesulfonyl chloride (1.28 g, 6.72 mmol), heated to room temperature, and stirred for 2 h. The reaction mixture was concentrated and purified by silica gel column chromatography (petroleum ether:ethyl acetate (V/V) = 5:1 to 1:1) to obtain the title compound methyl 1-((1r,3r)-3-fluorocyclobutyl)-6-oxo-4-(toluenesulfonyloxy)-1,6-dihydropyridine-3-carboxylate (**B19-2**) (2.2 g, yield: 99%) as a white solid.

LC-MS, M/Z (ESI): 396.1 $[M+H]^+$.

Step 3: Synthesis of methyl 4-acetamido-1-((1r,3r)-3-fluorocyclobutyl)-6-oxo-1,6-dihydropyridine-3-carboxylate (**B19-3**)

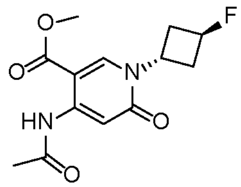

**B19-3**

To dioxane (50 mL) was added methyl 1-((1r,3r)-3-fluorocyclobutyl)-6-oxo-4-(toluenesulfonyloxy)-1,6-dihydropyridine-3-carboxylate (1.2 g, 3.0 mmol) at room temperature, the reaction mixture was added with potassium phosphate (700 mg, 3.3 mmol), Xantphos (173 mg, 0.3 mmol) and palladium ($\pi$-cinnamyl) chloride dimer (212 mg, 0.3 mmol), heated and stirred at reflux for 2 h under nitrogen atmosphere. The reaction mixture was cooled to room temperature, concentrated and purified by silica gel column chromatography (petroleum ether:ethyl acetate (V/V) = 3:1 to 1:1) to obtain the title compound methyl 4-acetamido-1-((1r,3r)-3-fluorocyclobutyl)-6-oxo-1,6-dihydropyridine-3-carboxylate (**B19-3**) (1.4 g, yield: 87%) as a white solid.

LC-MS, M/Z (ESI): 283.1 $[M+H]^+$.

Step 4: Synthesis of 6-((1r,3r)-3-fluorocyclobutyl)-4-hydroxy-2-methylpyrido[4,3-d]pyrimidin-7(6H)-one

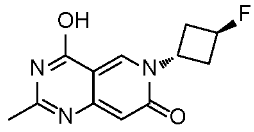

B19-4

To a 7 M solution of ammonia in methanol (10 mL) was added methyl 4-acetamido-1-((1r,3r)-3-fluorocyclobutyl)-6-oxo-1,6-dihydropyridine-3-carboxylate (1.4 g, 4.96 mmol) at room temperature, and the reaction mixture was stirred at room temperature for 5 d. The reaction mixture was concentrated to 3 mL and filtered to obtain the title compound 6-((1r,3r)-3-fluorocyclobutyl)-4-hydroxy-2-methylpyrido[4,3-d]pyrimidin-7(6H)-one (1 g, yield: 81%) as a white solid.
LC-MS, M/Z (ESI): 250.1 $[M+H]^+$.

Step 5: Synthesis of 6-((1r,3R)-3-fluorocyclobutyl)-2-methyl-4-(((R)-1-(3-(pentafluorosulfanyl)phenyl)ethyl)amino)pyrido[4,3-d]pyrimidin-7(6H)-one (**I-19**)

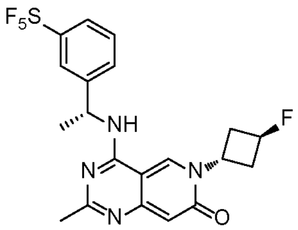

I-19

To acetonitrile (10 mL) was added 6-((1r,3r)-3-fluorocyclobutyl)-4-hydroxy-2-methylpyrido[4,3-d]pyrimidin-7(6H)-one (200 mg, 0.80 mmol), the reaction mixture was added with compound (R)-1-(3-(pentafluorosulfanyl)phenyl)ethan-1-amine hydrochloride (250 mg, 0.88 mmol) and anhydrous potassium phosphate (426 mg, 2.0 mmol), stirred at room temperature for 24 h, then added with phosphonitrilic chloride trimer (418 mg, 1.20 mmol) and triethylamine (0.28 mL, 1.60 mmol), and stirred continuously at room temperature for 24 h. The reaction mixture was concentrated under reduced pressure and purified by silica gel column chromatography (dichloromethane:methanol = 50:1 to 10:1) to obtain 6-((1r,3R)-3-fluorocy cl obutyl)-2-m ethyl-4-(((R)-1-(3 - (pentafluorosulfanyl)phenyl)ethyl)amino)pyrido[4,3-d] pyrimidin-7(6H)-one (**I-19**, 150 mg, yield: 39.1%) as a white solid.
$^1H$ NMR (400 MHz, $CDCl_3$) δ 8.89 (s, 1H), 7.82 (s, 1H), 7.59 - 7.62 (m, 3H), 7.36 - 7.40 (m, 1H), 6.40 (s, 1H), 5.70 - 5.72 (m, 1H), 5.33 - 5.37 (m, 1H), 5.23 (s, 1H), 5.09 (s, 1H), 2.72 - 2.78 (m, 4H), 2.43 (s, 3H), 1.66 (d, 3H).
LC-MS, M/Z (ESI): 479.1 $[M+H]^+$.

**Embodiment 20: Synthesis of compound 1-20**

The compound synthetic route is as follows:

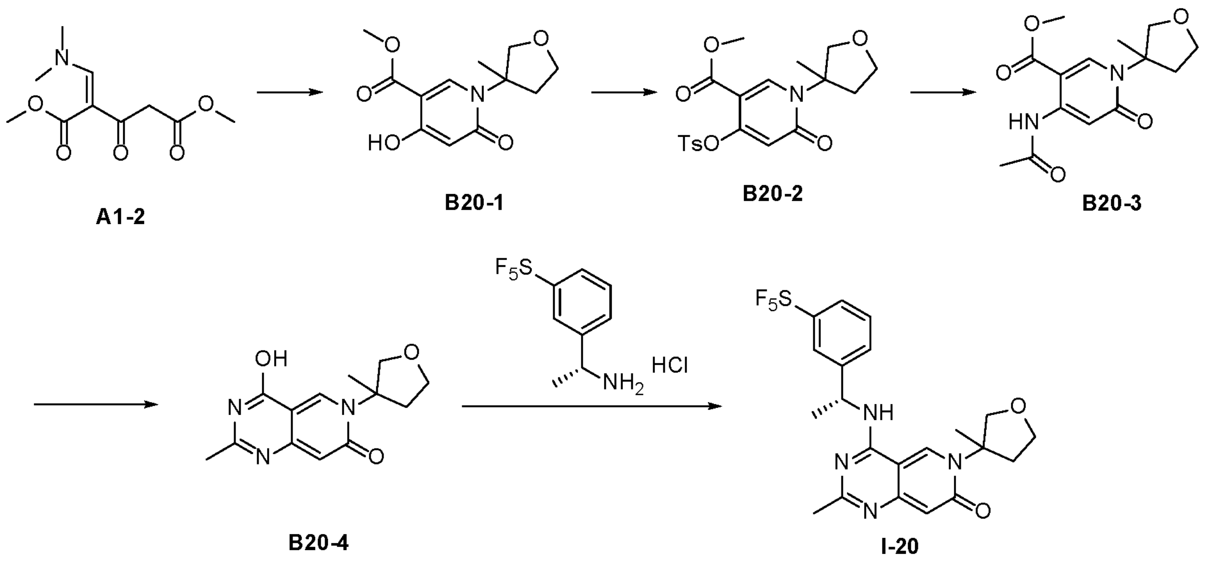
A1-2 B20-1 B20-2 B20-3

B20-4 I-20

Step 1: Synthesis of methyl 4-hydroxy-1-(3-methyltetrahydrofuran-3-yl)-6-oxo-1,6-dihydropyridine-3-carboxylate (**B20-1**)

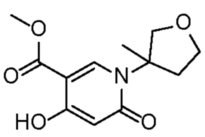

B20-1

To 2-methyltetrahydrofuran (30 mL) was added dimethyl (Z)-2-((dimethylamino)methylene)-3-oxoglutarate (1.3 g, 5.67 mmol) at room temperature, the reaction mixture was added with 4 N hydrochloric acid (10 mL), and stirred for 3 h. The liquid was separated, and the aqueous phase was extracted with ethyl acetate (100 mL × 3). The organic phases were combined, dried over anhydrous sodium sulfate, filtered, concentrated, added with methanol (30 mL), then added with 3-methyltetrahydrofuran-3-amine (0.57 g, 5.67 mmol), and stirred at room temperature for 16 h. The system was added with sodium methoxide (0.61 g, 11.34 mmol), and stirred for 2 h. The reaction mixture was added with concentrated hydrochloric acid to adjust the pH to 2, and filtered to obtain a crude product of the title compound methyl 4-hydroxy-1-(3-methyltetrahydrofuran-3-yl)-6-oxo-1,6-dihydropyridine-3-carboxylate (**B20-1**) (0.7 g, yield: 48.7%) as a white solid.

LC-MS, M/Z (ESI): 254.1 $[M+H]^+$.

Step 2: Synthesis of methyl 1-(3-methyltetrahydrofuran-3-yl)-6-oxo-4-(p-toluenesulfonyloxy)-1,6-dihydropyridine-3-carboxylate (**B20-2**)

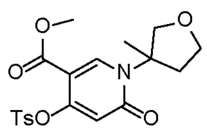

B20-2

To acetonitrile (20 mL) was added methyl 4-hydroxy-1-(3-methyltetrahydrofuran-3-yl)-6-oxo-1,6-dihydropyridine-3-carboxylate (0.7 g, 2.76 mmol) at room temperature, the reaction mixture was cooled to 0°C, added with triethylamine (0.77 mL, 5.53 mmol) and p-toluenesulfonyl chloride (553 mg, 2.90 mmol), heated to room temperature, and stirred for 2 h. The reaction mixture was concentrated and purified by silica gel column chromatography (petroleum

ether: ethyl acetate (V/V) = 5:1 to 1:1) to obtain the title compound methyl 1-(3-methyltetrahydrofuran-3-yl)-6-oxo-4-(p-toluenesulfonyloxy)-1,6-dihydropyridine-3-carboxylate (**B20-2**) (1 g, yield: 89%) as a white solid.
LC-MS, M/Z (ESI): 408.1 $[M+H]^+$.

Step 3: Synthesis of methyl 4-acetamido-1-(3-methyltetrahydrofuran-3-yl)-6-oxo-1,6-dihydropyridine-3-carboxylate (**B20-3**)

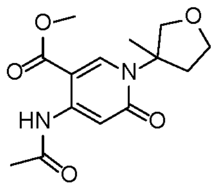

**B20-3**

To dioxane (50 mL) was added methyl 1-(3-methyltetrahydrofuran-3-yl)-6-oxo-4-(p-toluenesulfonyloxy)-1,6-dihydropyridine-3-carboxylate (1 g, 2.45 mmol) at room temperature, the reaction mixture was added with potassium phosphate (0.78 g, 3.68 mmol), Xantphos (142 mg, 0.25 mmol) and palladium ($\pi$-cinnamyl) chloride dimer (225 mg, 0.25 mmol), heated at reflux for 2 h under nitrogen atmosphere. The reaction mixture was cooled to room temperature, concentrated and purified by silica gel column chromatography (petroleum ether: ethyl acetate (V/V) = 3:1 to 1:1) to obtain the title compound methyl 4-acetamido-1-(3-methyltetrahydrofuran-3-yl)-6-oxo-1,6-dihydropyridine-3-carboxylate (**B20-3**) (0.56 g, yield: 78%) as a white solid.
LC-MS, M/Z (ESI): 295.1 $[M+H]^+$.

Step 4: Synthesis of 4-hydroxy-2-methyl-6-(3-methyltetrahydrofuran-3-yl)pyrido[4,3-d]pyrimidin-7(6H)-one

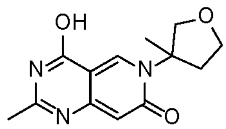

**B20-4**

To a 7 M solution of ammonia in methanol (10 mL) was added methyl 4-acetamido-1-(3-methyltetrahydrofuran-3-yl)-6-oxo-1,6-dihydropyridine-3-carboxylate (560 mg, 1.90 mmol) at room temperature, and the reaction mixture was stirred at room temperature for 12 h. The reaction mixture was concentrated to 3 mL and filtered to obtain the title compound 4-hydroxy-2-methyl-6-(3-methyltetrahydrofuran-3-yl)pyrido[4,3-d]pyrimidin-7(6H)-one (350 mg, yield: 70.4%) as a white solid.
LC-MS, M/Z (ESI): 262.1 $[M+H]^+$.

Step 5: Synthesis of 2-methyl-6-(3-methyltetrahydrofuran-3-yl)-4-(((R)-1-(3-(pentafluorosulfanyl)phenyl)ethyl)amino)pyrido[4,3-d]pyrimidin-7(6H)-one (**I-20**)

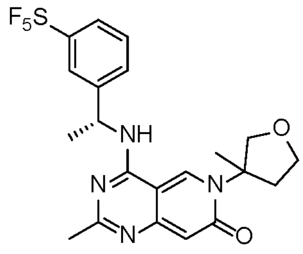

I-20

To acetonitrile (10 mL) was added 4-hydroxy-2-methyl-6-(3-methyltetrahydrofuran-3-yl)pyrido[4,3-d]pyrimidin-7(6H)-one (350 mg, 1.34 mmol), the reaction mixture was added with compound (R)-1-(3-(pentafluorosulfanyl)phenyl)ethan-1-amine hydrochloride (418 mg, 1.47 mmol) and anhydrous potassium phosphate (711 mg, 3.35 mmol), stirred at room temperature for 24 h, then added with phosphonitrilic chloride trimer (699 mg, 2.01 mmol) and triethylamine (0.47 mL, 2.68 mmol), and stirred continuously at room temperature for 24 h. The reaction mixture was concentrated under reduced pressure and purified by silica gel column chromatography (dichloromethane:methanol = 50:1 to 10:1) to obtain 2-methyl-6-(3-methyltetrahydrofuran-3-yl)-4-(((R)-1-(3 - (pentafluorosulfanyl)phenyl)ethyl)amino)pyrido[4,3-d] pyrimidin-7(6H)-one (**I-20**, 200 mg, yield: 30.4%) as a white solid.

$^1H$ NMR (400 MHz, $CDCl_3$) $\delta$ 8.60 (s, 1H), 7.86 (s, 1H), 7.62 - 7.65 (m, 2H), 7.42 - 7.45 (m, 1H), 6.40 (d, 1H), 5.68 - 5.72 (m, 1H), 4.38 - 4.42 (m, 1H), 4.02 - 4.06 (m, 1H), 3.95 - 3.97 (m, 2H), 2.51 - 2.57 (m, 2H), 2.43 (s, 3H), 1.68 - 1.74 (m, 6H).

LC-MS, M/Z (ESI): 491.1 $[M+H]^+$.

**Embodiment 21: Synthesis of compound 1-21**

The synthetic route is as follows:

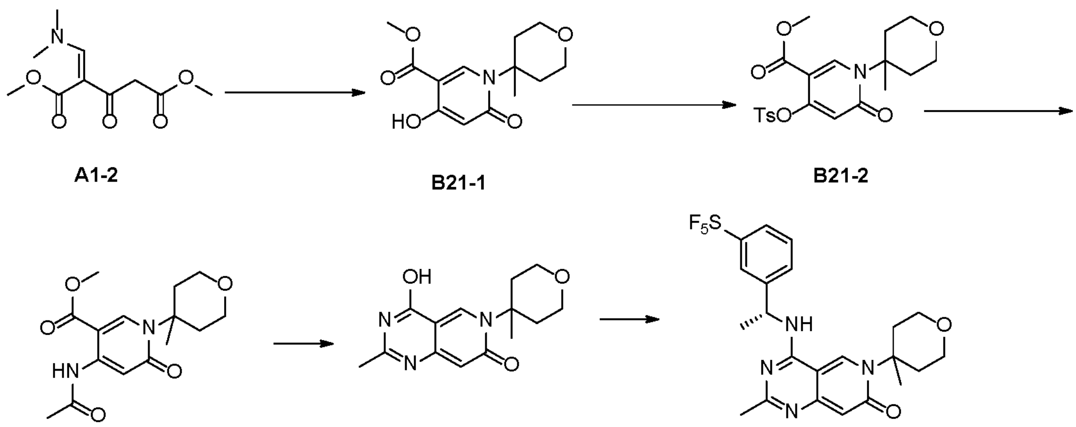

A1-2 B21-1 B21-2

B21-3 B21-4 I-21

Step 1: Synthesis of methyl 4-hydroxy-1-(4-methyltetrahydro-2H-pyran-4-yl)-6-oxo-1,6-dihydropyridine-3-carboxylate (**B21-1**)

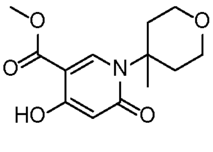

B21-1

To methanol (60 mL) was added compound dimethyl (Z)-2-((dimethylamino)methylene)-3-oxoglutarate (12 g, 52.3 mmol) at room temperature, the reaction mixture was added with 4-methyltetrahydro-2H-pyran-4-amine hydrochloride (7.9 g, 52.3 mmol), and stirred at room temperature for 16 h. The system was added with sodium methoxide (6.5 g, 120.0 mmol), and stirred for 2 h. The reaction mixture was added with concentrated hydrochloric acid to adjust the pH to 2, and filtered to obtain a crude product of the title compound methyl 4-hydroxy-1-(4-methyltetrahydro-2H-pyran-4-yl)-6-oxo-1,6-dihydropyridine-3-carboxylate (2.2 g, yield: 15.9%) as a brown solid.

LC-MS, M/Z (ESI): 268.1 $[M+H]^+$.

Step 2: Synthesis of methyl 1-(4-methyltetrahydro-2H-pyran-4-yl)-6-oxo-4-(tolyloxy)-1,6-dihydropyridine-3-carboxylate (**B21-2**)

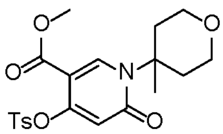

**B21-2**

To acetonitrile (20 mL) was added raw material methyl 4-hydroxy-1-(4-methyltetrahydro-2H-pyran-4-yl)-6-oxo-1,6-dihydropyridine-3-carboxylate (2.2 g, 8.3 mmol) at room temperature, the reaction mixture was cooled to 0°C, added with triethylamine (1.68 g, 16.6 mmol) andp-toluenesulfonyl chloride (1.58 g, 8.3 mmol), heated to room temperature, and stirred for 2 h. The reaction mixture was concentrated and purified by silica gel column chromatography (petroleum ether: ethyl acetate (V/V) = 5:1 to 1:1) to obtain the title compound methyl 1-(4-methyltetrahydro-2H-pyran-4-yl)-6-oxo-4-(tolyloxy)-1,6-dihydropyridine-3-carboxylate (1.26 g, yield: 57.2%) as a white solid.

LC-MS, M/Z (ESI): 422.1 $[M+H]^+$.

Step 3: Synthesis of methyl 4-acetamido-1-(4-methyltetrahydro-2H-pyran-4-yl)-6-oxo-1,6-dihydropyridine-3-carboxylate (**B21-3**)

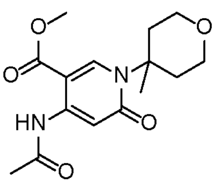

**B21-3**

To dioxane (50 mL) was added raw material methyl 1-(4-methyltetrahydro-2H-pyran-4-yl)-6-oxo-4-(tolyloxy)-1,6-dihydropyridine-3-carboxylate (1.26 g, 3.0 mmol) at room temperature, the reaction mixture was added with potassium phosphate (700 mg, 3.3 mmol), Xantphos (173 mg, 0.3 mmol) and palladium ($\pi$-cinnamyl) chloride dimer (212 mg, 0.3 mmol), heated and stirred at reflux for 2 h under nitrogen atmosphere. The reaction mixture was cooled to room temperature, concentrated and purified by silica gel column chromatography (petroleum ether:ethyl acetate (V/V) = 3:1 to 1:1) to obtain the title compound methyl 4-acetamido-1-(4-methyltetrahydro-2H-pyran-4-yl)-6-oxo-1,6-dihydropyridine-3-carboxylate (742 mg, yield: 79.3%) as a white solid.

LC-MS, M/Z (ESI): 309.1 $[M+H]^+$.

Step 4: Synthesis of 4-hydroxy-2-methyl-6-(4-methyltetrahydro-2H-pyran-4-yl)pyrido[4,3-d]pyrimidin-7(6H)-one (**B21-4**)

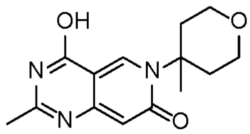

**B21-4**

To a 7 mol/L solution of ammonia in methanol (10 mL) was added raw material methyl 4-acetamido-1-(4-methyltetrahydro-2H-pyran-4-yl)-6-oxo-1,6-dihydropyridine-3-carboxylate (742 mg, 2.41 mmol) at room temperature, and the reaction mixture was stirred at room temperature for 5 d. The reaction mixture was concentrated to 3 mL and filtered to obtain the title compound 4-hydroxy-2-methyl-6-(4-methyltetrahydro-2H-pyran-4-yl)pyrido[4,3-d]pyrimidin-7(6H)-one (220 mg, yield: 33.5%) as a white solid.
LC-MS, M/Z (ESI): 276.1 $[M+H]^+$.

Step 5: Synthesis of (R)-2-methyl-6-(4-methyltetrahydro-2H-pyran-4-yl)-4-((1-(3-(pentafluorosulfanyl)phenyl)ethyl)amino)pyrido[4,3-d]pyrimidin-7(6H)-one (**I-21**)

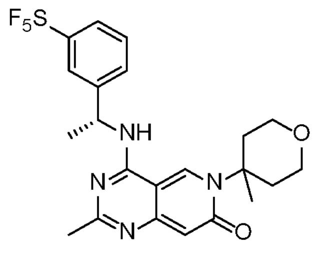

**I-21**

To acetonitrile (20 mL) was added raw material 4-hydroxy-2-methyl-6-(4-methyltetrahydro-2H-pyran-4-yl)pyrido[4,3-d]pyrimidin-7(6H)-one (220 mg, 0.80 mmol) at room temperature, the reaction mixture was added with potassium phosphate (678 mg, 3.20 mmol) and phosphonitrilic chloride trimer (416 mg, 1.20 mmol), and stirred at room temperature for 16 h. To DCM (10 mL) was added raw material (R)-1-(3-(pentafluorosulfanyl)phenyl)ethan-1-amine hydrochloride (246 mg, 0.87 mmol), the reaction mixture was added with DIPEA (2 mL), and stirred for 0.5 h. The above system was added with the reaction mixture, and stirred at room temperature for 6 h. The reaction mixture was concentrated and purified by silica gel column chromatography (dichloromethane:methanol = 50:1 to 10:1) to obtain (R)-2-methyl-6-(4-methyltetrahydro-2H-pyran-4-yl)-4-((1-(3-(pentafluorosulfanyl)phenyl)ethyl)amino)pyrido[4,3-d]pyrimidin-7(6H)-one (I-21, 40 mg, yield: 9.9%) as a white solid.
$^1H$ NMR (400 MHz, DMSO-$d_6$) δ 8.90 (s, 1H), 8.75 (s, 1H), 7.94 (s, 1H), 7.77 - 7.71 (m, 2H), 7.57 (t, 1H), 6.04 (s, 1H), 5.63 (m, 1H), 3.75 - 3.62 (m, 4H), 2.48 - 2.41 (m, 2H), 2.30 - 2.23 (m, 2H), 2.20 (s, 3H), 1.72 (s, 3H), 1.60 (d, 3H).
LC-MS, M/Z (ESI): 505.1 $[M+H]^+$.

**Embodiment 22: Synthesis of compound 1-22**

The synthetic route is as follows:

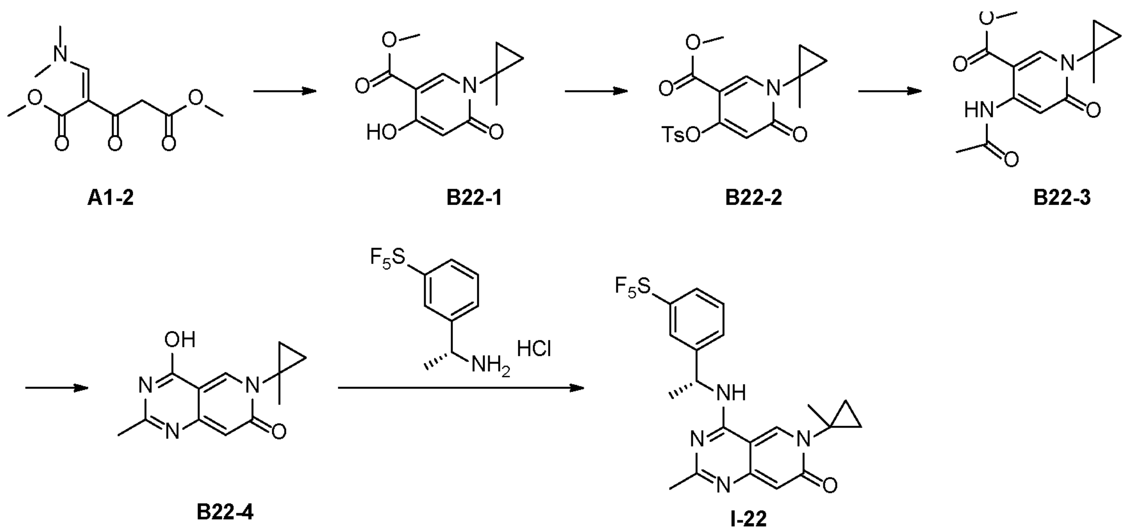

A1-2 B22-1 B22-2 B22-3

B22-4 I-22

To acetonitrile (10 mL) was added raw material 6-(1-(fluoromethyl)cyclopropyl)-4-hydroxy-2-methylpyrido[4,3-d]pyrimidin-7(6H)-one (7) (100 mg, 0.43 mmol), the reaction mixture was then added with compound (R)-1-(3-(pentafluorosulfanyl)phenyl)ethan-1-amine hydrochloride (135 mg, 0.48 mmol) and anhydrous potassium phosphate (229 mg, 1.08 mmol), stirred at room temperature for 24 h, then added with phosphonitrilic chloride trimer (150 mg, 0.43 mmol) and triethylamine (137 mg, 1.29 mmol), and stirred continuously at room temperature for 24 h. The reaction mixture was concentrated under reduced pressure and purified by silica gel column chromatography (dichloromethane:methanol = 50:1 to 10:1) to obtain (R)-2-methyl-6-(1-methylcyclopropyl)-4-((1-(3-(pentafluorosulfanyl)phenyl)ethyl)amino)pyrido[4,3-d]pyrimidin-7(6H)-one (**I-22**, 42 mg, yield: 22.1% as a white solid.

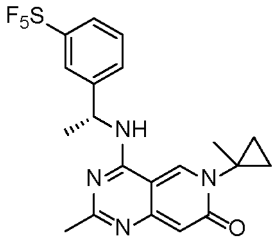

**I-22**

$^1$H NMR (400 MHz, DMSO) δ 9.23 (s, 1H), 8.90 (s, 1H), 7.95 (s, 1H), 7.74 (t, *J* = 9.0 Hz, 2H), 7.49 - 7.63 (m, 2H), 5.48 - 5.66 (m, 1H), 2.11 - 2.25 (m, 3H), 1.59 (d, *J* = 7.1 Hz, 3H), 1.49 (d, *J* = 14.2 Hz, 3H), 1.27 - 1.37 (m, 1H), 1.21 (s, 1H), 1.09 (t, *J* = 13.0 Hz, 1H), 0.94 - 1.05 (m, 1H).

LC-MS, M/Z (ESI): 461.5 $[M+H]^+$.

**Embodiment 23: Synthesis of target compound 1-23**

The synthetic route is as follows:

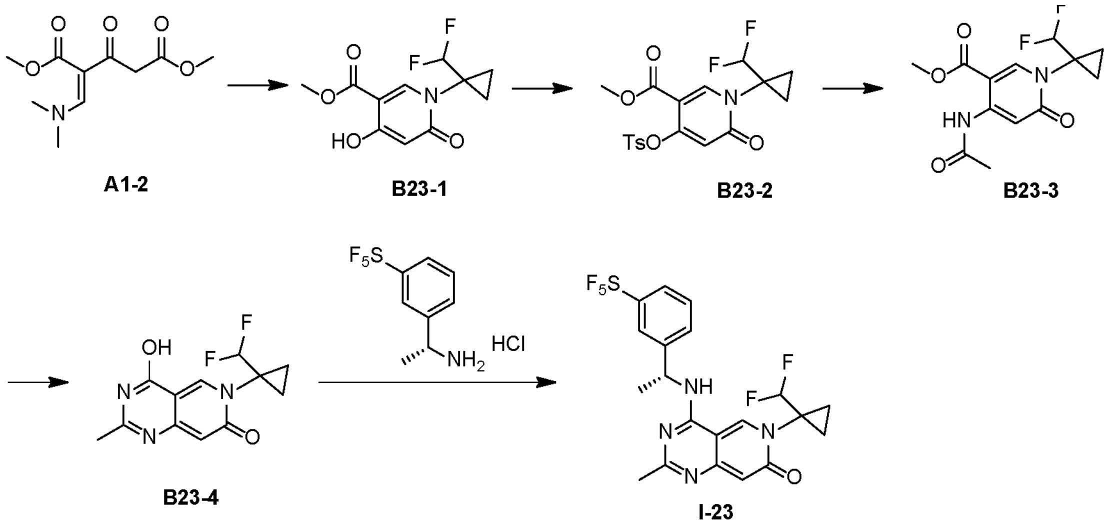

Step 1: Synthesis of methyl 1-(1-(difluoromethyl)cyclopropyl)-4-hydroxy-6-oxo-1,6-dihydropyridine-3-carboxylate (**B23-1**)

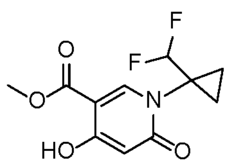

**B23-1**

Compound dimethyl (Z)-2-((dimethylamino)methylene)-3-oxoglutarate (5.0 g, 21.81 mmol) and 1-(difluoromethyl)cyclopropane-1-amine hydrochloride (3.44 g, 23.99 mmol) were dissolved in methanol (50.0 mL), and the reaction system was stirred at room temperature for 16 h. The reaction mixture was then added with sodium methoxide (1.76 g, 32.58 mmol), and the reaction system was stirred at 25°C for 0.5 h. The reaction system was added with HCl (1.0 N, 15.0 mL) to adjust the pH to 1 to 2, at which time a white solid was precipitated. The reaction mixture was filtered, and the filter cake was washed with 10.0 mL of methanol and dried to obtain compound methyl 1-(1-(difluoromethyl)cyclopropyl)-4-hydroxy-6-oxo-1,6-dihydropyridine-3-carboxylate (**B23-1**) (2.4 g, yield: 42.5%) as a white solid.
LC-MS, M/Z (ESI): 260.3 $[M+H]^+$.

Step 2: Synthesis of methyl 1-(1-(difluoromethyl)cyclopropyl)-6-oxo-4-(toluenesulfonyloxy)-1,6-dihydropyridine-3-carboxylate (**B23-2**)

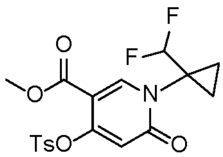

**B23-2**

Methyl 1-(1-(difluoromethyl)cyclopropyl)-4-hydroxy-6-oxo-1,6-dihydropyridine-3-carboxylate (2.4 g, 9.26 mmol) was dissolved in acetonitrile (24.0 mL), the reaction system was then added with triethylamine (1.41 g, 13.89 mmol), slowly added with *p*-toluenesulfonyl chloride (1.77 g, 9.26 mmol) in batches, and the reaction system was stirred at 25°C for 2 h. The reaction mixture was diluted with dichloromethane (20.0 mL), and added with 1 N HCl (10.0 mL) to adjust the pH to 2 to 3. The reaction mixture was extracted, and the organic phase was evaporated to dryness by rotary evaporation to obtain methyl 1-(1-(difluoromethyl)cyclopropyl)-6-oxo-4-(toluenesulfonyloxy)-1,6-dihydropyridine-3-carboxylate (**B23-2**) (3.4 g, yield: 89%) as a light yellow solid.
LC-MS, M/Z (ESI): 414.5 $[M+H]^+$.

Step 3: Synthesis of methyl 4-acetamido-1-(1-(difluoromethyl)cyclopropyl)-6-oxo-1,6-dihydropyridine-3-carboxylate (**B23-3**)

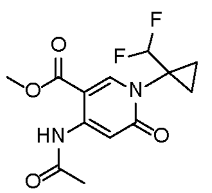

**B23-3**

To a 100 mL single-necked flask was successively added compound 5 (3.3 g, 7.98 mmol), acetamide (707.0 mg, 11.97 mmol), Xantphos (924.0 mg, 1.60 mmol), potassium phosphate (3.39 g, 15.97 mmol) and palladium ($\pi$-cinnamyl) chloride dimer (1.46 g, 1.60 mmol), which were dissolved in dioxane (30.0 mL). The reaction system was replaced with nitrogen three times, heated to 115°C, and stirred for 16 h. The reaction mixture was cooled, and then filtered. The resulting mother liquor was evaporated to dryness by rotary evaporation, then the sample was mixed, and the mixture was purified by silica gel column chromatography (petroleum ether: ethyl acetate (V/V) = 10:1 to 1:1) to obtain methyl 4-acetamido-1-(1 -(difluoromethyl)cyclopropyl)-6-oxo-1,6-dihydropyridine-3 -carboxylate (**B23-3**) (1.6 g, yield: 67%) as a white solid.

Step 4: 6-(1-(Difluoromethyl)cyclopropyl)-4-hydroxy-2-methylpyrido[4,3-d]pyrimidin-7(6H)-one (**B23-4**)

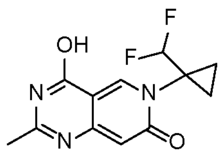

**B23-4**

To a 50.0 mL digestion vessel was added methyl 4-acetamido-1-(1-(difluoromethyl)cyclopropyl)-6-oxo-1,6-dihydropyridine-3-carboxylate (1.6 g, 5.33 mmol) which was dissolved in a solution of ammonia in methanol (7 N, 15.0 mL), and the reaction system was heated to 50°C and stirred for 12 h. The reaction mixture was cooled and then filtered, and the filter cake was washed with methanol (15.0 mL) to obtain 6-(1-(difluoromethyl)cyclopropyl)-4-hydroxy-2-methylpyrido[4,3-d]pyrimidin-7(6H)-one (**B23-4**) (900 mg, yield: 63%) as a light yellow solid.

LC-MS, M/Z (ESI): 268.2 $[M+H]^+$.

Step 5: (R)-6-(1-(difluoromethyl)cyclopropyl)-2-methyl-4-((1-(3-(pentafluorosulfanyl)phenyl)ethyl)amino)pyrido[4,3-d] pyrimidin-7(6H)-one (**I-23**)

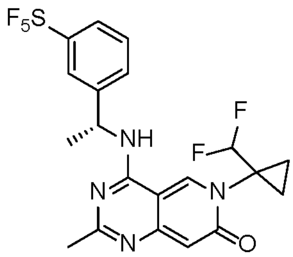

**I-23**

6-(1-(Difluoromethyl)cyclopropyl)-4-hydroxy-2-methylpyrido[4,3-d]pyrimidin-7(6H)-one (300.0 mg, 1.12 mmol) was dissolved in acetonitrile (15.0 mL), the reaction mixture was added with potassium phosphate (596.0 mg, 2.81 mmol) and then phosphonitrilic chloride trimer (585.0 mg, 1.68 mmol), stirred at room temperature for 2 h, then added with (R)-1-(3-(pentafluorosulfanyl)phenyl)ethan-1-amine hydrochloride (318.0 mg, 1.12 mmol), and reacted and stirred overnight at room temperature. The reaction mixture was first filtered, the filter cake was washed with acetonitrile (15.0 mL), and the filtrate was evaporated to dryness by rotary evaporation to obtain a crude product, which was purified by silica gel column chromatography (petroleum ether:ethyl acetate = 10:1 to 1:1) to obtain (R)-6-(1-(difluoromethyl)cyclopropyl)-2-methyl-4-((1-(3-(pentafluorosulfanyl)phenyl)ethyl)amino)pyrido[4,3-d]pyrimidin-7(6H)-one (**I-23**, 166.0 mg, yield: 30%) as a light yellow solid.

$^1$H NMR (400 MHz, DMSO-$d_6$) δ 9.11 (s, 1H), 8.91 (d, *J* = 7.1 Hz, 1H), 7.95 (s, 1H), 7.77 (dd, *J* = 8.2, 2.1 Hz, 1H), 7.71 (d, *J* = 7.7 Hz, 1H), 7.59 (t, *J* = 8.0 Hz, 1H), 6.31 (t, *J* = 57.1 Hz, 1H), 6.10 (s, 1H), 5.60 (t, *J* = 6.9 Hz, 1H), 2.18 (d, *J* = 26.4 Hz, 3H), 1.60 (d, *J* = 7.1 Hz, 3H), 1.48 (s, 2H), 1.37 (s, 2H).

LC-MS, M/Z (ESI): 497.2 $[M+H]^+$.

In the test examples of the present disclosure, control compound I was prepared with reference to patent WO2019122129A1, and control compound II was prepared with reference to patent WO2019122129A1. Their structures are as follows:

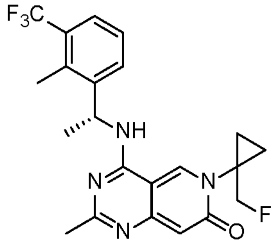

Control compound I

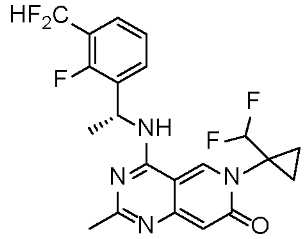

Control compound II

**Test example 1: Inhibition test of compounds on KRAS G12C::SOS1 binding**

The compound to be tested was prepared into a 10 mM stock solution with DMSO, and the compound was serially diluted using 1X test buffer. 0.1 μL of the compound solution with different concentrations was transferred to a 384-well plate. 5 μL of GST-KRAS G12C was added to the 384-well plate, and centrifuged at 1000 rpm for 1 min. 5 μL of His-SOS1 was added to the 384-well plate, centrifuged at 1000 rpm for 1 min, and incubated at room temperature for 15 min.

After incubation, 10 μL of a mixed solution of anti-6his-Tb monoclonal antibody (Cisbio, Cat. No. 61HI2TLA) and anti-GST-XL665 monoclonal antibody (Cisbio, Cat. No. 61GSTXLA) was added to the test well, centrifuged at 1000 rpm for 1 min, and incubated at room temperature for 1 h.

After incubation, the fluorescence signal ratios at 665 nm and 615 nm were read on a multifunctional microplate reader (Perkin Elmer, Envision 2104), and the $IC_{50}$ values were calculated using Graphpad 5 software.

Table 1: Inhibition results of test compounds on KRAS G12C::SOS1 binding

| Test compound | $IC_{50}$ (nM) |
|---|---|
| Control compound I | 8.58 |
| Control compound II | 8.15 |
| I-1 | 6.87 |
| I-2 | 7.73 |
| I-3 | 3.94 |
| I-5 | 10.0 |
| I-12 | 22.2 |
| I-15 | 14.4 |
| I-16 | 16.1 |
| I-19 | 9.5 |
| I-21 | 6.9 |
| I-22 | 21.7 |
| I-23 | 4.9 |

The experimental results indicate that the compounds of the present disclosure have a significant inhibitory effect on KRAS G12C:: SOS 1 binding.

**Test example 2: Inhibition test of compounds on KRAS G12C-SOS1 activity**

The inhibitory effect of compounds on KRAS G12C-SOS1 was detected using Transcreener®GDP-FI (BellBrook, Cat. No. 3014-1K).

The compound to be tested was prepared into a 10 mM stock solution with DMSO, and serially diluted using 1X test buffer (modified Tris buffer). The resulting compound solution was transferred to a 384-well plate, and the final content of DMSO was 0.25%, and an additional DMSO well without compound was set up as a high signal control.

1X test buffer (modified Tris buffer) was prepared. KRAS G12C (SignalChem, Cat. No. R06-32DH-BLTLK), SOS1 (Cytoskeleton, Inc., Cat. No. GE02-XL) and GTP solution (BellBrook, Cat. No. 3014-1K) were prepared respectively using the test buffer. 10 μL of KRAS G12C was transferred to the 384-well plate, and another 10 μL of the buffer was transferred to an empty well as a low signal control. 5 μL of SOS1 and 5 μL of GTP were transferred to the 384-well plate, respectively.

GDP detection reagent was prepared using the test buffer. 10 μL of the detection reagent solution was transferred to the 384-well plate, and incubated at room temperature for 2 h. After incubation, the plate was read on a multifunctional microplate reader (SpectraMax Paradigm) with an excitation wavelength of 580 nm and an emission wavelength of 620 nm. The fluorescence signal was read, and the inhibition rate was calculated as:

inhibition percentage = (high signal control - sample signal) / (high signal control - low signal control) × 100, and $IC_{50}$ values were calculated using Graphpad 5 software.

Table 2: Inhibition results of test compounds on KRAS G12C-SOS1 activity

| Test compound | $IC_{50}$ (nM) |
|---|---|
| Control compound I | 45 |
| I-1 | 59 |
| I-2 | 102 |

The experimental results indicate that the compounds of the present disclosure have a significant inhibitory effect on KRAS G12C-SOS1.

**Test example 3: Inhibition test of compounds on ERK phosphorylation level in DLD-1 cells**

Intracellular western blot quantitative analysis was used to detect the inhibitory level of compounds on ERK phosphorylation in DLD-1 cells.

DLD-1 cells (ATCC, CCL-221) were seeded in a T75 culture flask at $2.5 \times 10^6$ cells/flask, and cultured in a RPMI 1640 medium containing 10% FBS for 2 days. On day 3, the cells were seeded on a 384-well plate, and cultured overnight at 37°C, 5% $CO_2$. After overnight culture, the plate was added with serially diluted compounds (the final content of DMSO was 0.5%), the negative group was added with DMSO, and incubated in a 37°C, 5% $CO_2$ incubator.

The cells were fixed, washed once with PBS, treated to disrupt membranes, and blocked at room temperature for 1 h. The blocking solution was removed. The plate was added with primary antibody (CST, Cat. No. #4370S), and incubated overnight at 4°C. The plate was washed 3 times with PBST (PBS solution added with 0.05% Tween-20), and soaked for 2 min each time. The plate was added with secondary antibody (LI-COR, Cat. No. 926-32211), and incubated at room temperature in the dark. The plate was washed 3 times with PBST, and soaked for 2 min each time. The plate was centrifuged at 1000 rpm for 1 min, scanned on a two-color infrared laser imaging system (Odyssey® CLX) to read the signal.

Relative signal = 800-channel signal values/700-channel signal values.

Relative expression level of ERK phosphorylation = (test compound - control compound I)/(DMSO group - control compound I)

$IC_{50}$ values were calculated using Graphpad 5 software.

Table 3: Inhibition results of test compounds on ERK phosphorylation level in DLD-1 cells

| Test compound | $IC_{50}$ (nM) |
|---|---|
| Control compound I | 72 |
| I-1 | 102 |
| I-2 | 58 |
| I-3 | 56 |
| I-12 | 196 |
| I-13 | 109 |
| I-17 | 119 |
| I-18 | 115 |
| I-19 | 131 |
| I-20 | 92 |
| I-21 | 141 |
| I-23 | 159 |

The experimental results indicate that the compounds of the present disclosure have a significant inhibitory effect on the ERK phosphorylation level in DLD-1 cells.

**Test example 4: Inhibition test of compounds on 3D cell proliferation**

H358 cells were seeded in a T75 culture flask, and cultured in a RPMI 1640 medium containing 10% FBS for 2 days for subsequent experiments in which the cells would be cultured or seeded on a 384-well plate.

On day 1, the cells were seeded on the 384-well plate, with 40 μL of medium added per well and serially diluted compounds or DMSO added per well, and an additional well without seeded cells but with medium was set up as a blank control. After culture at 37°C, 5% $CO_2$ for 7 days, the plate was added with 3D CellTiter-Glo reagent (Promega, Cat. No. G9683) on day 8, shaken at 320 rpm for 20 min, and left at room temperature for 2 h. Luminescence signals were read on a multifunctional microplate reader. Cell viability inhibition rate was calculated as:

$$\text{Cell viability inhibition rate} = (\text{DMSO group - test compound})/(\text{DMSO group - blank control group}) \times 100\%$$

$IC_{50}$ values were calculated using Graphpad 5 software.

Table 4: Inhibition results of test compounds on 3D proliferation of H358 cells

| Test compound | $IC_{50}$ (nM) |
|---|---|
| Control compound I | 13 |
| I-1 | 26 |
| I-2 | 13.9 |
| I-3 | 11.5 |
| I-13 | 15.7 |
| I-17 | 12.1 |
| I-18 | 9.2 |
| I-19 | 137.3 |
| I-20 | 31.0 |
| I-21 | 85.1 |
| I-22 | 10.7 |
| I-23 | 12.0 |

The experimental results indicate that the compounds of the present disclosure have a strong inhibitory effect on the 3D proliferation of H358 cells.

**Test example 5: Human liver microsome stability test**

The human liver microsome stability test was performed by incubating the compound and human liver microsomes *in vitro*. First, the compound to be tested was prepared into a 10 mM stock solution in DMSO solvent, and then the compound was diluted to 0.5 mM with acetonitrile. Human liver microsomes (Corning) were diluted with PBS into a microsome/buffer solution, and the solution was used to dilute 0.5 mM compound into a working solution, in which the concentration of the compound was 1.5 μM, and the concentration of human liver microsomes was 0.75 mg/mL. A deep-well plate was taken, 30 μL of the working solution was added per well, and then 15 μL of pre-heated 6 mM NADPH solution was added thereto to initiate a reaction, and the reaction was incubated at 37°C. At 0, 5, 15, 30 and 45 min of the incubation, 135 μL of acetonitrile was added to the corresponding wells to terminate the reaction. After the reaction was terminated with acetonitrile at the last time point of 45 min, the deep-well plate was vortexed and vibrated for 10 min (600 rpm/min), and then centrifuged for 15 min. After centrifugation, the supernatant was collected, and added with purified water in a ratio of 1:1 to perform LC-MS/MS detection. A ratio of a peak area of compound to a peak area of

internal standard at each time point was obtained, and the peak area ratios of the compound at 5, 15, 30 and 45 min were compared with the peak area ratio at 0 min to calculate the remaining percentage of the compound at each time point. $T_{1/2}$ was calculated by using GraphPad 5 software.

Table 5: Results of human liver microsome stability test

| Compound | Remaining percentage (%) of compound after incubation for 30 min | $T_{1/2}$ (min) |
|---|---|---|
| Control compound I | 70.2 | 64.2 |
| I-1 | 76.4 | 87.5 |
| I-2 | 96.5 | 475 |
| I-3 | 98.7 | 765 |

The experimental results indicate that, compared with the control compound I, the compounds of the present disclosure exhibit better hepatic metabolic stability, slower metabolism in the human body, and higher exposure.

**Test example 6: Inhibition test of compounds on cytochrome P450**

The inhibitory potential of compounds on cytochrome P450 (CYP450) subtype CYP3A4 (two substrates of midazolam and testosterone) was detected. First, the compound to be tested was prepared into a 10 mM stock solution in DMSO solvent, and the CYP3A4 inhibitor ketoconazole was prepared as 10 mM, 2.5 mM and 2.5 mM stock solutions in DMSO solvent. The test compound and ketoconazole were diluted to 400-fold final concentration (compound: 10 μM, ketoconazole: 2.5 μM) with acetonitrile.

NADPH cofactor (10 mL of potassium phosphate buffer added with 66.7 mg NADPH) at 4-fold final concentration and substrates were prepared with potassium phosphate buffer (0.1 M, pH = 7.4), with CYP3A4 substrate midazolam at a final concentration of 320 μM and CYP3A4 substrate testosterone at a final concentration of 20 μM.

A human liver microsome solution at a concentration of 0.2 mg/mL was prepared with potassium phosphate buffer on ice. A solution of the compound to be tested and a control inhibitor (control compound) solution at 2-fold final concentration were prepared with the human liver microsome solution on ice. The test wells were added with 30 mL of the test compound solution and the control inhibitor solution respectively, and then added with 15 mL of the substrate for duplication. A 96-well assay plate and a NADPH solution were incubated at 37 °C for 5 min, and 15 μL of pre-heated 8 mM NADPH solution was added to the assay plate to initiate the reaction. A CYP3A4 assay plate was pre-incubated at 37°C for 5 min. The plate was added with 120 μL of acetonitrile to terminate the reaction, and after quenching, the plate was shaken on a shaker (IKA, MTS 2/4) for 10 min (600 rpm/min), and then centrifuged for 15 min. After centrifugation, the supernatant was collected, and added with purified water in a ratio of 1:1 to perform LC-MS/MS detection. A ratio of a peak area of compound to a peak area of internal standard was obtained, and the peak area ratio of the compound was compared with the peak area ratio of the control inhibitor to calculate the inhibition rate.

Table 6: Inhibition results of test compounds on CYP450 enzyme

| Compound (10 μM) | CYP inhibition rate (%) | |
|---|---|---|
| | CYP3A4 (midazolam) | CYP3A4 (testosterone) |
| Control compound I | 33.52 | 8.5 |
| I-1 | 17.8 | 0 |
| I-2 | 10.8 | 0 |
| I-3 | 0 | 0 |

The experimental results indicate that, compared with the control compound I, the compounds of the present disclosure have a weak or no inhibitory effect on CYP3A4 enzymes at 10 μM, and have a low risk of potential drug-drug interactions. Herein, compound I-3 has a more significant inhibitory effect on CYP3A4 enzymes.

**Test example 7: Plasma protein binding rate of compounds**

Plasma protein binding rate of compounds was detected by equilibrium dialysis (HTDialysis, HTD 96b). The compound was prepared into a 0.5 nM stock solution with DMSO, and then 25-fold diluted with 0.05 M sodium phosphate

buffer as a working solution. A blank 96-well plate was taken and preloaded with 380 μL of plasma per well. The plasma was then added with the working solution at 20 μL/well and mixed well, with the compound at a final concentration of 1 μM, containing 0.2% DMSO per well.

100 μL of 0.05 M sodium phosphate buffer was added to the reception-side of each dialysis chamber (HTD 96b), and then 100 μL of plasma containing the compound was added to the supply-side. The dialysis chamber was covered with a plastic lid, shaken and incubated at 37°C for 5 h.

After incubation, 25 μL of samples was taken from each of the supply-side and the reception-side of the dialysis chamber, and placed in the blank 96-well plate. An equal volume of plasma was added to each of the supply-side samples while an equal volume of 0.05 M sodium phosphate buffer was added to each of the reception-side samples, and mixed well. The 96-well plate was added with an acetonitrile solution containing internal standard at 200 μL per well, vortexed and shaken at 600 rpm for 10 min, and centrifuged at 5594 g for 15 min (Thermo Multifuge × 3R). 50 μL of the supernatant was then transferred to a new 96-well plate, and the samples were mixed with 50 μL of ultra-pure water for LC-MS/MS analysis.

Plasma protein binding rate and free fraction were calculated using the following formulas: % binding rate = 100 × ([supply-side concentration]$_{5h}$ - [reception-side concentration]$_{5h}$) / [supply-side concentration]$_{5h}$. % free fraction = 100 - % binding rate

Table 7: Free fraction of test compounds in plasma

| Compound | Human (%) | Mouse (%) |
|---|---|---|
| Control compound I | 0.9 | 0.5 |
| I-1 | 4.2 | 5.4 |
| I-2 | 1.5 | 2.8 |
| I-3 | 1.7 | 1.1 |
| I-5 | 7.7 | 11.4 |

The experimental results indicate that the compounds of the present disclosure have a higher ratio of free drug in both human and mouse plasma relative to the control drug, and have good druggability.

**Test example 8: Pharmacokinetic test in mice**

Male ICR mice (20 to 25 g) were used, and fasted overnight. Three mice were taken and orally administered by gavage (10 mg/kg). Blood was collected before the administration, and at 15 min, 30 min, 1 h, 2 h, 4 h, 8 h and 24 h after the administration. 6,800 g of the blood samples were centrifuged at 2 to 8°C for 6 min, and plasma was collected and stored at -80°C. The plasma at each time point was taken and added with an acetonitrile solution containing internal standard in 3 to 5 times the amount, vortexed and mixed for 1 min, and centrifuged at 13,000 rpm/min at 4°C for 10 min. The supernatant was collected, added with water in 3 times the amount, and mixed. An appropriate amount of the mixture was taken for LC-MS/MS analysis. The main pharmacokinetic parameters were analyzed using WinNonlin 7.0 software with non-compartmental model.

Table 8: Results of pharmacokinetic test in mice of the test compounds

| Compound | Pharmacokinetic parameters of mice (oral administration by gavage) | | | |
|---|---|---|---|---|
| | Cmax (ng/mL) | Tmax (hr) | $AUC_{0-t}$ (h * ng/mL) | $T_{1/2}$ (hr) |
| Control compound I | 176 | 0.25 | 365 | 1.35 |
| Control compound II | 669 | 0.50 | 1002 | 0.96 |
| I-2 | 256 | 0.50 | 517 | 0.99 |
| I-3 | 1442 | 1.00 | 3080 | 0.97 |
| I-23 | 4244 | 2.00 | 44126 | 2.33 |

The experimental results of pharmacokinetic test in mice indicate that the compounds of the present disclosure have high oral exposure, good pharmacokinetic properties and good druggability.

**Test example 9: Mia Paca-2 pancreatic cancer *in vivo* efficacy test**

After one week of adaptive feeding of mice, Mia Paca-2 cells in a log phase were resuspended in serum-free DMEM, and then mixed with Matrigel in a ratio of 1:1. $1 \times 10^7$ Mia Paca-2 cells were inoculated subcutaneously at the right flank at 100 μL per mouse, and tumor growth was observed regularly. When the tumor grew to an average volume of 150 to 200 $mm^3$, the mice were randomly divided into a model group and an administration group (single drug, in combination with trametinib) based on the tumor size and body weight. The tumor size and body weight were measured and recorded before and during the administration. After the treatment, the tumor size in the model group was compared with that in the administration group to determine the efficacy.

Table 9: Tumor inhibitory ability of the test compounds at the level of tumor weight

| Drug | Dose (mg/kg) | Average tumor weight at the end of the treatment (g) | Tumor weight inhibition rate (relative to the vehicle group) |
|---|---|---|---|
| Vehicle | -- | 1.14 | -- |
| Control compound II | 50, BID | 0.46 | 59% |
| I-3 | 25, BID | 0.35 | 70% |
| I-3 | 50, BID | 0.27 | 76% |
| I-23 | 25, BID | 0.45 | 61% |
| I-23 | 50, BID | 0.38 | 67% |
| I-3 + Trametinib | I-3: 25, BID Trametinib: 0.125, BID | 0.13 | 89% |
| I-23 + Trametinib | I-23: 25, BID Trametinib: 0.125, BID | 0.16 | 86% |
| Trametinib | 0.125, BID | 0.22 | 80% |
| "--" in the table means no testing. | | | |

The experimental results indicate (see FIG. 1 for details) that the compounds of the present disclosure, alone or in combination with trametinib, have a significant inhibitory effect on Mia Paca-2 tumor growth, and the combined use is more effective than the single use.

**Test example 10: LOVO colorectal cancer *in vivo* efficacy test**

After one week of adaptive feeding of mice, LOVO cells in a log phase were resuspended in serum-free F12K, $5 \times 10^6$ LOVO cells were inoculated subcutaneously at the right flank at 100 μL per mouse, and tumor growth was observed regularly. When the tumor grew to an average volume of 150 to 200 $mm^3$, the mice were randomly divided into a model group and an administration group based on the tumor size and body weight. The tumor size and body weight were measured and recorded before and during the administration. After the treatment, the tumor size in the model group was compared with that in the administration group to determine the efficacy.

Table 10: Tumor inhibitory ability of the test compounds at the level of tumor weight

| Drug | Dose (mg/kg) | Average tumor weight at the end of the treatment (g) | Tumor weight inhibition rate (relative to the vehicle group) |
|---|---|---|---|
| Vehicle | -- | 1.39 | -- |
| Control compound II | 50, BID | 1.08 | 23% |
| I-3 | 50, BID | 0.75 | 46% |

(continued)

| Drug | Dose (mg/kg) | Average tumor weight at the end of the treatment (g) | Tumor weight inhibition rate (relative to the vehicle group) |
|---|---|---|---|
| I-23 | 25, BID | 0.65 | 53% |
| "--" in the table means no testing. | | | |

The experimental results indicate (see FIG. 2 for details) that the compounds of the present disclosure have a significant inhibitory effect on LOVO tumor tissue growth, which is better than that of the control compound II.

Although the embodiments of the present disclosure are illustrated and described above, it can be understood that the above-mentioned embodiments are illustrative and should not be construed as limiting the present disclosure. Those skilled in the art can make changes, modifications, substitutions and variations based on the above-mentioned embodiments within the scope of the present disclosure.

## Claims

**1.** A pyridopyrimidinone derivative as represented by formula I, a tautomer thereof, a stereoisomer thereof, a hydrate thereof, a solvate thereof, a pharmaceutically acceptable salt thereof or a prodrug thereof:

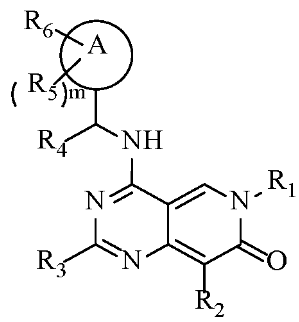

I;

wherein ring A is a 6- to 10-membered aromatic ring or a 9- to 11-membered heteroaromatic ring;
$R_1$ is 3- to 10-membered cycloalkyl or 4- to 10-membered heterocycloalkyl, the $R_1$ is optionally substituted by one or more than one $R_{11}$, the $R_{11}$ is a substituent selected from: halogen, hydroxyl, $C_1$-$C_6$ alkyl, $C_1$-$C_6$ haloalkyl, $C_1$-$C_6$ alkoxy,

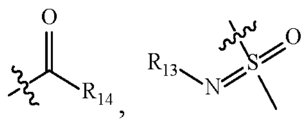

or

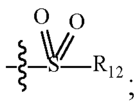

when there is more than one substituent $R_{11}$, the substituents $R_{11}$ are the same or different;
the $R_{11}$ is optionally substituted by a substituent selected from: $C_1$-$C_6$ alkyl, $C_1$-$C_6$ alkoxy, halogen, hydroxyl;
$R_{12}$ is $C_1$-$C_6$ alkyl, $C_1$-$C_6$ alkyl substituted by one or more than one F, or 3-to 6-membered cycloalkyl;
$R_{13}$ is hydrogen, $C_1$-$C_6$ alkyl or cyano;
$R_{14}$ is hydrogen, $C_1$-$C_6$ alkyl, $C_1$-$C_6$ haloalkyl;
$R_2$ is hydrogen or a substituent selected from: halogen, $C_1$-$C_6$ alkyl, 3- to 6-membered cycloalkyl, $C_1$-$C_6$ alkoxy;

the $C_1$-$C_6$ alkyl, 3- to 6-membered cycloalkyl, $C_1$-$C_6$ alkoxy are each independently substituted by one or more than one $R_{21}$; the $R_{21}$ is a substituent selected from: hydroxyl, halogen, $C_1$-$C_3$ alkoxy; when there is more than one substituent, the $R_{21}$ are the same or different;
$R_3$ is hydrogen or a substituent selected from: halogen, $C_1$-$C_6$ alkyl, $C_1$-$C_6$ haloalkyl;
$R_4$ is $C_1$-$C_6$ alkyl or $C_1$-$C_6$ haloalkyl;
$R_5$ is hydrogen or a substituent selected from: halogen, $C_1$-$C_6$ alkyl, $C_1$-$C_6$ haloalkyl;
$R_6$ is $-SF_5$,

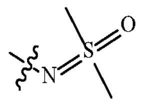 , 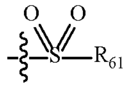 or 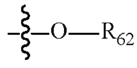 ;

the $R_{61}$ and the $R_{62}$ are each independently $C_1$-$C_6$ alkyl substituted by halogen, or 3- to 6-membered cycloalkyl; or, the ring A together with $R_6$ and $R_5$ forms a group moiety

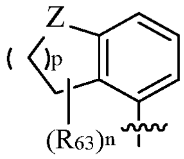 ;

wherein Z is

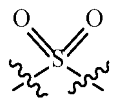 ;

$R_{63}$ is hydrogen or a substituent selected from: halogen, hydroxyl, $C_1$-$C_6$ alkyl, $C_1$-$C_6$ alkyl substituted by halogen; when there is more than one substituent $R_{63}$, the $R_{63}$ are the same or different;
m is 1 or 2; p is 1, 2, or 3; and n is 1, 2, or 3.

**2.** The pyridopyrimidinone derivative as represented by formula I, the tautomer thereof, the stereoisomer thereof, the hydrate thereof, the solvate thereof, the pharmaceutically acceptable salt thereof or the prodrug thereof according to claim 1, wherein,

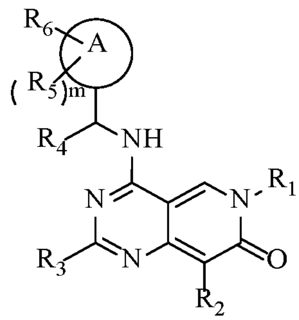

I;

wherein ring A is a 6- to 10-membered aromatic ring or a 9- to 11-membered heteroaromatic ring;
$R_1$ is 3- to 10-membered cycloalkyl or 4- to 10-membered heterocycloalkyl, the cycloalkyl is optionally substituted by one or more than one $R_{11}$, the $R_{11}$ is a substituent selected from: halogen, $C_1$-$C_6$ alkyl, $C_1$-$C_6$ haloalkyl, $C_1$-$C_6$ alkoxy,

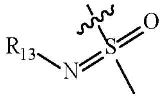
or
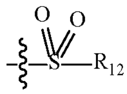
;

when there is more than one substituent $R_{11}$, the substituents $R_{11}$ are the same or different;
$R_{12}$ is $C_1$-$C_6$ alkyl, $C_1$-$C_6$ alkyl substituted by one or more than one F, or 3- to 6-membered cycloalkyl; $R_{13}$ is hydrogen, $C_1$-$C_6$ alkyl or cyano;
$R_2$ is hydrogen or a substituent selected from: halogen, $C_1$-$C_6$ alkyl, 3- to 6-membered cycloalkyl, $C_1$-$C_6$ alkoxy; the $C_1$-$C_6$ alkyl, 3- to 6-membered cycloalkyl, $C_1$-$C_6$ alkoxy are each independently substituted by one or more than one $R_{21}$; the $R_{21}$ is a substituent selected from: hydroxyl, halogen, $C_1$-$C_3$ alkoxy; when there is more than one substituent, the $R_{21}$ are the same or different;
$R_3$ is hydrogen or a substituent selected from: halogen, $C_1$-$C_6$ alkyl, $C_1$-$C_6$ haloalkyl;
$R_4$ is $C_1$-$C_6$ alkyl or $C_1$-$C_6$ haloalkyl;
$R_5$ is hydrogen or a substituent selected from: halogen, $C_1$-$C_6$ alkyl, $C_1$-$C_6$ haloalkyl;
$R_6$ is $-SF_5$,

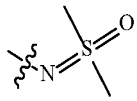
,
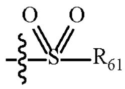
or
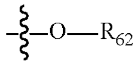
;

the $R_{61}$ and the $R_{62}$ are each independently $C_1$-$C_6$ alkyl substituted by halogen, or 3- to 6-membered cycloalkyl; or, the ring A together with $R_6$ and $R_5$ forms a group moiety

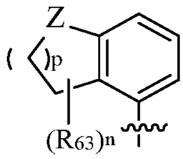
;

wherein Z is

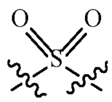
;

$R_{63}$ is hydrogen or a substituent selected from: halogen, hydroxyl, $C_1$-$C_6$ alkyl, $C_1$-$C_6$ alkyl substituted by halogen; when there is more than one substituent $R_{63}$, the $R_{63}$ are the same or different;
m is 1 or 2; p is 1, 2, or 3; and n is 1, 2, or 3.

**3.** The pyridopyrimidinone derivative as represented by formula I, the tautomer thereof, the stereoisomer thereof, the hydrate thereof, the solvate thereof, the pharmaceutically acceptable salt thereof or the prodrug thereof according to claim 1 or 2, wherein the pyridopyrimidinone derivative as represented by formula I has a structure of I-1,

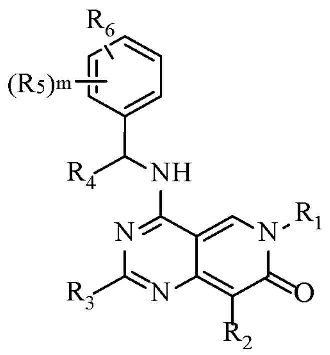

I-1

;

preferably, the pyridopyrimidinone derivative as represented by formula I has a structure of I-2,

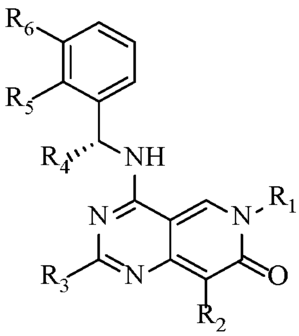

I-2

;

wherein $R_1$, $R_2$, $R_3$, $R_5$, $R_6$ and m are as defined in claim 1; $R_4$ is methyl or $-CH_2F$; preferably, $R_4$ is methyl.

**4.** The pyridopyrimidinone derivative as represented by formula I, the tautomer thereof, the stereoisomer thereof, the hydrate thereof, the solvate thereof, the pharmaceutically acceptable salt thereof or the prodrug thereof according to claim 1 or 2, wherein $R_5$ is hydrogen, fluorine or methyl; m is 1 or 2; preferably, m is 1;

$R_6$ is $-SF_5$,

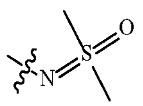 , 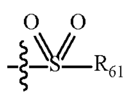 or 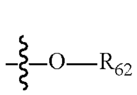 ;

the $R_{61}$ and the $R_{62}$ are each independently $C_1$-$C_6$ alkyl substituted by one or more than one F, or 3- to 6-membered cycloalkyl;
preferably, $R_{61}$ is $-CH_2F$, $-CHF_2$, $-CF_3$, $-CF_2CH_3$ or cyclopropyl;
preferably, $R_{62}$ is $-CH_2F$, $-CHF_2$, $-CF_3$; more preferably, $R_{62}$ is $-CHF_2$;
for example,

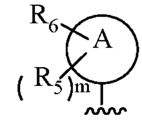 is 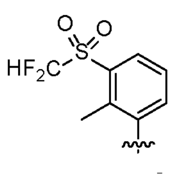 , 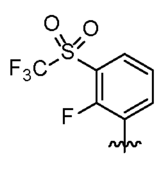 , 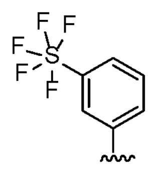 , 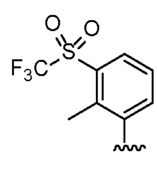 ,

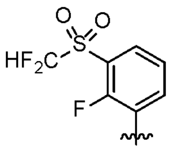, 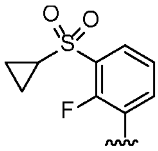, 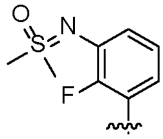, 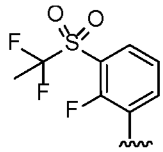, 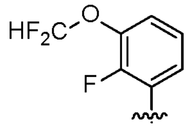.

**5.** The pyridopyrimidinone derivative as represented by formula I, the tautomer thereof, the stereoisomer thereof, the hydrate thereof, the solvate thereof, the pharmaceutically acceptable salt thereof or the prodrug thereof according to claim 1 or 2, wherein the ring A together with $R_6$ and $R_5$ forms a group moiety

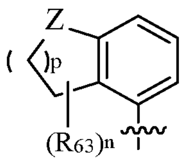;

wherein Z is

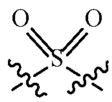;

p is 1, n is 2 or 3, $R_{63}$ is fluorine or hydroxyl, and when there is more than one $R_{63}$, the $R_{63}$ are the same or different; preferably, the group moiety

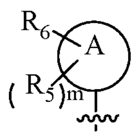

has a structure of

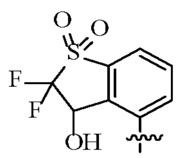;

more preferably,

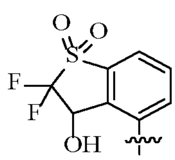 is 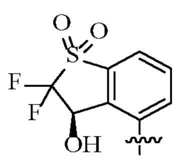.

**6.** The pyridopyrimidinone derivative as represented by formula I, the tautomer thereof, the stereoisomer thereof, the hydrate thereof, the solvate thereof, the pharmaceutically acceptable salt thereof or the prodrug thereof according to claim 3, wherein benzene ring together with $R_6$ and $R_5$ forms a group moiety

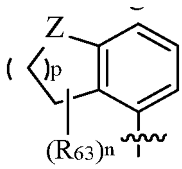
;

wherein Z is

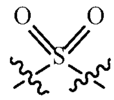
;

p is 1, n is 2 or 3, $R_{63}$ is fluorine or hydroxyl, and when there is more than one $R_{63}$, the $R_{63}$ are the same or different; preferably, the group moiety

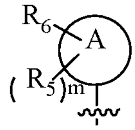

has a structure of

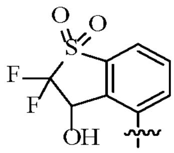
;

more preferably,

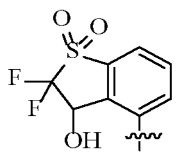
is
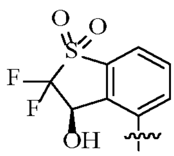
.

7. The pyridopyrimidinone derivative as represented by formula I, the tautomer thereof, the stereoisomer thereof, the hydrate thereof, the solvate thereof, the pharmaceutically acceptable salt thereof or the prodrug thereof according to claim 1 or 2, wherein $R_5$ is hydrogen; $R_6$ is $-SF_5$,

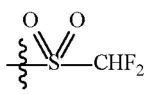
or
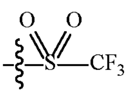
;

preferably, $R_6$ is $-SF_5$.

8. The pyridopyrimidinone derivative as represented by formula I, the tautomer thereof, the stereoisomer thereof, the hydrate thereof, the solvate thereof, the pharmaceutically acceptable salt thereof or the prodrug thereof according to claim 1 or 2, wherein $R_4$ is $C_1$-$C_3$ alkyl or $C_1$-$C_3$ haloalkyl; preferably, $R_4$ is methyl or $-CH_2F$; more preferably, $R_4$ is methyl.

9. The pyridopyrimidinone derivative as represented by formula I, the tautomer thereof, the stereoisomer thereof, the

hydrate thereof, the solvate thereof, the pharmaceutically acceptable salt thereof or the prodrug thereof according to claim 1 or 2, wherein the halogen is fluorine, chlorine, bromine; preferably, the halogen is fluorine.

**10.** The pyridopyrimidinone derivative as represented by formula I, the tautomer thereof, the stereoisomer thereof, the hydrate thereof, the solvate thereof, the pharmaceutically acceptable salt thereof or the prodrug thereof according to claim 1 or 2, wherein in the $R_1$, the 3- to 10-membered cycloalkyl comprises a monocyclic, bicyclic, tricyclic, spiro or bridged ring; the 4- to 10-membered heterocycloalkyl has one or more than one heteroatom, and the heteroatom is N, O or S;

preferably, the 3- to 10-membered cycloalkyl is: cyclopropyl, cyclobutyl, cyclopentyl, cyclohexyl, cycloheptyl, bicyclo[1.1.1]pentyl, bicyclo[2.2.0]hexyl, bicyclo[3.2.0]heptyl, bicyclo[3.2.1]octyl, bicyclo[2.2.2]octyl, bicyclo[4.3.0]nonyl (octahydroindenyl), bicyclo[4.4.0]decyl (decahydronaphthalene), bicyclo[2.2.1]heptyl (norbornyl), bicyclo[4.1.0]heptyl (norcaranyl), bicyclo[3.1.1]heptyl (pinanyl), spiro[2.5]octyl, spiro[3.3]heptyl; more preferably, the 3- to 10-membered cycloalkyl is:

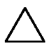 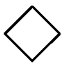 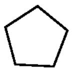 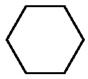 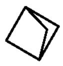 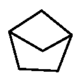 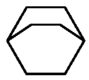, 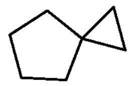;

preferably, the 4- to 10-membered heterocycloalkyl is: tetrahydrofuranyl, pyrrolidinyl, pyrrolinyl, imidazolidinyl, thiazolidinyl, imidazolinyl, pyrazolidinyl, pyrazolinyl, piperidinyl, piperazinyl, oxiranyl, aziridinyl, azetidinyl, 1,4-dioxanyl, azepanyl, diazepanyl, morpholinyl, thiomorpholinyl, homomorpholinyl, homopiperidinyl, homopiperazinyl, homothiomorpholinyl, thiomorpholinyl-S-oxide, thiomorpholinyl-S,S-dioxide, 1,3-dioxolanyl, tetrahydropyranyl, tetrahydrothiopyranyl, [1.4]-oxazepanyl, tetrahydrothienyl, homothiomorpholinyl-S, S-dioxide, oxazolidonyl, dihydropyrazolyl, dihydropyrrolyl, dihydropyrazinyl, dihydropyridinyl, dihydropyrimidinyl, dihydrofuranyl, dihydropyranyl, tetrahydrothienyl-S-oxide, tetrahydrothienyl-S,S-dioxide, homothiomorpholinyl-S-oxide, 2,3-dihydroazetidinyl, 2H-pyrrolyl, 4H-pyranyl, 1,4-dihydropyridinyl, 8-aza-bicyclo[3.2.1]octyl, 8-aza-bicyclo[5.1.0]octyl, 2-oxa-5-azabicyclo[2.2.1]heptyl, 8-oxa-3-aza-bicyclo[3.2.1]octyl, 3,8-diaza-bicyclo[3.2.1]octyl, 2,5-diaza-bicyclo-[2.2.1]heptyl, 1-aza-bicyclo[2.2.2]octyl, 3,8-diaza-bicyclo[3.2.1]octyl, 3,9-diaza-bicyclo[4.2.1]nonyl, 2,6-diaza-bicyclo[3.2.2]nonyl;
more preferably, the 4- to 10-membered heterocycloalkyl is:

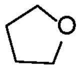, 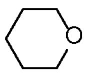, 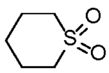,

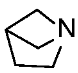, 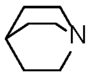, 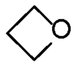, 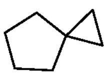, 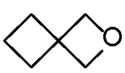, 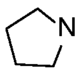, 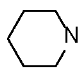;

or, the 4- to 10-membered heterocycloalkyl is:

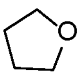 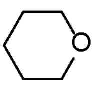 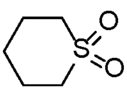 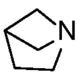 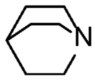 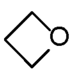 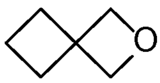.

**11.** The pyridopyrimidinone derivative as represented by formula I, the tautomer thereof, the stereoisomer thereof, the hydrate thereof, the solvate thereof, the pharmaceutically acceptable salt thereof or the prodrug thereof according to claim 1 or 2, wherein $R_1$ is 3- to 6-membered cycloalkyl or 4- to 6-membered heterocycloalkyl, the cycloalkyl is optionally substituted by one or more than one $R_{11}$, and the $R_{11}$ is a substituent selected from: halogen, $C_1$-$C_6$ alkyl, $C_1$-$C_6$ haloalkyl; when there is more than one substituent $R_{11}$, the substituents $R_{11}$ are the same or different; more preferably, $R_1$ is

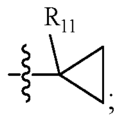

wherein the $R_{11}$ is a substituent selected from: halogen, $C_1$-$C_6$ alkyl, $C_1$-$C_6$ haloalkyl; preferably, $R_{11}$ is $C_1$-$C_6$ alkyl or $C_1$-$C_6$ alkyl substituted by fluorine; more preferably, $R_{11}$ is methyl, $-CH_2F$ or $-CHF_2$.

**12.** The pyridopyrimidinone derivative as represented by formula I, the tautomer thereof, the stereoisomer thereof, the hydrate thereof, the solvate thereof, the pharmaceutically acceptable salt thereof or the prodrug thereof according to claim 1 or 2, wherein $R_1$ is 3- to 10-membered cycloalkyl or 4- to 10-membered heterocycloalkyl, the heterocycloalkyl is optionally substituted by one or more than one $R_{11}$, and the $R_{11}$ is hydroxyl or

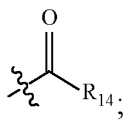

when there is more than one substituent $R_{11}$, the substituents $R_{11}$ are the same or different;

the $R_{11}$ is optionally substituted by a substituent selected from: $C_1$-$C_6$ alkyl, $C_1$-$C_6$ alkoxy, halogen, hydroxyl; preferably, the $R_{11}$ is optionally substituted by a substituent selected from: $C_1$-$C_3$ alkyl, $C_1$-$C_3$ alkoxy, halogen, hydroxyl;
$R_{14}$ is hydrogen, $C_1$-$C_6$ alkyl, $C_1$-$C_6$ haloalkyl; preferably, $R_{14}$ is hydrogen, $C_1$-$C_3$ alkyl, $C_1$-$C_3$ haloalkyl.

**13.** The pyridopyrimidinone derivative as represented by formula I, the tautomer thereof, the stereoisomer thereof, the hydrate thereof, the solvate thereof, the pharmaceutically acceptable salt thereof or the prodrug thereof according to claim 1 or 2, wherein $R_1$ is 3- to 6-membered cycloalkyl or 4- to 6-membered heterocycloalkyl, the $R_1$ is optionally substituted by one or more than one $R_{11}$, and the $R_{11}$ is a substituent selected from: halogen, hydroxyl, $C_1$-$C_6$ alkyl, $C_1$-$C_6$ alkoxy,

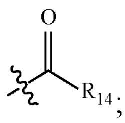

$R_{14}$ is hydrogen, $C_1$-$C_6$ alkyl, $C_1$-$C_6$ haloalkyl; preferably, $R_{14}$ is hydrogen, $C_1$-$C_3$ alkyl, $C_1$-$C_3$ haloalkyl;
the $R_{11}$ is optionally substituted by a substituent selected from: $C_1$-$C_3$ alkoxy, halogen; preferably, the halogen is fluorine.

**14.** The pyridopyrimidinone derivative as represented by formula I, the tautomer thereof, the stereoisomer thereof, the hydrate thereof, the solvate thereof, the pharmaceutically acceptable salt thereof or the prodrug thereof according to claim 1 or 2, wherein $R_1$ is selected from cyclopropyl, cyclobutyl, tetrahydrofuranyl, tetrahydropyranyl, epoxypropanyl, pyrrolidinyl or piperidinyl; the $R_1$ is optionally substituted by one or more than one Rn, and the $R_{11}$ is a substituent selected from: fluorine, hydroxyl, $C_1$-$C_6$ alkyl, $C_1$-$C_6$ alkyl substituted by $C_1$-$C_3$ alkoxy, $C_1$-$C_6$ alkoxy,

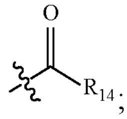

$R_{14}$ is $C_1$-$C_6$ alkyl;
preferably, $R_1$ is selected from:

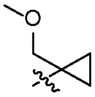, 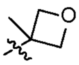, 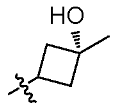, 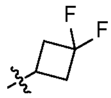, 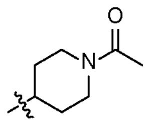,

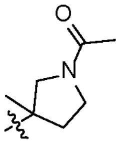, 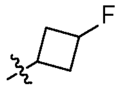, 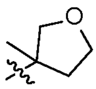, 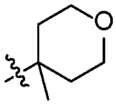, 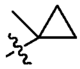, 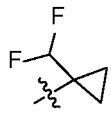, 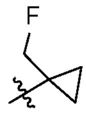, 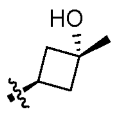, 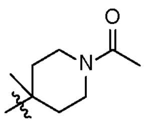

and

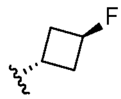.

**15.** The pyridopyrimidinone derivative as represented by formula I, the tautomer thereof, the stereoisomer thereof, the hydrate thereof, the solvate thereof, the pharmaceutically acceptable salt thereof or the prodrug thereof according to claim 1 or 2, wherein $R_5$ is hydrogen; $R_6$ is $-SF_5$; $R_4$ is methyl; $R_3$ is methyl; and $R_2$ is hydrogen.

**16.** The pyridopyrimidinone derivative as represented by formula I, the tautomer thereof, the stereoisomer thereof, the hydrate thereof, the solvate thereof, the pharmaceutically acceptable salt thereof or the prodrug thereof according to claim 1 or 2, wherein $R_2$ is hydrogen or a substituent selected from: halogen, $C_1$-$C_6$ alkyl, 3- to 6-membered cycloalkyl; preferably, $R_2$ is hydrogen or halogen; more preferably, $R_2$ is hydrogen.

**17.** The pyridopyrimidinone derivative as represented by formula I, the tautomer thereof, the stereoisomer thereof, the hydrate thereof, the solvate thereof, the pharmaceutically acceptable salt thereof or the prodrug thereof according to claim 1 or 2, wherein $R_3$ is hydrogen, $C_1$-$C_6$ alkyl or $C_1$-$C_6$ haloalkyl; preferably, $R_3$ is $C_1$-$C_6$ alkyl; more preferably, $R_3$ is methyl.

**18.** The pyridopyrimidinone derivative as represented by formula I, the tautomer thereof, the stereoisomer thereof, the hydrate thereof, the solvate thereof, the pharmaceutically acceptable salt thereof or the prodrug thereof according to claim 1, wherein the pyridopyrimidinone derivative comprises:

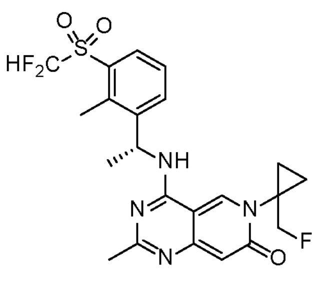

I-1

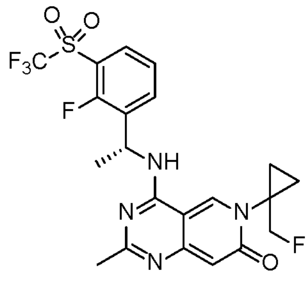

I-2

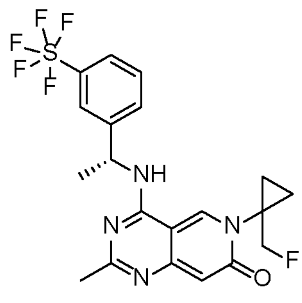

I-3

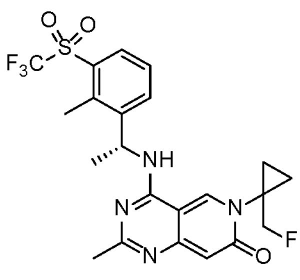
I-4

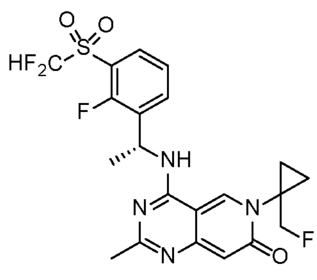
I-5

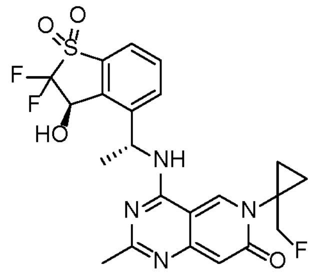
I-6

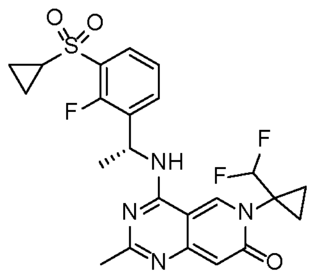
I-7

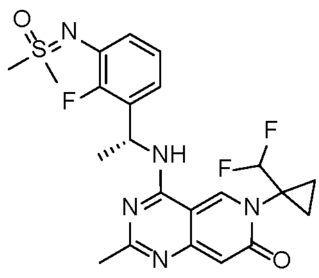
I-8

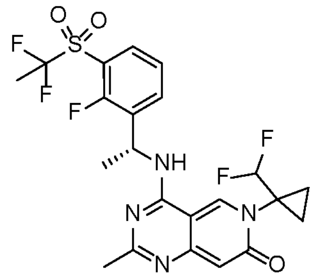
I-9

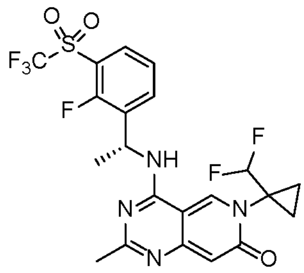
I-10

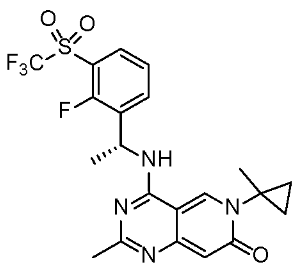
I-11

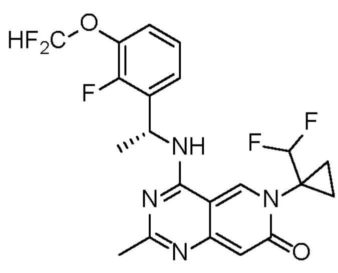
I-12

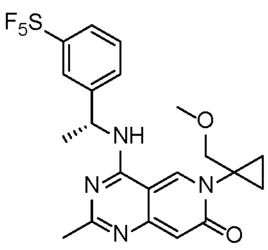
I-13

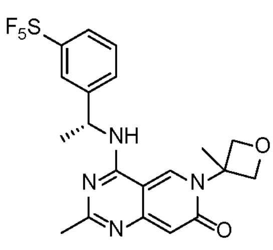
I-14

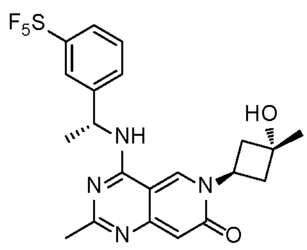
I-15

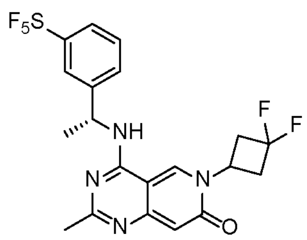
I-16

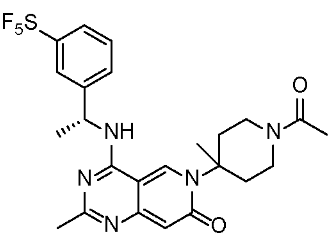
I-17

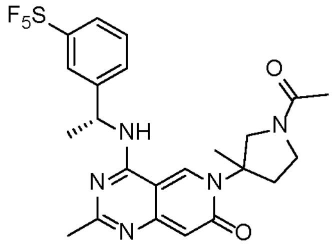
I-18

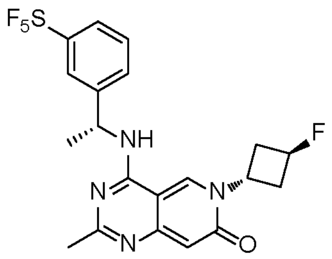

I-19

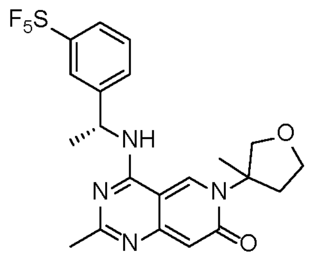

I-20

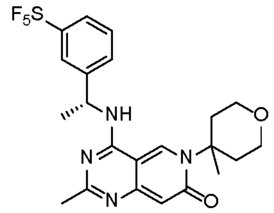

I-21

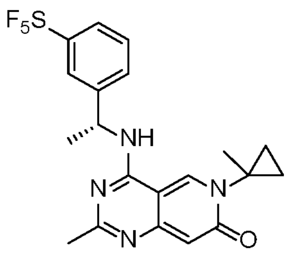

I-22

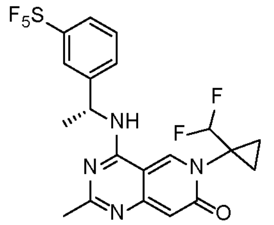

I-23.

**19.** An intermediate represented by formula B-1, a tautomer thereof, a stereoisomer thereof, and a salt thereof,

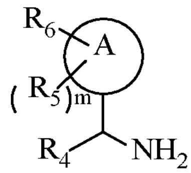

B-1;

wherein ring A, $R_4$, $R_5$ and $R_6$ are as defined in claim 1; m is 1 or 2.

**20.** The intermediate represented by formula B-1, the tautomer thereof, the stereoisomer thereof, and the salt thereof according to claim 19, wherein the intermediate has a structure of:

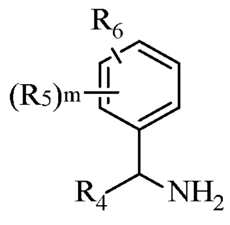

;

wherein ring A, $R_4$, $R_5$, $R_6$ and m are as defined in claim 14;
preferably, $R_4$ is methyl or $-CH_2F$; more preferably, $R_4$ is methyl.

**21.** The intermediate represented by formula B-1, the tautomer thereof, the stereoisomer thereof, and the salt thereof according to claim 19, wherein $R_5$ is hydrogen, fluorine or methyl; m is 1 or 2; preferably, m is 1;

$R_6$ is $-SF_5$,

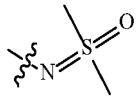 , 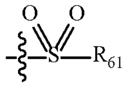 or 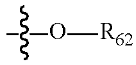 ;

the $R_{61}$ and the $R_{62}$ are each independently $C_1$-$C_6$ alkyl substituted by one or more than one F, or 3- to 6-membered cycloalkyl;
preferably, $R_{61}$ is $-CH_2F$, $-CHF_2$, $-CF_3$, $-CF_2CH_3$ or cyclopropyl;
preferably, $R_{62}$ is $-CH_2F$, $-CHF_2$, $-CF_3$; more preferably, $R_{62}$ is $-CHF_2$.

**22.** The intermediate represented by formula B-1, the tautomer thereof, the stereoisomer thereof, and the salt thereof according to claim 19, wherein the ring A together with $R_6$ and $R_5$ forms a group moiety

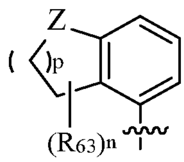 ;

wherein Z is

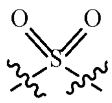 ;

p is 1, n is 2 or 3, $R_{63}$ is fluorine or hydroxyl, and when there is more than one $R_{63}$, the $R_{63}$ are the same or different; preferably, the group moiety

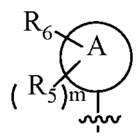

has a structure of

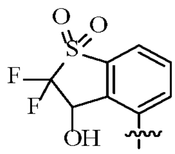 ;

more preferably,

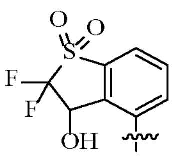 is 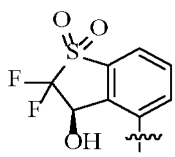 .

**23.** The intermediate represented by formula B-1, the tautomer thereof, the stereoisomer thereof, and the salt thereof according to claim 19, comprising:

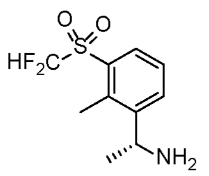
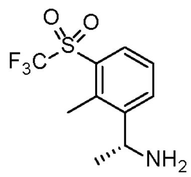
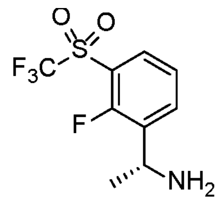

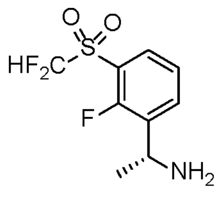
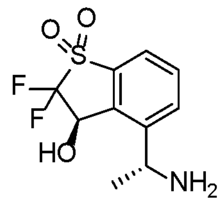
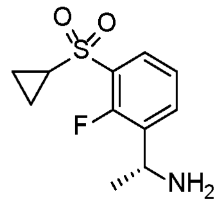

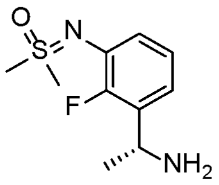
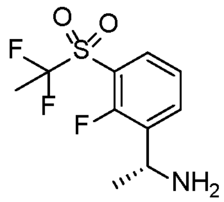
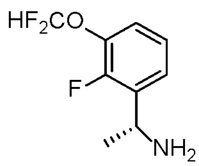

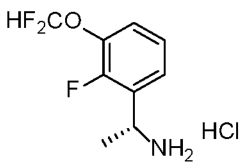
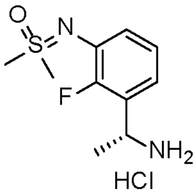

.

**24.** A method for preparing the pyridopyrimidinone derivative as represented by formula I, the tautomer thereof, the stereoisomer thereof, the hydrate thereof, the solvate thereof, the pharmaceutically acceptable salt thereof or the prodrug thereof according to claim 1 or 2, comprising:

1) reacting an intermediate B-1 with an intermediate B-2 to obtain the pyridopyrimidinone derivative as represented by formula I,

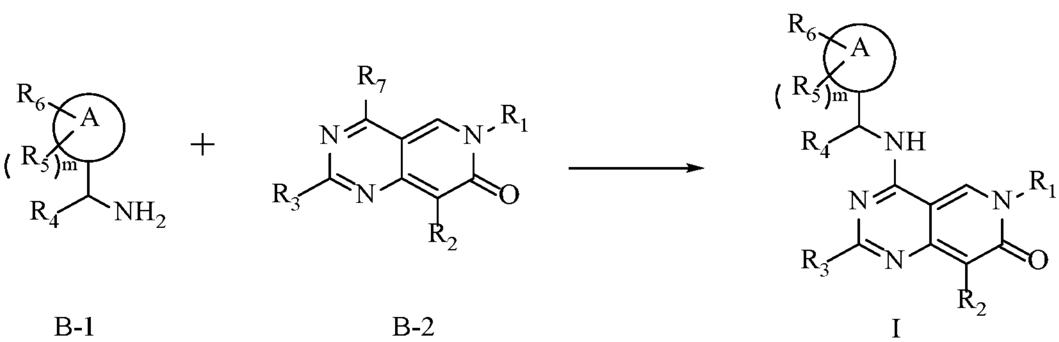

B-1 B-2 I

;

wherein ring A, $R_1$, $R_2$, $R_3$, $R_4$, $R_5$, $R_6$, m, p and n are as defined in claim 1 or 2;
$R_7$ is hydroxyl, chlorine, bromine, iodine or sulfonate; preferably, $R_7$ is hydroxyl;
preferably, the sulfonate is $-SO_3R_{71}$, wherein $R_{71}$ is methyl, $-CF_3$, phenyl or 2,4,6-trimethylbenzene.

**25.** The method according to claim 24, wherein ring A is phenyl; preferably, the group moiety

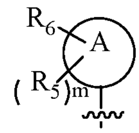

has a structure of

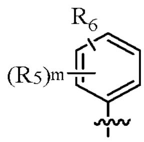

.

**26.** The method according to claim 24, wherein $R_5$ is hydrogen, fluorine or methyl; m is 1 or 2; preferably, m is 1;

$R_6$ is $-SF_5$,

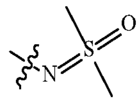 , 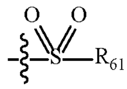 or 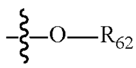 ;

the $R_{61}$ and the $R_{62}$ are each independently $C_1$-$C_6$ alkyl substituted by one or more than one F, or 3- to 6-membered cycloalkyl;
or, the ring A together with $R_6$ and $R_5$ forms a group moiety; wherein Z is

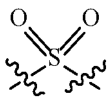

;

p is 1, n is 2 or 3, $R_{63}$ is fluorine or hydroxyl, and when there is more than one $R_{63}$, the $R_{63}$ are the same or different; preferably, the group moiety

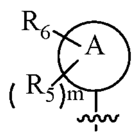

has a structure of

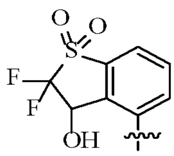

;

more preferably,

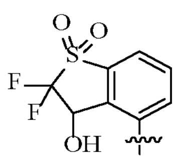 is 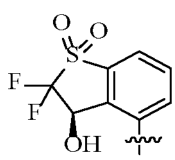 ;

preferably, $R_{61}$ is $-CH_2F$, $-CHF_2$, $-CF_3$, $-CF_2CH_3$ or cyclopropyl;
preferably, $R_{62}$ is $-CH_2F$, $-CHF_2$, $-CF_3$; more preferably, $R_{62}$ is $-CHF_2$.

**27.** The method according to claim 24, wherein the method further comprises: 2) converting the intermediate B-1 to an amine salt of B-1 and then reacting with the intermediate B-2 to obtain the pyridopyrimidinone derivative as represented by formula I.

**28.** A pharmaceutical composition comprising: the pyridopyrimidinone derivative as represented by formula I, the tautomer thereof, the stereoisomer thereof, the hydrate thereof, the solvate thereof, the pharmaceutically acceptable salt thereof or the prodrug thereof according to any one of claims 1 to 18; and a pharmaceutically acceptable carrier.

**29.** A pharmaceutical composition comprising: the pyridopyrimidinone derivative as represented by formula I, the tautomer thereof, the stereoisomer thereof, the hydrate thereof, the solvate thereof, the pharmaceutically acceptable salt thereof or the prodrug thereof according to any one of claims 1 to 18; and at least one other pharmacologically active inhibitor.

**30.** The pharmaceutical composition according to claim 29, wherein the other pharmacologically active inhibitor is an inhibitor of MEK and/or a mutant thereof; or,
the other pharmacologically active inhibitor is trametinib.

**31.** A use of the pyridopyrimidinone derivative as represented by formula I, the tautomer thereof, the stereoisomer thereof, the hydrate thereof, the solvate thereof, the pharmaceutically acceptable salt thereof or the prodrug thereof according to any one of claims 1 to 18, or a use of the pharmaceutical composition according to claim 28, or a use of the pharmaceutical composition according to claim 29, comprising:

inhibiting an interaction between SOS1 and RAS family proteins;
and/or, preventing and/or treating a disease related to SOS1 and RAS family proteins;
and/or, preparing a medicament, pharmaceutical composition or formulation for inhibiting an interaction between SOS1 and RAS family proteins, and/or preventing and/or treating a disease related to SOS1 and RAS family proteins;
and/or, preparing a medicament, for example, preparing a medicament for preventing and/or treating cancer and RASopathies.

**32.** The use according to claim 31, wherein the disease related to SOS1 and RAS family proteins comprises: cancer and RASopathies;

and/or, the RASopathies comprise Noonan syndrome, cardio-facio-cutaneous syndrome, hereditary gingival fibromatosis type 1, neurofibromatosis type 1, capillary malformation-arteriovenous malformation syndrome, Costello syndrome and Legius syndrome;
and/or, the RAS family protein is KRAS, such as KRAS G12C;
and/or, the cancer is selected from melanoma, skin cancer, liver cancer, kidney cancer, lung cancer, nasopharyngeal cancer, gastric cancer, esophageal cancer, colorectal cancer, gallbladder carcinoma, cholangiocarcinoma, choriocarcinoma, pancreatic cancer, polycythemia vera, pediatric tumor, cervical cancer, ovarian cancer, breast cancer, bladder cancer, urothelial carcinoma, ureteral tumor, prostate cancer, seminoma, testicular cancer, leukemia, head and neck tumor, endometrial cancer, thyroid cancer, lymphoma, sarcoma, osteoma, neuroblastoma, neuroblastoma, brain tumor, myeloma, astrocytoma, glioblastoma and glioma; the liver cancer is preferably hepatocellular carcinoma; the head and neck tumor is preferably head and neck squamous cell carcinoma; the sarcoma is preferably osteosarcoma; and the colorectal cancer is preferably colon cancer or rectal cancer.

**33.** The use according to claim 31, wherein the pyridopyrimidinone derivative as represented by formula I, the tautomer thereof, the stereoisomer thereof, the hydrate thereof, the solvate thereof, the pharmaceutically acceptable salt thereof or the prodrug thereof are used in combination with trametinib.

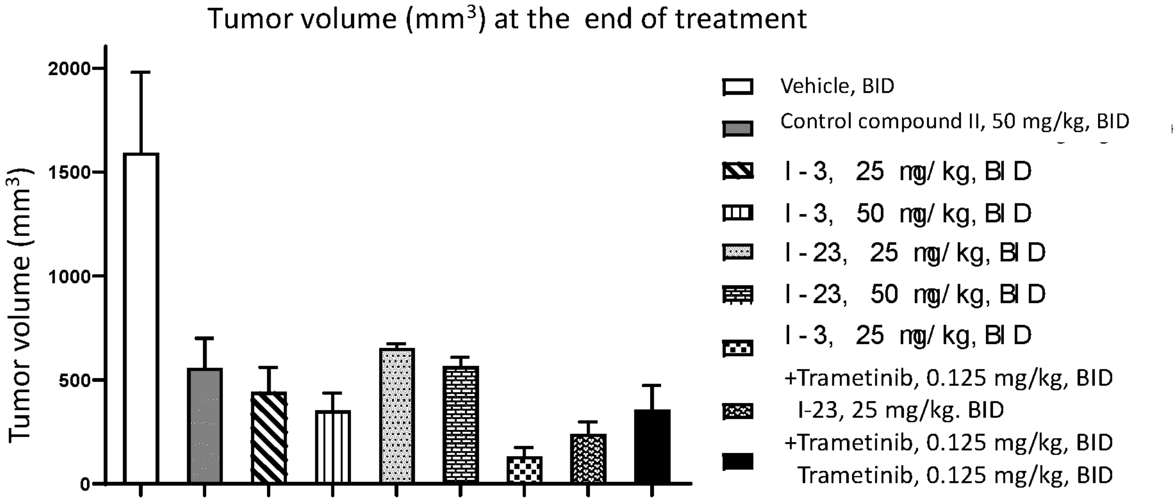
Tumor volume (mm3) at the end of treatment
Tumor volume (mm3)
2000
1500
1000
500
0
Vehicle, BID
Control compound II, 50 mg/kg, BID
I-3, 25 mg/kg, BID
I-3, 50 mg/kg, BID
I-23, 25 mg/kg, BID
I-23, 50 mg/kg, BID
I-3, 25 mg/kg, BID
+Trametinib, 0.125 mg/kg, BID
I-23, 25 mg/kg. BID
+Trametinib, 0.125 mg/kg, BID
Trametinib, 0.125 mg/kg, BID

FIG. 1

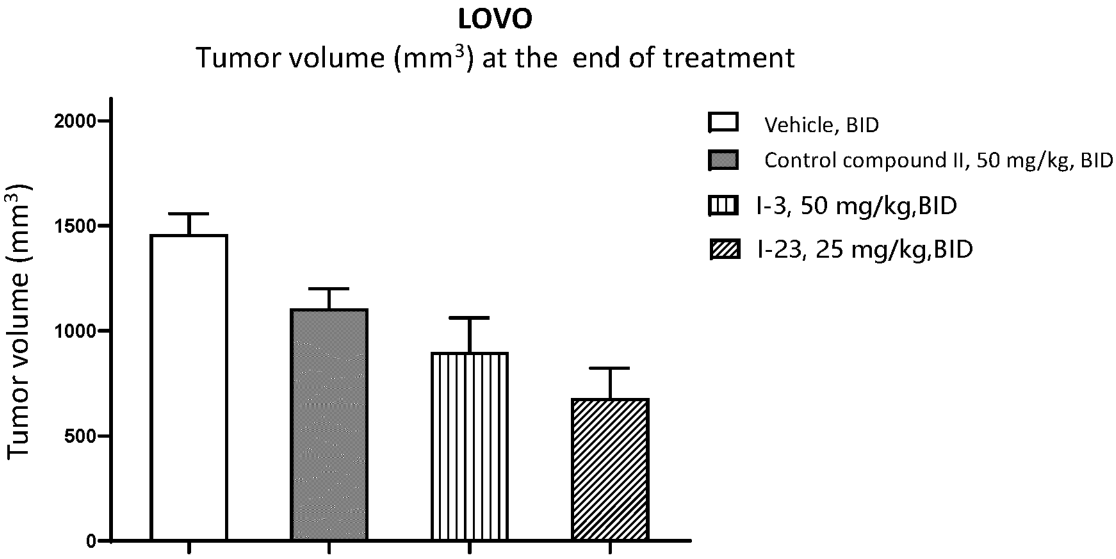
LOVO
Tumor volume (mm3) at the end of treatment
Tumor volume (mm3)
2000
1500
1000
500
0
Vehicle, BID
Control compound II, 50 mg/kg, BID
I-3, 50 mg/kg,BID
I-23, 25 mg/kg,BID

FIG. 2

**INTERNATIONAL SEARCH REPORT**

International application No.
**PCT/CN2022/075428**

**A. CLASSIFICATION OF SUBJECT MATTER**

C07D 471/04(2006.01)i; C07D 453/02(2006.01)i; C07D 519/00(2006.01)i; A61K 31/519(2006.01)i; A61P 35/00(2006.01)i; A61P 35/02(2006.01)i

According to International Patent Classification (IPC) or to both national classification and IPC

**B. FIELDS SEARCHED**

Minimum documentation searched (classification system followed by classification symbols)

C07D471/-; C07D453/-; C07D519/-; A61K31/-; A61P35/-

Documentation searched other than minimum documentation to the extent that such documents are included in the fields searched

Electronic data base consulted during the international search (name of data base and, where practicable, search terms used)

CNTXT; CNKI; DWPI; WPABS; REGISTRY (STN); CAPLUS (STN); 人福; 吡啶; 嘧啶酮; 鸟苷酸; 抑制剂; 癌; 肿瘤; HUMANWELL; pyridopyrimidinone; SOS; RAS; inhibit+; cancer; tumor; 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| PX | CN 113200981 A (HANGZHOU INNOGATE PHARMA CO., LTD.) 03 August 2021 (2021-08-03)<br>description, paragraphs [0007]-[0016], [0019], [0021], seven compounds fluoromethoxy groups of which are on phenyl rings, and paragraphs [0042]-[0058] | 1-33 |
| X | "CAS STN REGISTRY"<br>*RN 2091635-76-4, etc.,* 18 April 2017 (2017-04-18), | 19-21 |
| X | CN 101687797 A (SANOFI AVENTIS) 31 March 2010 (2010-03-31)<br>embodiment 77 | 19-21 |
| X | CN 104169281 A (SANOFI SA) 26 November 2014 (2014-11-26)<br>description, p. 119, compound 16bf intermediate | 19-21 |
| X | WO 2020221209 A1 (ABBISKO THERAPEUTICS CO., LTD.) 05 November 2020 (2020-11-05)<br>embodiments, intermediates 1-6 | 19-21 |
| X | WO 2016186888 A1 (MERCK SHARP & DOHME CORP.) 24 November 2016 (2016-11-24)<br>preparation embodiments 11 and 15 | 19-21 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 April 2022** | **26 April 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

INTERNATIONAL SEARCH REPORT

International application No.
PCT/CN2022/075428

C. DOCUMENTS CONSIDERED TO BE RELEVANT

| Category* | Citation of document, with indication, where appropriate, of the relevant passages | Relevant to claim No. |
|---|---|---|
| A | CN 111372932 A (BOEHRINGER INGELHEIM INTERNATIONAL GMBH) 03 July 2020 (2020-07-03)<br>description, paragraphs [0023]-[0071], and table 20 | 1-33 |
| A | CN 110167928 A (BOEHRINGER INGELHEIM INTERNATIONAL GMBH) 23 August 2019 (2019-08-23)<br>description, paragraphs [0021]-[0069], and table 21 | 1-33 |

Form PCT/ISA/210 (second sheet) (January 2015)

**INTERNATIONAL SEARCH REPORT**

International application No.
**PCT/CN2022/075428**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **31-33**
because they relate to subject matter not required to be searched by this Authority, namely:

[1] Claims 31-33 relate to a method for treating a disease. The present report is formed on the basis of a pharmaceutical use of the compound according to claims 1-18 or the pharmaceutical composition according to claims 28 and 29.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/075428**

| Patent document cited in search report | | | Publication date (day/month/year) | Patent family member(s) | | | Publication date (day/month/year) |
|---|---|---|---|---|---|---|---|
| CN | 113200981 | A | 03 August 2021 | None | | | |
| CN | 101687797 | A | 31 March 2010 | JP | 2010532769 | A | 14 October 2010 |
| | | | | CA | 2693396 | A1 | 15 January 2009 |
| | | | | EP | 2176223 | A1 | 21 April 2010 |
| | | | | BR | PI0814627 | A2 | 27 January 2015 |
| | | | | KR | 20100031732 | A | 24 March 2010 |
| | | | | AU | 2008274655 | A1 | 15 January 2009 |
| | | | | WO | 2009007015 | A1 | 15 January 2009 |
| | | | | US | 2010249101 | A1 | 30 September 2010 |
| CN | 104169281 | A | 26 November 2014 | US | 2015018342 | A1 | 15 January 2015 |
| | | | | MX | 2014009356 | A | 08 September 2014 |
| | | | | LT | 2809669 | T | 10 May 2017 |
| | | | | PT | 2809669 | T | 02 May 2017 |
| | | | | WO | 2013113860 | A1 | 08 August 2013 |
| | | | | UY | 34610 | A | 30 September 2013 |
| | | | | KR | 20140129065 | A | 06 November 2014 |
| | | | | RU | 2014135759 | A | 27 March 2016 |
| | | | | AR | 089863 | A1 | 24 September 2014 |
| | | | | SI | 2809669 | T1 | 31 May 2017 |
| | | | | JP | 2015521155 | A | 27 July 2015 |
| | | | | SG | 11201403852U | A | 26 September 2014 |
| | | | | PL | 2809669 | T3 | 31 July 2017 |
| | | | | DK | 2809669 | T3 | 01 May 2017 |
| | | | | HR | P20170618 | T1 | 30 June 2017 |
| | | | | AU | 2013214226 | A1 | 21 August 2014 |
| | | | | CA | 2860933 | A1 | 08 August 2013 |
| | | | | US | 2016159793 | A1 | 09 June 2016 |
| | | | | EP | 2809669 | A1 | 10 December 2014 |
| | | | | ES | 2623167 | T3 | 10 July 2017 |
| | | | | HU | E031729 | T2 | 28 July 2017 |
| | | | | CY | 1119108 | T1 | 14 February 2018 |
| WO | 2020221209 | A1 | 05 November 2020 | EP | 3964518 | A1 | 09 March 2022 |
| | | | | AU | 2020264642 | A1 | 02 September 2021 |
| | | | | BR | 112021019887 | A2 | 18 January 2022 |
| | | | | CN | 113366008 | A | 07 September 2021 |
| | | | | TW | 202106311 | A | 16 February 2021 |
| | | | | KR | 20210126051 | A | 19 October 2021 |
| | | | | CA | 3130253 | A1 | 05 November 2020 |
| WO | 2016186888 | A1 | 24 November 2016 | WO | 2016183741 | A1 | 24 November 2016 |
| | | | | EP | 3294297 | A1 | 21 March 2018 |
| | | | | US | 2018256575 | A1 | 13 September 2018 |
| CN | 111372932 | A | 03 July 2020 | AU | 2018390927 | A1 | 28 May 2020 |
| | | | | WO | 2019122129 | A1 | 27 June 2019 |
| | | | | US | 2021009588 | A1 | 14 January 2021 |
| | | | | IL | 275379 | D0 | 30 July 2020 |
| | | | | US | 2019194192 | A1 | 27 June 2019 |
| | | | | PE | 20210163 | A1 | 26 January 2021 |
| | | | | CR | 20210307 | A | 27 July 2021 |
| | | | | EA | 202091491 | A1 | 13 November 2020 |

Form PCT/ISA/210 (patent family annex) (January 2015)

**INTERNATIONAL SEARCH REPORT**
**Information on patent family members**

International application No.
**PCT/CN2022/075428**

| Patent document cited in search report | Publication date (day/month/year) | Patent family member(s) | Publication date (day/month/year) |
|---|---|---|---|
| | | CO 2020007218 A2 | 19 June 2020 |
| | | TW 201938557 A | 01 October 2019 |
| | | KR 20200111163 A | 28 September 2020 |
| | | CL 2021000907 A1 | 29 October 2021 |
| | | CL 2020001501 A1 | 13 November 2020 |
| | | CA 3085835 A1 | 27 June 2019 |
| | | JP 2021506864 A | 22 February 2021 |
| | | EC SP20040257 A | 31 August 2020 |
| | | CR 20200312 A | 11 September 2020 |
| | | SG 11202005881 Y A | 29 July 2020 |
| | | JO P20200154 A1 | 21 June 2019 |
| | | MA 51290 A | 31 March 2021 |
| | | EP 3728254 A1 | 28 October 2020 |
| | | BR 112020010123 A2 | 10 November 2020 |
| | | AR 114164 A1 | 29 July 2020 |
| CN 110167928 A | 23 August 2019 | JP 2020504742 A | 13 February 2020 |
| | | WO 2018115380 A1 | 28 June 2018 |
| | | EP 3878850 A1 | 15 September 2021 |
| | | US 2019358230 A1 | 28 November 2019 |
| | | US 2021177851 A1 | 17 June 2021 |
| | | EP 3558979 A1 | 30 October 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2021101723722
- CN 2021113158687
- WO 2019122129 A1


### Non-patent literature cited in the description


- **HUNTER et al.** *Mol. Cancer Res.,* 2015, vol. 13 (9), 1325-1335
- **HOBBS GA et al.** *J Cell Sci.,* 2016, vol. 129 (7), 1287-1292
- **COX, ADRIENNE D. et al.** *Nat Rev Drug Discov.,* 2014, vol. 13 (11), 828-851
- **LIU P et al.** *Acta Pharm Sin B.,* 2019, vol. 9 (5), 871-879
- **PIERRE S et al.** *Biochem Pharmacol.,* 2011, vol. 82 (9), 1049-1056
- **HILLIG, ROMAN C. et al.** *Proc Nat Acad Sci USA.,* 2019, vol. 116 (7), 2551-2560
- **HOFMANN, MARCO H et al.** *Cancer Discov.,* 2020
- **JOSÉ M ROJAS et al.** *Genes Cancer.,* 2011, vol. 2 (3), 298-305
- **NARUMI, YOKO et al.** *J Hum Genet.,* 2008, vol. 53 (9), 834-841
- **JANG SI et al.** *J Biol Chem.,* 2007, vol. 282 (28), 20245-20255
- **CAREY ; SUNDBERG.** ADVANCED ORGANIC CHEMISTRY 4TH ED. Plenum Press, New York, 2000, vol. A,B
- **MAEHR.** *J. Chem. Ed.,* 1985, vol. 62, 114-120